# EUROPEAN PATENT APPLICATION

(11) **EP 4 085 874 A1**
(43) Date of publication of application: **09.11.2022**
(21) Application number: 20910675.6
(22) Date of filing: 15.12.2020
(51) Int. Cl.: A61F 2/34, A61F 2/36

(54) **CONNECTION STRUCTURE OF POROUS SURFACE STRUCTURE AND SUBSTRATE, PREPARATION METHOD FOR CONNECTION STRUCTURE, AND PROSTHESIS**

(30) Priority: 30.12.2019 CN 201911394644; 04.09.2020 CN 202010922145
(71) Applicant: JY MEDICAL DEVICES (SHANGHAI) CO. LTD., Zone Shanghai 200131 (CN); YBNX MEDICAL TECHNOLOGIES (SUZHOU) CO. LTD., Jiangsu 215558 (CN)
(72) Inventor: YAO, Jianqing, Shanghai 200131 (CN); SHI, Jinhu, Shanghai 200131 (CN); ZHU, Yongliang, Shanghai 200131 (CN)
(74) Representative: Yang, Shu
(86) International application number: PCT/CN2020/136527
(87) International publication number: WO 2021/135927

(57) **Abstract**

The present invention discloses a connected structure of a porous surface structure and a substrate, a method for preparing the same, and a prosthesis of the same. The connected structure consists of a pre-connected or integrally formed composite body of a porous surface structure and an intermediate; and a substrate, which is connected to the the said intermediate to achieve the connection of the the said composite body to the said substrate; the composite body comprising a first composite region corresponding to a first stiffness; a remaining composite region in the composite body other than the first composite region, which at least contains a second composite region corresponding to a second stiffness; and the first stiffness is less than the second stiffness. The present invention achieves a fastened connection between the composite and the substrate and largely maintains the mechanical properties of the substrate; and it provides a prosthesis with excellent bone ingrowth properties and that the strength of the substrate is not substantially affected.

## Description

### Technology Field

The present invention relates to connection technology for mechanical structures, and in particular to medical devices, providing a connected structure of a porous surface structure and a substrate, a method for preparing the same, and a prosthesis with the same.

### Background

Engineering applications often have different requirements for the overall performance and surface properties of mechanical structures. For example, the overall performance (e.g., fatigue strength) of the acetabular cup and femoral stem of an artificial hip joint must meet the fatigue resistance requirements of the prosthesis under the dynamic loads that it will endure during an average of one to two million walking cycles per year for decades after implantation, and there are specific performance needs for the surface of the prosthesis to meet so that the surface of the prosthesis may be firmly bonded to the patient's bone structure to ensure that the prosthesis does not fail due to loosening; otherwise, the patient will have pain and the prosthesis must be removed, which means that the patient needs to undergo a revision surgery to implant a new prosthesis. Similar situations and needs exist for other orthopedic implants (e.g., spine). In fact, in other fields, there are situations where the substrate and the surface have different performance needs and a reliable and effective connection between the two is required.

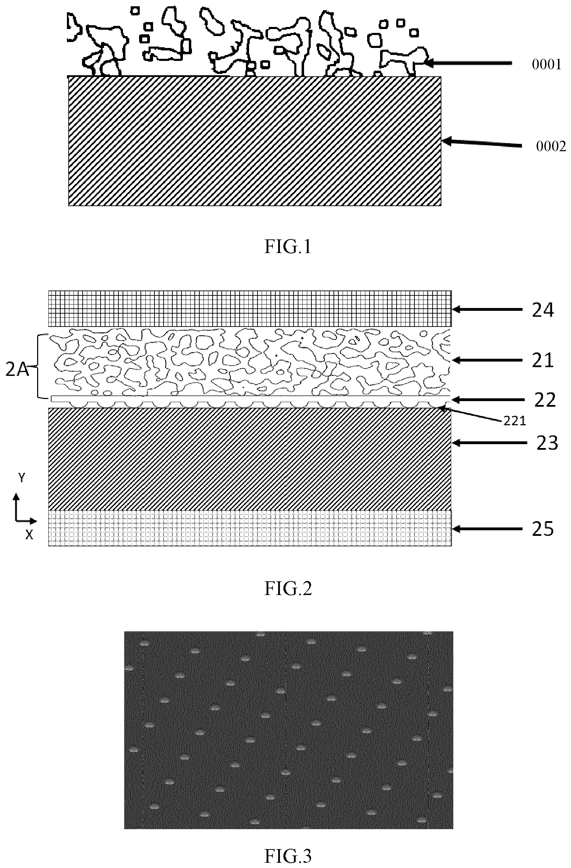

Commonly used materials for joint prostheses are titanium alloy/ cobalt-chromium alloy/ stainless steel, etc., which do not form an effective biological or chemical bond with bone. The interface between the prosthesis and the bone is primarily through physical/mechanical interlocking. For example, a highly polished prosthetic surface and bone tissue cannot form an effective bond, so increased osteoconduction, osteoinduction, and bone regeneration are needed to accelerate or enhance the bonding of bone tissue to the prosthetic surface and further improve bone growth onto or into the bone. Sometimes titanium wires or titanium beads, etc. can be used to form a porous coating on the surface of the prosthesis (e.g., acetabular cup/femoral stem) using methods such as sintering or diffusion bonding. Alternatively, a thin sheet 0001 with a porous structure is prefabricated using metal 3D printing additive manufacturing processes, vapor phase deposition processes, etc., and then the sheet 0001 is bonded to the solid substrate 0002 of the prosthesis through diffusion bonding, as shown in Figure 1. These methods provide a porous surface for the prosthesis, and the bone tissue in contact with the prosthesis is able to regenerate, filling the interconnected pores with new bone tissue and achieving the effect of "bone ingrowth" to the prosthesis. However, these processes have the inevitable consequence that the mechanical strength of the substrate is significantly reduced, thereby increasing the risk of fracture, especially when the prosthesis (e.g., femoral stem) is subjected to bending torque or tensile stress. Therefore, it is a design/process challenge to reliably and securely bond a porous structure to its substrate while ensuring that the mechanical properties of the substrate are not too significantly affected.

Relatively speaking, the laser welding process has a lower influence on the mechanical properties of the substrate. However, when the porosity of porous structures is high (>50%), the interconnected scaffolds are few in numbers and weak in strength; a large number of pores are formed between the scaffolds. Such a highly porous structure, whether achieved by 3D printing additive manufacturing process or other processes such as sintering, when the direct laser welding is used to connect the porous structure and the substrate, and the effective diameter of the laser beam is close to or even greater than the width of the scaffold, the laser energy may directly break the scaffolds, hence destroy the porous structure and can not achieve effective welding of the porous structure and the substrate. Alternatively, when diffusion bonding is used to connect the porous structure to the substrate, the strength of the substrate structure is significantly reduced due to the high temperature and elevated pressure of the process.

To avoid the above-mentioned limitations of laser welding and diffusion bonding, the resistance welding process could be used to effectively connect the porous structure and the substrate, where the two welding workpieces are pressed between two electrodes and an effective bonding is formed through the resistance heat generated by the electric current flowing through the physical contact between the two workpieces. However, for highly porous structures, when the porous structure and the substrate are directly connected using resistance welding, then the welding efficiency is low, resulting in insufficient welding strength or requiring an excessively high current in order to achieve sufficient welding strength. However, the latter results in excessive heat at the contact areas between the upper electrode and the upper surface of the porous structure, causing thermal damage to the surface of the porous structure, including the structural sinking of the porous structure, etc. Therefore, in the present invention, an intermediate structure between the porous structure and the substrate is designed to form a composite body comprised of the porous surface structure and the intermediate structure base plate and the composite body is then tightly bonded to the substrate, so as to improve the welding efficiency of the porous structure and the substrate with sufficient welding strength. While maintaining sufficient structural strength of the connected structure of the composite body and the substrate, the present invention has devised corresponding novel structures to avoid sub-optimal welding quality between the composite body and the substrate due to their inadequate fit to each other as a consequence of excessive stiffness of the composite body.

### Summary

The purpose of the present invention is to provide a connected structure of a porous surface structure and a substrate, a method for preparing the same and a prosthesis with the same. While forming strong connection between the porous surface structure, the intermediate structure and the substrate by resistance welding method, the mechanical properties of the substrate are largely maintained. In addition, while maintaining sufficient structural strength of the connected structure of the composite body and the substrate, the present invention has devised hollowed-out or other altered intermediate structures, achieving effective welding by avoiding the inadequate fit between the composite and the substrate as a consequence of excessive stiffness of the composite body. The surface of the porous structure based on the present invention can ensure the prosthetic implant to have excellent bone ingrowth properties, and the strength of the substrate is not substantially affected.

In order to achieve the above objectives, the present invention is realized by the following technical solutions:
A connected structure of a porous surface structure and a substrate, comprising: a pre-connected or integrally formed composite body, comprising a porous surface structure and an intermediate; a substrate, connected to the said intermediate and/or the said porous surface structure to achieve the connection of the said composite body to the said substrate; the said composite body comprising a first composite region corresponding to a first stiffness; the remaining composite regions in the said composite body other than the first composite region, comprising at least a second composite region corresponding to a second stiffness; the said first stiffness being less than the said second stiffness.

Preferably, the thickness of the said composite body at the said first composite region is less than the thickness of the said composite body at the said second composite region.

Preferably, the connected structure comprises any one of (a)-(c) below or any combination thereof:
(a) the thickness of the porous surface structure at the first composite region is less than the thickness of the porous surface structure at the second composite region; (b) the thickness of the main body of the intermediate at the first composite region is less than the thickness of the main body of the intermediate at the second composite region; (c) the gap between the side of the main body of the intermediate at the first composite region facing the substrate and the substrate is greater than the gap between the side of the main body of the intermediate at the second composite region facing the substrate and the substrate; and there is no porous surface structure at the gap; wherein the said main body of the intermediate is the portion of the intermediate located between the said porous surface structure and the said substrate.

Preferably, the thickness of the main body of the intermediate at the first composite region is 0, namely there is no main body of the intermediate at the first composite region, forming a hollowed-out structure.

Preferably, the gap between the substrate-facing side of the main body of the intermediate at the said first composite region and the substrate forms a pocket structure between the intermediate and the substrate.

Preferably, the said substrate is connected to the said intermediate and/or the said porous surface structure by laser welding and/or resistance welding.

Preferably, the said substrate, the said porous surface structure, the said intermediate are made of conductive materials.

Preferably, the said porous surface structure in the said composite is called a first porous structure; the said intermediate is a solid structure, or, alternatively, the said intermediate is a second porous structure and at least a portion of the said second porous structure has a higher solid volume fraction than the said first porous structure.

Preferably, the said intermediate further comprises: an insertion portion disposed within the porous surface structure, and/or a raised structure formed on a side of the composite body proximal to the said substrate; one or more of the said main body of the intermediate, the said insertion portion and the said raised structure having a higher solid volume fraction than that of the said porous surface structure.

Preferably, the said insertion portion comprises a support post.

Preferably, the said main body of the intermediate comprises an intermediate plate structure, the said intermediate plate structure being provided with a plurality of raised structures, the said raised structures being provided on the side of the said intermediate plate structure proximal to the said substrate, the bumps of the said raised structures being in contact with and fixedly connected to the said substrate.

Preferably, the said main body of the intermediate is the said second porous structure, the said second porous structure comprising a plurality of raised structures, the said raised structures being formed on a side of the said second porous structure proximal to the said substrate, the bumps of the said raised structures being in contact with the said substrate.

Preferably, the said first composite region comprises a plurality of dispersedly arranged composite sub-regions; each of the said sub-regions being between a plurality of adjacent raised structures corresponding thereto, or, alternatively, between a plurality of adjacent insertion portions corresponding thereto.

Preferably, all or at least part of the said insertion portion is located within the porous surface structure; the said insertion portion is arranged in correspondence with and in contact with the said raised structure, or the said insertion portion is staggered and not in direct contact with the said raised structure.

Preferably, the end surface of the insertion portion away from the substrate protrudes above or subsides below or is flush with the surface of the said porous surface structure;
Preferably, the end surface of the said insertion portion away from the substrate side protrudes above the surface of the said porous surface structure, the protruded portion is cut off after the resistance welding is completed.

Preferably, when the surface of the said insertion portion on the side away from the substrate protrudes above the surface of the said porous surface structure: the said insertion portion is a multi-segment structure comprising at least a first segment portion that protrudes above the said porous surface structure and a remaining second segment portion; the said first segment portion is porous; the said second segment portion is porous or solid, the said surface of the second section on the side away from the substrate is flush with the surface of the said porous surface structure such that the first segment is heated by contact with the first polar electrode causing the said insertion section to sink to the surface of the said second section on the side away from the substrate.

Preferably, the said raised structures on the said intermediate are located near the contact positions between the said porous surface structure and the said intermediate.

Preferably, at least some pores within the said porous surface structure are filled with an electrically conductive material.

Preferably, at least some pores within the said porous surface structure are filled with powdered or filamentary or mesh-like conductive materials.

Preferably, at least some pores of the porous surface structure are filled with a conductive medium in the molten state, and/or, at least some portions of the pores of the porous surface structure are filled with a conductive medium which becomes molten subsequently at high temperature; the said conductive medium has a melting point lower than the melting point of the substrate and/or that of the porous surface structure.

Preferably, the said substrate is a solid structure, or, alternatively, the said substrate is a third porous structure and the porosity of the said third porous structure is less than the porosity of the said porous surface structure.

Preferably, the said substrate is made by forging or casting or machining or powder metallurgy or metal injection molding process.

Preferably, the said porous surface structure and the said intermediate of the said composite are manufactured with 3D printing additive manufacturing or vapor phase deposition process.

Preferably, the said porous surface structure, the said intermediate and the said insertion portion are integrally formed.

Preferably, the said first composite region of the composite body is made by machining.

Preferably, the said substrate comprises a surface attachment layer, the said surface attachment layer being pre-connected or integrally formed with the main body of the substrate, the said surface attachment layer being located between the said intermediate of the composite and the main body of the substrate, or, the said surface attachment layer being located between the porous surface structure of the said composite and the main body of the substrate. the said surface attachment layer comprising raised structures on the substrate, the bumps of the said raised structures of the said surface attachment layer being in contact with and fixedly connected to the said intermediate and/or porous surface structure of the said composite body; or, the said raised structures are located on the surface of the said substrate proximal to the composite, being in contact with and fixedly connected to the said intermediate and/or porous surface structure of the said composite body.

Preferably, the said surface attachment layer of the said substrate is pre-welded to the body of the said substrate.

Preferably, the said main body of the intermediate has a flat surface proximal to the said substrate , or, alternatively, the said main body of the intermediate has raised structures on the surface proximal to the said substrate staggered relative to the raised structures on the surface attachment layer of the said substrate. The said main body of the intermediate is located between the said porous surface structure and the said substrate.

The present invention also provides a connected structure of a porous surface structure and a substrate, comprising a plurality of composites as described above and being respectively connected to different regions of the substrate.

Preferably, the plurality of composites comprises at least a first composite and a second composite, an intermediate in the first composite contacting and fixedly connected to a part of the said substrate, and intermediates in the said second composite body contacting and fixedly connected to another part of the said substrate to achieve the connection of the composite body to the said substrate; the said part of the said substrate and the said other part of the said substrate are respectively located on opposite sides of the substrate.

Preferably, the said first composite and the said second composite have the same structure; alternatively, the said first composite and the said second composite have different structures.

The present invention also provides a connected structure of a porous surface structure and a substrate, comprising: a pre-connected or integrally formed composite body comprising of a porous surface structure and an intermediate; a substrate, connected to the said intermediate and/or porous surface structure to achieve the connection of the said composite body to the said substrate; wherein it further comprises any one of (a)-(c) or any combination thereof: (a) the said intermediate being divided into a first region and remaining regions other than the first region, the said remaining regions comprising at least a second region, the thickness of the main body of the intermediate of the said intermediate at the said second region being greater than that at the said first region; the thickness of the main body of the intermediate at the said first region being greater than or equal to 0; (b) the said porous surface structure being divided into a first region and remaining regions other than the first region, the said remaining regions comprising at least a second region in which the thickness of the porous surface structure is greater than that at the said first region; (c) the said composite body being divided into a first composite region and remaining composite regions other than the first composite region, the said remaining composite regions comprising at least a second composite region, the gap between the side of the main body of the intermediate at the second composite region facing the substrate and the substrate is smaller than the gap between the side of the main body of the intermediate at the first composite region facing the substrate and the substrate; and there is no porous surface structure at the gap; wherein the said main body of the intermediate is the portion of the intermediate located between the said porous surface structure and the said substrate.

The present invention further provides a method for preparing a connected structure of a porous surface structure and a substrate, comprising the following process: forming a composite by pre-connecting the porous surface structure to an intermediate or integrally forming the composite; bringing the said intermediate and/or the said porous surface structure into contact with the substrate; bringing the said first polar electrode into conductive contact with the said porous surface structure and/or intermediate of the said composite and bringing the said substrate into conductive contact with a second polar electrode, thereby forming a current circuit; further comprising any one of (a)-(c) or any combination thereof: (a) the said intermediate being divided into a first region and remaining regions other than the first region, the said remaining regions comprising at least a second region, the thickness of the main body of the intermediate of the said intermediate at the said second region being greater than that at the said first region; the thickness of the main body of the intermediate at the said first region being greater than or equal to 0; (b) the said porous surface structure being divided into a first region and remaining regions other than the first region, the said remaining regions comprising at least a second region in which the thickness of the porous surface structure is greater than that at the said first region; (c) the said composite body being divided into a first composite region and remaining composite regions other than the first composite region, the said remaining composite regions comprising at least a second composite region, the gap between the side of the main body of the intermediate at the second composite region facing the substrate and the substrate is smaller than the gap between the side of the main body of the intermediate at the first composite region facing the substrate and the substrate; and there is no porous surface structure at the gap; wherein the said main body of the intermediate is the portion of the intermediate located between the said porous surface structure and the said substrate; the first composite region and the second composite region of the said composite body are resistance welded to the said substrate, respectively, to achieve the connection of the composite body to the substrate.

The present invention provides a method for preparing a connected structure of a porous surface structure and a substrate, comprising the following process: forming a composite by pre-connecting the porous surface structure to an intermediate or integrally forming the composite; bringing the said intermediate and/or the said porous surface structure into contact with the substrate; bringing the said first polar electrode into conductive contact with the said porous surface structure and/or intermediate of the said composite and bringing the said substrate into conductive contact with a second polar electrode, thereby forming a current circuit; the said composite body comprising a first composite region corresponding to a first stiffness; a remaining composite region in the composite body other than the first composite region, which at least contains a second composite region corresponding to a second stiffness; and the first stiffness is less than the second stiffness; the first composite region and the second composite region of the said composite body are resistance welded to the said substrate, respectively, to achieve the connection of the composite body to the substrate.

Preferably, the thickness of the composite at the said first composite region, is less than the thickness of the composite at the said second composite region.

Preferably, the method further comprises any one of (a)-(c) or any combination thereof: the thickness of the porous surface structure at the first composite region is less than the thickness of the porous surface structure at the second composite region; (b) the thickness of the main body of the intermediate at the first composite region is less than the thickness of the main body of the intermediate at the second composite region; (c) the gap between the side of the main body of the intermediate at the first composite region facing the substrate and the substrate is greater than the gap between the side of the main body of the intermediate at the second composite region facing the substrate and the substrate; and there is no porous surface structure at the gap; wherein the said main body of the intermediate is the portion of the intermediate located between the said porous surface structure and the said substrate.

Preferably, the thickness of the main body of the intermediate at the first composite region is 0; there is no main body of the intermediate at the first composite region, forming a hollowed-out structure.

Preferably, the gap between the substrate-facing side of the main body of the intermediate at the said first composite region and the substrate causes the intermediate to form a pocket structure with the substrate.

Preferably, the porous surface structure in the said composite is referred to as a first porous structure; the said intermediate is a solid structure, or, the said intermediate is a second porous structure and at least a portion of the said second porous structure has a higher solid volume fraction than the solid volume fraction of the said first porous structure.

Preferably, the said intermediate further comprises: an insertion portion disposed within a porous surface structure, and/or a raised structure formed on a side of the composite proximal to the said substrate; one or more of the said main body of the intermediate, the said insertion portion, and the said raised structure having a higher solid volume fraction than the solid volume fraction of the said porous surface structure.

Preferably, the said substrate is connected to the said intermediate by laser welding and/or resistance welding.

Preferably, when the said resistance welding is a projection resistance welding, the said first polar electrode is a continuous planar electrode or a segmented plurality of electrode monomers, and the said second polar electrode is a continuous planar electrode or a segmented plurality of electrode monomers; when the said resistance welding is a spot resistance welding, the said first polar electrode and/or the said second polar electrode is a segmented plurality of electrode monomers. Preferably, for spot resistance welding, moving from the current welding position to the next is achieved by moving any one or more of the following components: the first polar electrode, the second polar electrode, the combined structure of the intermediate and the substrate already welded at at least one contact position.

Preferably, when the said first polar electrode is divided into a plurality of electrode monomers, the said electrode monomers are inserted into prefabricated gaps within the porous surface structure, the electrode monomers being in close proximity to the said intermediates such that the said electrode monomers after insertion are in conductive contact with the said intermediates or such that the said electrode monomers after insertion are in conductive contact with the said intermediates after passing through the porous surface structure .

Preferably, the said electrode monomers pass through the surface of the porous surface structure up to the surface of the intermediate or into the interior of the intermediate, such that the said electrode monomers after insertion are in conductive contact with the said intermediate Preferably, the said electrode monomers are in lateral clearance-fit with the said porous surface structure, such that they are not in contact at all with the said porous surface structure.

Preferably, the said plurality of electrode monomers are connected in parallel to another planar electrode and the said other planar electrode is connected to the power supply end, or, alternatively, the said plurality of electrode monomers are connected in parallel and directly to the power supply end.

Preferably, the said first polar electrode is a flexible electrode, which is deformed under pressure to conform to the surface of the said porous surface structure, thereby increasing the area of contact between the said flexible electrode and the surface of the said porous surface structure. Preferably, the said first polar electrode is a positive electrode and the said second polar electrode is a negative electrode; or, that the said first polar electrode is a negative electrode and the said second polar electrode is a positive electrode.

Preferably, the said first polar electrode and the said second polar electrode are made of conductive materials; the said substrate, the said porous surface structure , and the said intermediate are made of conductive materials.

Preferably, the said insertion portion comprises a support post.

Preferably, the said main body of the intermediate comprises an intermediate plate structure, the said intermediate plate structure being provided with a plurality of raised structures, the said raised structures being provided on the side of the said intermediate plate structure proximal to the said substrate, the bumps of the said raised structures being in contact with the said substrate; or, alternatively, the said main body of the intermediate is the said second porous structure, the said second porous structure comprising a plurality of raised structures, the said raised structures being formed on the side of the said second porous structure proximal to the said substrate, the bumps of the said raised structures being in contact with the said substrate.

Preferably, the multiple composite sub-regions distributed across the said first composite region comprise of at least portions of the composite region between any two adjacent raised structures or between any two adjacent insertion portions.

Preferably, all or at least some parts of the said insertion portions are located within the porous surface structure; the said insertion portions are placed in contact with the corresponding raised structures , or misplaced with the said raised structures without contacting each other.

Preferably, the end surface of the insertion portion on the side away from the substrate protrudes above, or subsides below or is flush with the surface of the said porous surface structure. Preferably, the end surface of the said insertion portion away from the substrate side protrudes above the surface of the said porous surface structure, the protruded portion is cut off after the resistance welding is completed.

Preferably, when the surface of the said insertion portion on the side away from the substrate protrudes above the surface of the said porous surface structure: the said insertion portion is a multi-segment structure comprising at least a first segment portion that protrudes above the said porous surface structure and a remaining second segment portion; the said first segment portion is porous; the said second segment portion is porous or solid, the said surface of the second section on the side away from the substrate is flush with the surface of the said porous surface structure such that the first segment is heated by contact with the first polar electrode causing the said insertion section to sink to the surface of the said second section on the side away from the substrate.

Preferably, the said raised structures are placed on the said intermediate close to locations where the said porous surface structure is in contact with the said intermediate.

Preferably, at least a portion of the pores of the said porous surface structure are filled with conductive materials.

Preferably, at least a portion of the pores of the said porous surface structure are filled with powdered or filamentary or mesh-like conductive materials.

Preferably, at least a portion of the surface of the porous surface structure is covered with a solid film-like deformable conductive medium, the said deformable conductive medium being located between the said first polar electrode and the said porous surface structure; and/or, at least a portion of the surface of the porous surface structure is sprayed with the conductive medium between the surface of the porous surface structure and the said first polar electrode

Preferably, at least some pores of the porous surface structure are filled with a conductive medium in molten form, and/or, at least some pores of the porous surface structure are built with a conductive medium which is made molten by high temperature; the said conductive medium has a melting point lower than the melting point of the substrate and/or that of the porous surface structure.

Preferably, the said substrate is a solid structure, or, alternatively, the said substrate is a third porous structure and the porosity of the said third porous structure is less than the porosity of the said porous surface structure.

Preferably, the said substrate is manufactured with a forging or casting or machining or powder metallurgy or metal injection molding process.

Preferably, the porous surface structure and the intermediate of the said composite, is manufactured with a 3D printing additive manufacturing process, or a vapor phase deposition process.

Preferably, the said porous surface structure, the said main body of the intermediate and the said insertion portion are integrally formed.

Preferably, the first composite region of the composite body is made by machining..

Preferably, the said substrate comprises a surface attachment layer, the said surface attachment layer being pre-connected or integrally formed with the main body of the substrate, the said surface attachment layer being located between the said intermediate of the composite and the main body of the substrate, or, the said surface attachment layer being located between the porous surface structure of the said composite and the main body of the substrate. the said surface attachment layer comprising raised structures on the substrate, the bumps of the said raised structures of the said surface attachment layer being in contact with and fixedly connected to the said intermediate and/or porous surface structure of the said composite body; or, the said raised structures are located on the surface of the said substrate proximal to the composite, being in contact with and fixedly connected to the said intermediate and/or porous surface structure of the said composite body.

Preferably, the said surface attachment layer of the said substrate is pre-welded to the body of the said substrate.

Preferably, the said main body of the intermediate has a flat surface proximal to the said substrate, or, alternatively, the said main body of the intermediate has raised structures on the surface proximal to the said substrate staggered relative to the raised structures on the surface attachment layer of the said substrate; wherein the said main body of the intermediate is the portion of the intermediate located between the said porous surface structure and the said substrate.

Preferably, the said first polar electrode is in conductive contact with a side of at least a portion of the composite.

Preferably, the said first polar electrode is in electrically conductive contact with one or more of the sides of the porous surface structure, the sides of the insertion portion of the intermediate, and the sides of the main body of the intermediate.

The present invention further provides a connected structure of a porous surface structure and a substrate, comprising: a composite, comprising a pre-connected or integrally formed porous surface structure and an intermediate; a substrate, in contact with the said intermediate and/or the said porous surface structure; the said intermediate comprising a plurality of insertion portions, all or at least part of the said insertion portions being located within the porous surface structure, the said insertion portions being conductively connected to an accessory structure conductively connected, and the said accessory structure conductively connected to the said porous surface structure, the said accessory structure being disposed within the porous surface structure or, between the porous surface structure and the intermediate, or between the porous surface structure and the substrate.

Preferably, the said composite body comprises a first composite region corresponding to a first stiffness; the said remaining composite regions in the composite body, other than the first composite region, comprises at least a second composite region corresponding to a second stiffness; the said first stiffness being less than the said second stiffness.

Preferably, the thickness of the said composite body at the said first composite region, is less than the thickness of the said composite body at the said second composite region.

Preferably, the connected structure comprises any one of (a)-(c) below or any combination thereof: (a) the thickness of the porous surface structure at the first composite region is less than the thickness of the porous surface structure at the second composite region; (b) the thickness of the main body of the intermediate at the first composite region is less than the thickness of the main body of the intermediate at the second composite region; (c) the gap between the side of the main body of the intermediate at the first composite region facing the substrate and the substrate is greater than the gap between the side of the main body of the intermediate at the second composite region facing the substrate and the substrate; and there is no porous surface structure at the gap; wherein the said main body of the intermediate is the portion of the intermediate located between the said porous surface structure and the said substrate.

Preferably, the thickness of the main body of the intermediate at the first composite region is 0, namely there is no main body of the intermediate at the first composite region, forming a hollowed-out structure.

Preferably, the gap between the substrate-facing side of the main body of the intermediate at the said first composite region and the substrate forms a pocket structure between the intermediate and the substrate.

Preferably, the said insertion portion is good conductive solid structure or a third porous structure. Preferably, the said insertion portion comprises a support post.

Preferably, the said accessory structure is a good conductive solid structure or a fourth porous structure.

Preferably, the solid volume fraction of the said fourth porous structure is lower than the solid volume fractions of the said insertion portions.

Preferably, the said main body of the intermediate of the said intermediate is a portion disposed between the said porous surface structure and the said substrate, the said accessory structure being connected to the said main body of the intermediate, or, alternatively, the said accessory structure not being connected to the said main body of the intermediate.

Preferably, the respective accessory structures conductively connected on the plurality of insertion portions of the said intermediate are the same or different.

Preferably, any one of the insertion portions is conductively connected to an accessory structure in the shape of "I", or that a plurality of accessory structures conductively connected to any one of the insertion portions form the shape of "+".

Preferably, the said substrate and the said composite are placed between a first polar electrode and a second polar electrode, forming a current circuit by means of the said first polar electrode being in conductive contact with the said porous surface structure and/or intermediate, and the said substrate being in conductive contact with the second polar electrode, allowing the said intermediate and the said substrate to be resistance welding to achieve the connection of the said composite to the said substrate; one or more insertion portions are conductively connected to the corresponding accessory structures, allowing an increase in the current of the said current circuit. Preferably, the said substrate, the said porous surface structure, the said intermediate, and the said accessory structure are made of conductive materials.

Preferably, the said main body of the intermediate is a portion disposed between the said porous surface structure and the said substrate, the said main body of the intermediate being an intermediate plate structure, the said intermediate plate structure being provided with a plurality of raised structures, the said raised structures being provided on the side of the said intermediate plate structure proximal to the said substrate.

Preferably, the said main body of the intermediate is a portion disposed between the said porous surface structure and the said substrate, the said main body of the intermediate being the said second porous structure, the said second porous structure comprising a plurality of raised structures, the said raised structures being formed on the side of the said second porous structure proximal to the said substrate.

Preferably, the said intermediate comprises a plurality of raised structures distributed on the side of the said porous surface structure proximal to the substrate.

Preferably, he said insertion portions of the intermediate are placed at locations corresponding to the said raised structures and form contacts at these locations, or they are placed in staggers relative to the said raised structures of the intermediate without forming contacts.

Preferably, the end surface of the said insertion portion on the side away from the substrate protrudes above, or subsides below or is flush with the surface of the porous surface structure.

Preferably, when the end surface of the said insertion portion away from the substrate side protrudes above the surface of the said porous surface structure, the portion of the said insertion portion that protrudes above the said porous surface structure is cut off after the resistance welding is completed.

Preferably, when the surface of the said insertion portion on the side away from the substrate protrudes above the surface of the said porous surface structure: the said insertion portion is a multi-segment structure comprising at least a first segment portion that protrudes above the said porous surface structure and a remaining second segment portion; the said first segment portion is porous; the said second segment portion is porous or solid, the said surface of the second section on the side away from the substrate is flush with the surface of the said porous surface structure such that the first segment is heated by contact with the first polar electrode causing the said insertion section to sink to the surface of the said second section on the side away from the substrate.

Preferably, the said raised structures are located on the said intermediate in proximity to the contact position of the said porous surface structure with the said intermediate.

Preferably, at least some portions of the pores of the said porous surface structure are filled with conductive materials.

Preferably, ]at least some portions of the pores of the said porous surface structure are filled with powdered, filamentary or mesh-like conductive materials.

Preferably, at least some pores of the porous surface structure are filled with a conductive medium in the molten state, and/or, at least some portions of the pores of the porous surface structure are filled with a conductive medium which becomes molten subsequently at high temperature; the said conductive medium has a melting point lower than the melting point of the substrate and/or that of the porous surface structure.

Preferably, the said substrate is a solid structure, or, alternatively, the said substrate is a second porous structure and the porosity of the said second porous structure is less than the porosity of the said porous surface structure.

Preferably, the said substrate is made by a forging or casting or machining or powder metallurgy or metal injection molding process.

Preferably, the said porous surface structure and the said intermediate of the composite body are integrally formed.

Preferably, the said porous surface structure and the said intermediate of the composite are manufactured with a 3D printing additive manufacturing process, or a vapor phase deposition process.

Preferably, the said porous surface structure, the said intermediate, the said insertion portion and the said accessory structure are integrally formed.

The present invention further provides a connected structure of a porous surface structure and a substrate, comprising a plurality of composites in any one of the connected structures described above, and the plurality of composites are connected to different portions of the substrate, respectively.

Preferably, the plurality of composites comprises at least a first composite and a second composite, an intermediate in the first composite contacting and fixedly connected to a part of the said substrate, and an intermediate in the said second composite body contacting and fixedly connected to another part of the said substrate to achieve the connection of the composite body to the said substrate; the said part of the said substrate and the said other part of the said substrate are respectively located on opposite sides of the substrate.

Preferably, the said first composite and the said second composite have the same structure; or, that the said first composite and the said second composite have different structures.

The present invention further provides a method for preparing the connected structure of a porous surface structure and a substrate described above, comprising the following process: forming a composite by pre-connecting the porous surface structure to an intermediate or integrally forming the composite; bringing the said intermediate and/or the said porous surface structure into contact with the substrate; wherein the said intermediate comprises a plurality of insertion portions, each insertion portion being wholly or at least partially located within the porous surface structure, one or more insertion portions being conductively connected to corresponding accessory structures, and the said accessory structures being conductively connected to the said porous surface structure such that the current in the said current circuit is increased, the said accessory structures being placed within the porous surface structure, or between the porous surface structure and the intermediate, or, between the porous surface structure and the substrate; the said first polar electrode is in conductive contact with the said porous surface structure and/or intermediate, and the said substrate is in conductive contact with the said second polar electrode, forming a current circuit; the said composite is resistance welded to the said substrate to achieve the connection. The present invention further provides a prosthesis with a connected structure described above, comprising: (a) a pre-connected or integrally formed composite body of a porous surface structure and an intermediate, with the said composite body comprising a first composite region corresponding to a first stiffness and the remaining composite regions other than the first composite region, comprising at least a second composite region corresponding to a second stiffness; the said first stiffness being less than the said second stiffness; and (b) a substrate for forming a prosthetic body, at least a portion of the surface of the said prosthetic body serving as an area for connecting to the said composite body, the said intermediate and/or porous surface structure being connected to the said connection area of the said prosthetic body such that the said porous surface structure is located in the said connection area of the said prosthetic body.

Preferably, the said prosthesis is a joint prosthesis.

Preferably, the said composite is formed as a shell, wrapped around the connection region of the said prosthetic body; the outer layer of the said shell comprises a porous surface structure; and the inner layer of the said shell comprises an intermediate, which is connected to the connection region of the said prosthetic body.

Preferably, the said shell formed by the composite is an integral unit; or, the said shell formed by the composite comprises a plurality of shell units; wherein the plurality of shell units are independent of each other, or wherein adjacent shell units are connected to each other on at least one adjacent side.

Preferably, the said prosthesis comprises a femoral stem of the hip joint, the said femoral stem comprising a stem, which is formed as a substrate; the said connection region is positioned at the upper surface of the stem.

Preferably, the said lower surface of the said stem is a smooth surface, the said lower part of the said stem is provided with a number of longitudinal grooves, and the said lower part of the said stem is inserted into the femoral medullary cavity.

Preferably, the said femoral stem further comprises a top part and a neck, with the said top part, neck and the said stem body being integrally formed or assembled; the said top part of the femoral stem being of a conical structure, the first end of which is connected to the stem body through the neck and the second end of which is inserted into the femoral head; the top part and neck being arranged at an angle with respect to the stem body, in the form of an inclination with respect to the side of the stem body.

Preferably, the said composite body is formed as a shell wrapped around the periphery of the connection region of the said stem; the said composite body comprising a plurality of shell units. Preferably, the said prosthesis comprises an acetabular cup of the hip joint, the said acetabular cup comprising an inner cup body as the substrate and the outer surface of the acetabular cup as the connection region.

Preferably, at the said prosthesis comprises a tibial component, the said tibial component comprising a tibial tray which is formed as a substrate; the said connection area is positioned at the distal surface of the tibial tray.

Preferably, the said prosthesis comprises a femoral condyle, the said femoral condyle comprising the condylar body as the substrate and the inner fixation surface as the connection area.

Preferably, the said prosthesis is any one or more of: a patella prosthesis, a spinal fusion device, an intervertebral facet joint of the spine, an ankle joint prosthesis, a shoulder joint implant, an elbow joint prosthesis, a finger joint prosthesis, a toe joint implant, an artificial disc, a mandibular joint prosthesis, a wrist joint prosthesis.

The present invention provides a prosthesis with a connected structure as described above, comprising: a pre-connected or integrally formed composite of a porous surface structure and an intermediate, and a substrate, which is the main body of the prosthesis, of which at least a part of the surface being used as the connection area to connect with the said composite. The said intermediate and/or the said porous surface structure are connected with the connection area of the main body of the prosthesis so that the porous surface structure is located at the connection region of the main body of the prosthesis. The said intermediate comprises a plurality of insertion portions, each insertion portion being wholly or at least partially located within the porous surface structure, one or more insertion portions being conductively connected to corresponding accessory structures, and the said accessory structures being conductively connected to the said porous surface structure, the said accessory structures being placed within the porous surface structure, or between the porous surface structure and the intermediate, or, between the porous surface structure and the substrate.

Preferably, the said prosthesis is a joint prosthesis.

Preferably, the said composite is formed as a shell, wrapped around the connection region of the said prosthetic body; the outer layer of the said shell comprises a porous surface structure; and the inner layer of the said shell comprises an intermediate, which is connected to the connection region of the said prosthetic body.

Preferably, the said shell formed by the composite is an integral unit; or, the said shell formed by the composite comprises a plurality of shell units; wherein the plurality of shell units are independent of each other, or wherein adjacent shell units are connected to each other on at least one adjacent side.

Preferably, the said prosthesis comprises a femoral stem of the hip joint, the said femoral stem comprising a stem, which is formed as a substrate; the said connection region is positioned at the upper surface of the stem.

Preferably, the said lower surface of the said stem is a smooth surface, the said lower part of the said stem is provided with a number of longitudinal grooves, and the said lower part of the said stem is inserted into the femoral medullary cavity.

Preferably, the said femoral stem further comprises a top part and a neck, with the said top part, neck and the said stem body being integrally formed or assembled; the said top part of the femoral stem being of a conical structure, the first end of which is connected to the stem body through the neck and the second end of which is inserted into the femoral head; the top part and neck being arranged at an angle with respect to the stem body, in the form of an inclination with respect to the side of the stem body.

Preferably, the said composite body is formed as a shell wrapped around the periphery of the connection region of the said stem; the said composite body comprising a plurality of shell units. Preferably, the said prosthesis comprises an acetabular cup of the hip joint, the said acetabular cup comprising an inner cup body as the substrate and the outer surface of the acetabular cup as the connection region.

Preferably, at the said prosthesis comprises a tibial component, the said tibial component comprising a tibial tray which is formed as a substrate; the said connection area is positioned at the distal surface of the tibial tray.

Preferably, the said prosthesis comprises a femoral condyle, the said femoral condyle comprising the condylar body as the substrate and the inner fixation surface as the connection area.

Preferably, the said prosthesis is any one or more of: a patella prosthesis, a spinal fusion device, an intervertebral facet joint of the spine, an ankle joint prosthesis, a shoulder joint implant, an elbow joint prosthesis, a finger joint prosthesis, a toe joint implant, an artificial disc, a mandibular joint prosthesis, a wrist joint prosthesis, a dental implant.

Compared with the prior art, the beneficial effect of the present invention is that
(1) The present invention provides a method for preparing a connecting structure between a porous surface structure and a substrate by manufacturing a composite body containing a porous surface structure and an intermediate structure having a higher solid volume fraction relative thereto (e.g., a porous structure with a low porosity or a solid plate) by 3D printing or other processes, and the present invention uses resistance welding methods (e.g., projection resistance welding or spot resistance welding, etc.) to effectively connect the said composite body and the substrate. This projection welding process utilizes contact resistance to generate focused local heating. Hence, it not only avoid gaps the limitation of the laser welding method (where the laser energy directly breaks the scaffolds of the porous structure instead of welding them to the substrate), but also avoids largely the problem associated with high temperature and high pressure processes (e.g. diffusion bonding, where mechanical properties of the substrate may decrease substantially). The invention also entails the combined use of projection resistance welding and spot resistance welding to enhance the welding strength between the intermediate structure and the substrate as well as to reduce the surface damage to the porous surface structures.
(2) The present invention manufactures a solid (high solid volume fraction) substrate by processes such as forging, casting or machining, or a porous substrate where the porous surface structure has a lower solid volume fraction than the substrate, and the solid volume fraction of the intermediate structure is higher than that of the porous surface structure but lower than that of the substrate.
(3) The present invention provides solid structure support posts inside the porous surface structure to ensure that the height of the porous surface structure after resistance welding can reach a preset height, avoiding excessive compression of the porous surface structure; when the support posts are made of good conductive materials, most of the current output from the electrode flows preferentially through the support posts to the substrate, which can not only ensure the maximal welding strength between the intermediate structure and the substrate and but also reduce the damage to the porous surface structure. The present invention uses the above-mentioned support posts in combination with the bumps of the raised structures below the porous surface structure and also uses the direcrt contact between the bumps and the substrate to meet the requirements of both the high welding strength of the intermediate structure and the substrate and minimal damage caused to the surface of the porous surface structure.
(4) The present invention reduces the thickness of the composite of the porous surface structure and the intermediate at corresponding composite region (e.g. the intermediate of the composite incorporates a non-porous base plate with hollowed-out design) so that the stiffness of this composite region is reduced, which not only maintains the strength of the entire connected structure but also enables the raised structure for welding and the substrate to be in close contact with each other to enhance the welding efficiency between the non-porous base plate and the substrate. The present invention can also leave a certain gap between the non-porous base plate and the substrate to form a pocket structure, which can be used for expanding the usefulness of this connected structure.
(5) The present invention can improve uniformity of welding quality at different welding locations due to corresponding uneven thicknesses of the substrate by optimally varying the size of each support post and/or the size of each raised structure on the intermediate; the present invention can also provide conductive accessory structures to be connected to the support posts of the intermediate, so that most of the electric current flowing from the electrode is preferentially passing through the said support posts with accessory structures of good conductivity, greatly reducing the surface damage of the porous surface structure from resistance heating, and simultaneously enhancing the current conduction to ensure sufficient welding strength, and avoiding unsightly surface indentation marks or excessive stiffness of the composite with increased number or diameters of the support posts. The present invention allows tailored-design of the number and sizes of accessory structures connected to corresponding support posts according to specific circumstances of each support post, in order to ensure the uniformity and the magnitudes of the welding strengths between support posts and the substrate at different locations.
(6) The present process of the invention is simple, and reduces manufacturing costs and saves manufacturing time.
(7) Various implants, especially orthopedic prostheses such as femoral stems, acetabular cups, tibial trays and femoral condyles can be manufactured, using the methods and connecting structures disclosed in the present invention for a porous surface structure and its substrate, so that the implants are easy to manufacture, possess high strengths and exhibit optimal bone ingrowth through a porous surface structure as well as minimal cross-sectional area of the implants (e.g. hip stem).

### Briefly Descriptions of Drawings

Fig. 1 shows a schematic diagram of the prior art of the connecting structure of the porous surface structure and the substrate.
FIG. 2 is a schematic diagram of the connecting structure of the porous surface structure and the substrate of Embodiment I of the present invention.
FIG. 3 is a schematic diagram of the structure of the porous base plate of Embodiment I of the present invention.
Figure 4a shows a schematic diagram of the connecting structure of the porous surface structure and substrate of Embodiment II of the present invention (no bumps on the lower surface of the low porosity region).
Figure 4b shows a schematic diagram of the connecting structure between the porous surface structure and the substrate of Embodiment II of the present invention (bumps on the lower surface of the low porosity region).
FIG. 5a shows a schematic diagram of the connecting structure of the porous surface structure and the substrate of Embodiment III of the present invention;
FIG. 5b shows a schematic diagram of the connecting structure of the porous surface structure and the substrate of Embodiment IV of the present invention;
FIGS. 5c-d show schematic diagrams of the connecting structure of the porous surface structure and the substrate of Embodiment V of the present invention;
FIG. 6a shows a schematic diagram of the connecting structure of the porous surface structure and the substrate of Embodiment VI of the present invention;
FIG. 6b shows a schematic diagram of the connecting structure of the porous surface structure and the substrate of Embodiment VII of the present invention;
FIG. 7a shows a schematic diagram of the connecting structure of the porous surface structure and the substrate of Embodiment VIII of the present invention;
FIG. 7b shows a schematic diagram of the connecting structure of the porous surface structure and the substrate of Embodiment IX of the present invention;
FIGS. 8 & 8a show schematic diagrams of the connecting structure of the porous surface structure and the substrate of Embodiment X of the present invention;
FIG. 9a shows a schematic diagram of the connecting structure of the porous surface structure and the substrate of Embodiment XI of the present invention;
FIGS. 9b-f show schematic diagrams of the connecting structure of the porous surface structure and the substrate of Embodiment XII of the present invention;
FIG. 10 shows a schematic diagram of the connecting structure of the porous surface structure and the substrate of Embodiment XIII of the present invention;
FIGS. 11a-b show schematic diagrams of the connecting structure of the porous surface structure and the substrate of Embodiment XIV of the present invention;
FIG. 12 shows a schematic diagram of the connecting structure of the porous surface structure and the substrate of Embodiment XV of the present invention;
FIGS. 13-14 show schematic diagrams of the connecting structure of the porous surface structure and the substrate of Embodiment XVI of the present invention;
FIG. 15 shows a schematic diagram of the connecting structure of the porous surface structure and the substrate of Embodiment XVIII of the present invention;
FIG. 16 shows a schematic diagram of the connecting structure of the porous surface structure and the substrate of Embodiment XIX of the present invention;
FIG. 17 shows a schematic diagram of the connecting structure of the porous surface structure and the substrate of Embodiment XXI of the present invention;
FIGS. 17a-c show schematic diagrams of the hollowed-out structure of the non-porous base plate of Embodiment XXI of the present invention;
FIGS. 18a-b are schematic diagrams of the simple complete hollowed-out structure and incomplete hollowed-out structure of Embodiment XXI of the present invention, respectively;
FIG. 19 is a schematic diagram of the pocket structure of the non-porous base plate of Embodiment XXI of the present invention;
FIG. 20 is a schematic diagram of the structure of a support post with an accessory structure of Embodiment XXII of the present invention;
FIG. 21 is a schematic diagram of the porous surface structure and the substrate of Embodiment XXIV of the present invention;
FIG. 22a is a schematic diagram of the principle of variations of the connected structure of Embodiment XXV of the present invention;
FIG. 22b is a schematic diagram of the principle of variations of the connected structure of Embodiment XXV of the present invention;
FIGS. 23a-b are schematic diagrams of the hip femoral stem of the joint prosthesis of Embodiment XXVI of the present invention;
FIG. 23c is a schematic diagram of the cross-section of FIG. 23a of the present invention.
FIG. 23d shows an enlarged schematic view of M in FIG. 23c of the present invention;
FIGS. 24a-e show schematic views of the shells covering the stem body of a joint prosthesis of Embodiment XXVI of the present invention;
FIG. 25a shows a schematic view of the acetabular cup of the joint prosthesis of Embodiment XXVII of the present invention;
FIG. 25b shows an enlarged schematic view of FIG. 25a of the present invention;
FIG. 26a shows a schematic view of the tibial component of the joint prosthesis of Embodiment XXVIII of the present invention;
FIG. 26b is an enlarged view of FIG. 26a of the present invention;
FIG. 27a is a schematic diagram of the femoral condyles of the joint prosthesis of Embodiment XXIX of the present invention;
FIG. 27b is an enlarged view of FIG. 27a of the present invention.

### Detailed Descriptions

In order to clarify the purpose, technical solutions and advantages of the embodiments of the present invention, the technical solutions in the embodiments of the present invention will be described clearly and completely below with reference to the accompanying drawings in the embodiments of the present invention. It should be emphasized that the described embodiments are only exemplary but not exhaustive illustrations t of the embodiments of the present invention. Based on the exemplary embodiments in the present invention, all other embodiments obtained by a person of ordinary skills in the art without making creative labor fall within the scope of protection of the present invention.

### Embodiment I

As shown in FIG. 2, the present invention provides a connecting structure comprising of a substrate 23, an intermediate structure 22, and a porous surface structure 21, wherein the porous structure of the porous surface structure 21 contains a multitude of interconnected scaffolds (or beams), which form a number of multi-directionally interconnected pores with regular or irregular shapes. The intermediate structure 22 is disposed between the porous surface structure 21 and the substrate 23. Optionally, the intermediate structure 22 is a non-porous substrate, i.e., a solid substrate. Porous surface structure 21 and intermediate structure 22 are made of conductive material (e.g., metallic materials). The porous surface structure 21 and the intermediate structure 22 are integrally formed, through various manufacturing processes, for instance, 3D printing additive manufacturing or vapor phase deposition.

Exemplarily, the substrate 23 is solid, contributing to the overall strength of the connected structure. The substrate 23 may be made of conductive materials (e.g., metallic materials) and by various means such as forging and casting, and subsequent machining processes.

In this embodiment, the porous surface structure 21 is pre-connected to the intermediate structure 22 to form the composite 2A, and the intermediate structure 22 is effectively bonded to the substrate 23 by a resistance welding method to enable the connection of the composite 2A to the substrate 23. The said resistance welding method comprises a spot welding and/or a projection welding and/or other types. The following Embodimentsfocus on the connection of the intermediate structure 22 to the substrate 23 by the projection resistance welding method as illustration.

Specifically, at least a portion of the top surface of the porous surface structure 21 is in contact with the positive electrode 24. As the porous surface structure 21 is pre-connected to the intermediate structure 22, at least a portion of the lower part of the porous surface structure 21 is in contact with the top part of the intermediate structure 22. The bottom of the intermediate structure 22 is prefabricated with a plurality of raised structures 221 that are in contact with the top of the substrate 23, and the bottom of the substrate 23 is in contact with the negative electrode 25. Wherein, the raised structures 221 are raised towards the side of the substrate 23. Preferably, the plurality of raised structures 221 are manufactured at locations corresponding to the locations where the bottom of the porous surface structure 21 is in contact with the top of the intermediate structure 22 and the adjacent areas thereof. Wherein the positive direction of X-axis shown in FIG. 2 indicates right, the negative direction of X-axis indicates left, the positive direction of Y-axis indicates top, and the negative direction of Y-axis indicates bottom. The orientation provisions of the subsequent embodiments are the same as those in this Embodiment I, in order to more clearly describe the technical solution of the present invention, and the above orientation provisions are used only for illustration purposes without affecting the orientations in actual applications.

The composite 2A formed by the porous surface structure 21 and the intermediate structure 22, and the substrate 23 are pressed between the positive electrode 24 and the negative electrode 25, When the electrical current is provided, the current flows through the porous surface structure 21 and the intermediate structure 22 to the contact area between the raised structures 221 and the top of the substrate 23 and the adjacent area, resistance heating is generated from the contact resistance, thereby heating the raised structures 221 and the top of the substrate 23 to a molten or plastic state, and form welding nuggets between the raised structures 221 of the intermediate structure 22 and the top of the substrate 23, with eventual connection between the intermediate structure 22 and the substrate 23. Consequently, the composite body 2A formed by the porous surface structure 21 and the intermediate structure 22 is tightly bonded to the substrate 23.

Since the intermediate structure 22 is provided with a plurality of raised structures 221 at the bottom, so that the raised structures 221 are in contact with the top surface of the substrate 23, there is a contact resistance between the two. The electrical current provision enables the current to pass through the contacts thereby generating resistance heat to form welding points between the raised structures 221 and the substrate 23. The contact resistance is the resistance between two separate workpieces in contact, and the resistance heat Q is proportional to IR², where R is the contact resistance, I is the current through the workpiece..Hence, with greater current and greater contact resistance, the greater resistance heating is generated, and vice versa.

Based on the above, it can be seen that sufficient welding strength can be achieved between the raised structures 221 and the substrate 23 if the contact resistance between the raised structures 221 (For example, bumps) of the intermediate structure 22 and the substrate 23 is increased to generate a sufficient amount of resistance heating. Preferably, the substrate 23 is made of titanium alloy.

Optionally, the raised structures 221 of the intermediate structure 22 may be spherical-shaped or arc-shaped or ring-shaped or elongated or in any other shapes. Neither this nor other embodiments of the present invention specifically limit this aspect. , As shown in Figure 3, the intermediate structure 22 may have various bumps or textures to reduce the contact area and increase the contact resistance, thereby correspondingly increasing the welding efficiency with the substrate and improving the welding strength between the intermediate structure and the substrate. The raised structure in this Embodiment can be a solid body or a porous structure with high solid volume fraction (e.g. higher solid volume fraction than the porous surface structure), which is also applicable to the subsequent embodiments and will not be repeated in the present invention. The above-mentioned raised structure and the main body of the intermediate can be set separately or at the same time. If the main body of the intermediate and the raised structure are set separately, they are combined together in advance; if the main body of the intermediate and the raised structure are set at the same time, a raised structure can be formed on the bottom side of the main body of the intermediate facing the porous surface structure.

Exemplarily, the positive electrode 24 and negative electrode 25 are made of conductive materials (such as metallic materials); the top of the negative electrode 25 is in intimate contact with the bottom of the substrate 23, and similarly between the bottom of the positive electrode 24 and the top of the porous surface structure 21. The contact surfaces may be flat or arced or curved. The present invention does not specifically limit the shape and size of such contact surfaces, which can be designed according to the actual application.

Therefore, by adding an intermediate structure between the porous surface structure and the substrate, and using a resistance welding method (e.g., projection welding) to weld the composite formed by the intermediate structure and the porous surface structure, and the substrate, the present invention can ensure a high welding efficiency (e.g., 70% to 80%) even when the porosity of the porous structure is very high (>50%).

The positive electrode 24 and the negative electrode 25 in this Embodiment are also interchangeable, and this expansion is also applicable to each subsequent embodiment, which will not be repeated further in this invention.

### Embodiment II

For example I above, the porous surface structure 21 is a structure with a certain porosity, the intermediate structure 22 is located between the porous surface structure 21 and the substrate 23, and the intermediate structure 22 is a non-porous plate 22. In fact, the intermediate structure 22 can be a solid plate as described in Embodiment I, or a porous structure with a low porosity as elaborated in Embodiment II.

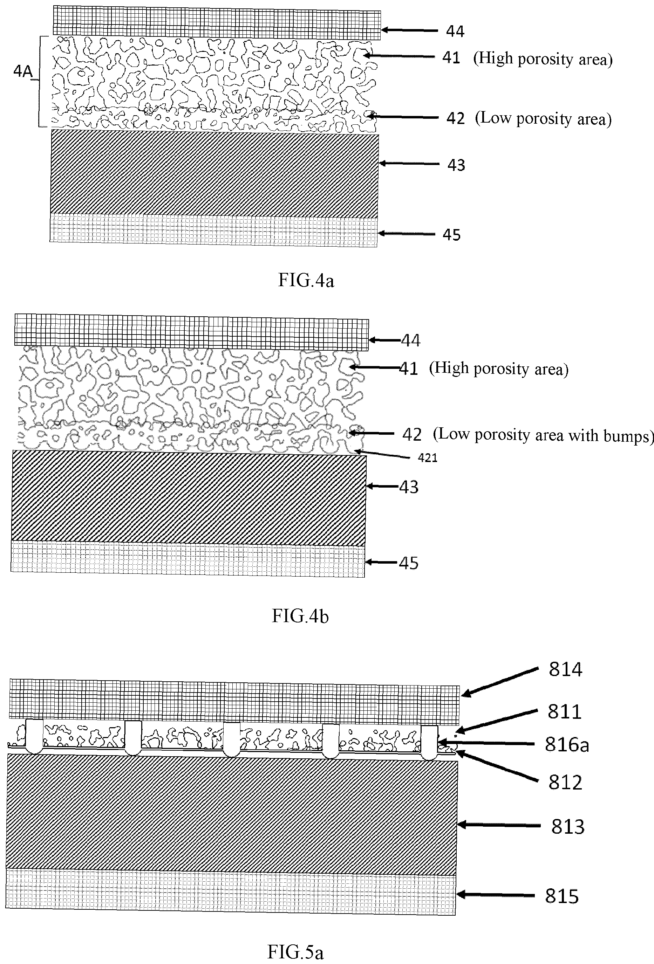

Thus, the main difference from Embodiment I is that the connecting structure of this Embodiment II comprises a first porous structure 41 in the high porosity region, a second porous structure 42 in the low porosity region (as an intermediate structure) and a substrate 43, as shown in Figure 4a. The second porous structure 42 is located between the first porous structure 41 and the substrate 23.

Exemplarily, the porous structures of both the first porous structure 41 and the second porous structure 42 contain a multitude of interconnected scaffolds (or beams), which form a number of multi-directionally interconnected pores with regular or irregular shapes. Among them, the porosity size of the first porous structure 41 is noted as a%, and the porosity size of the second porous structure 42 is noted as b%, with a% > b%. When the value of b% is equal to 0, the second porous structure 42 here is the solid structure intermediate structure as described in Embodiment 1. Thus, in comparison with the first porous structure 41 that constitutes a porous surface structure, the intermediate structure uses a second porous structure 42,with a higher solid volume fraction, as shown by a thicker scaffold(or beam) and/or lower porosity in the second porous structure 42.

In this embodiment, the first porous structure 41 and the second porous structure 42 are both made of conductive materials (e.g., metallic materials). The first porous structure 41 and the second porous structure 42 are integrally formed, which can be made with a 3D printing additive manufacturing process, or a vapor phase deposition process, etc.

The first porous structure 41 and the second porous structure 42 together are formed as a composite 4A, and the second porous structure 42 is effectively bonded to the substrate 43 by resistance welding method, such as projection resistance welding method: at least part of the scaffold (or beam) at the bottom of the second porous structure 42 is in contact with the top of the substrate 43, and resistance heat is generated due to the contact resistance, thus heating the contact part of both to a molten or plastic state. The second porous structure 42 and the top of the substrate 43 form a bonded metallic body so that the composite body is connected to the substrate 43.

At least a portion of the top of the first porous structure 41 is in contact with the positive electrode 44, at least a portion of the bottom of the second porous structure 42 is in contact with the top of the substrate 43, and the bottom of the substrate 43 is in contact with the negative electrode 45. The positive electrode 24 and the negative electrode 25 are made of metallic materials. The top of the negative electrode 45 is in close contact with the bottom of the substrate 43, and the bottom of the positive electrode 44 is in close contact with the top of the first porous structure 41.

The main difference with Embodiment 1 is: this Embodiment 2 uses the second porous structure 42 in the low porosity region to replace the solid structure intermediate structure of Embodiment 1. Although the intermediate structure of Embodiment 2 is a porous structure, its porosity is low and in a certain range, which can ensure that the second porous structure 42 maintains a certain contact area with the substrate 43, thus ensuring a certain welding efficiency. In principle, the smaller the porosity of the second porous structure 42, the higher the welding efficiency between the composite 4A and the substrate 43, and vice versa; at the same time, the final welding efficiency is also related to the specific spatial arrangement of the interconnected scaffolds (or beams) inside the porous structure, which can be designed according to the actual application.

The above-mentioned second porous structure 42 may also have a bumps 421 on the lower surface, as shown in FIG. 4b. When an electric current passes through the first porous structure 41 and the second porous structure 42, the contact between the bumps 421 of the second porous structure 42 and the top of the substrate 43 generates resistance heating so that the bottom of the second porous structure 42 forms a bonded metallic body with the top of the substrate 23, thus bonding tightly the composite 4A formed by the first porous structure 41 and the second porous structure 42 to the substrate 43 .

### Embodiment III

Based on the above-mentioned Embodiment I, this Embodiment III not only provides a non-porous base plate 812 (or a porous structure with low porosity) located between the porous surface structure 811 and the substrate 813, but also some insertion portions, and a plurality of raised structures prefabricated on the bottom surface of the non-porous base plate 812, which are in contact with the top of the substrate 813. Optionally, the said insertion portions comprise multiple support posts 816a. As shown in Figure 5a, the support posts 816a are placed on the surface of the non-porous base plate 812 proximal to the porous surface structure, and located between the non-porous base plate 812 and the positive electrode 814. The support posts 816a are located inside the porous surface structure 811, the top of the support posts 816a is substantially flush with the top of the porous surface structure, and the height of the support posts 816a is substantially equal to the height of the porous surface structure. Likewise, the top of the negative electrode 815 in this Embodiment III is in close contact with the bottom of the substrate 813. Of course the direction described here is the orientation shown in the illustration, and the above orientation provision is used only for illustration and not necessarily as the orientation in actual application, and this applies to the provisions of subsequent related embodiments therewith.

In this embodiment, the support posts 816a are good conductive solid structure. Each of the support posts 816a is spatially aligned in correspondence with the raised structures underneath, such that the area covered by the support posts 816a coincides at least partially with the contact portion between the raised structures and the substrate 813, and the size of the support posts 816a matches that of the raised structures.

Optionally, the non-porous base plate 812, the porous surface structure 811, and the support posts 816a are integrally formed, with manufacturing processes such as 3D printing or vapor phase deposition.

In this Embodiment , although the surface of the porous surface structure 811 is still in partial contact with the positive electrode 814 above it, the support posts 816a are good conductive solid structure, while the porous surface structure 811 contains numerous pores, most of the current from the positive electrode 814 flows preferentially through the solid structure of the good conductive support posts 816a, thereby not only greatly reducing the resistance-heat damage to the porous surface structure 811, but also enhancing the efficiency of the current conduction and consequently increasing the welding efficiency and ensuring the welding strength between the non-porous base plate 812 and the substrate 813. The structure of the insertion portion of this Embodiment is not limited to the above-mentioned support post, and any structural forms capable of achieving the corresponding technical effects of the Embodiment fall within the scope of protection of the present invention; furthermore, the support post in this Embodiment is not limited to solid body, but can also be a porous structure with high solid volume fraction (e.g., higher solid volume fraction than the porous surface structure). This is also applicable to each subsequent Embodiment and will not be repeated in this invention.

### Embodiment IV

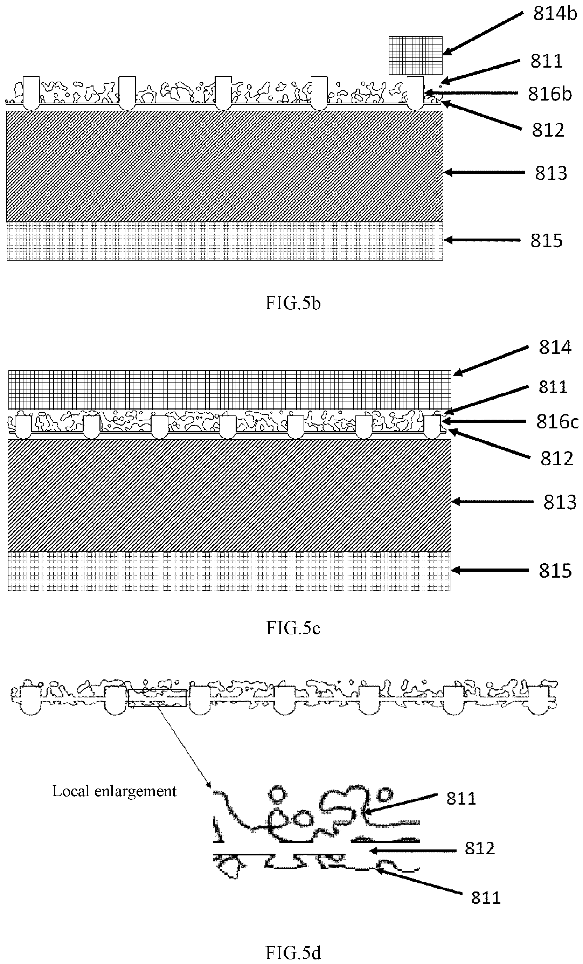

As a variant of Embodiment III, the variation of this Embodiment IV is that, in order to completely avoid damage to the surface of the porous surface structure caused by the resistive heat generated by the contact between the porous surface structure and the positive electrode above it, as shown in FIG. 5b, in this Embodiment IV, the top surfaces of all the support posts 816b are set higher than the top surface of the porous surface structure, and each support posts 816b is higher than its corresponding adjacent portion of the porous surface structure, therein the positive electrode will firstly contact the highly positioned surfaces of the support posts 816b, thus avoiding the direct contact of the positive electrode with the lowly positioned surface of the porous surface structure 811.

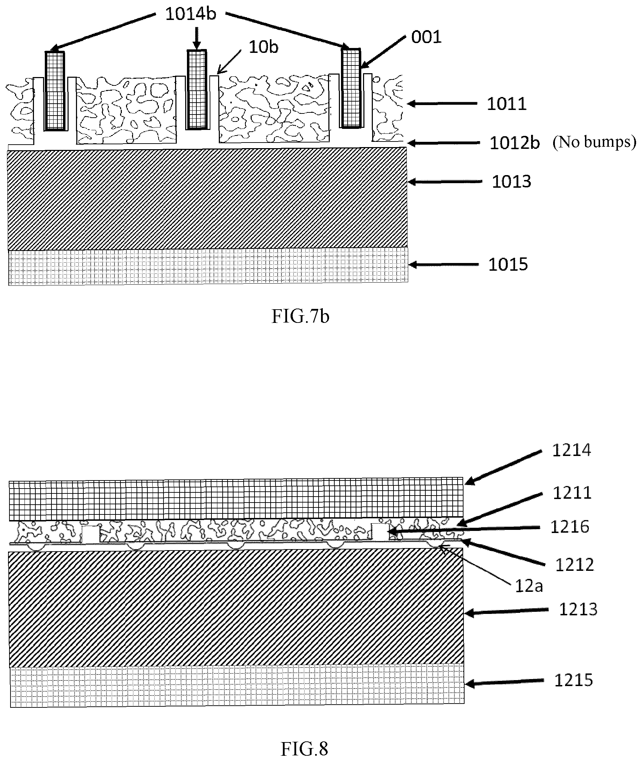

Since the surfaces of the support posts 816b are positioned higher in this Embodiment than the surface of the porous surface structure, in order to ensure the basic function of the entire connecting structure, the portions of the support posts 816b proud of the surface of the porous surface structure 811 can be removed by cutting or any other means after completing the welding process to ensure a flat surface. Further, as shown in Figure 8b, the positive electrode can not only be a continuous large planar positive electrode as shown in Figure 5b, but also a plurality of segmented positive electrodes 814b, each segment of positive electrodes 814b being pressed on the top of the corresponding support posts 816b, and the positive electrodes 814b are connected in parallel to a large planar electrode or directly to the positive end of the power supply.

### Embodiment V

Based on the above embodiments III and IV, this Embodiment V is further expanded in that: the top surface of each support posts 816c is lower than that of the corresponding porous surface structure, as shown in Figure 5c. The height of each support posts 816c is less than that the porous surface structure, and hence the said support posts 816a are hidden inside the porous surface structure 811, i.e., there is porous structure above support posts 816c. In this way, the positive electrode 814 will contact firstly the surface of the porous surface structure 811, resulting in some degrees of sinking of the top surface of the porous surface structure 811 under resistance heating until the top position of the support posts 816c in extreme (the maximal amount of sinking is limited, hence the sunk position is higher than the top position of the support posts). Because the support posts 816c is a solid structure, the support posts 816c acts as a position limit to ensure that the height of the final porous surface structure does not reduce below the top surfaces of the support posts so as to avoid excessive compression of the porous surface structure. Optionally, the top surfaces of the support posts 816c can also be a recessed concave structure, so that the top surfaces of the support posts 816c are lower than the top surface of the corresponding portion of the porous surface structure, therein the said support posts 816c can act as position limits.

Exemplarily, the non-porous base plate 812, the porous surface structure 811, and the support posts 816c may be manufactured as an integral structure through varieties of processes, such as 3D printing additive manufacturing and vapor phase deposition.

In this Embodiment , although the surface of the porous surface structure 811 is still in partial contact with the positive electrode 814 above it, the support posts 816c are good conductive solid structure, while the porous surface structure 811 contains numerous pores, most of the current flows preferentially through the solid structure of the good conductive support posts 816c to the raised structures of the intermediate structure and finally to the substrate 813, thereby not only ensuring the bonding strength between the non-porous base plate 812 and the substrate 813, but also greatly reducing the resistance-heat damage to the porous surface structure 811. Although this Embodiment V may still result in a certain degree of damage to the surface of the porous surface structure, as the top surfaces of the support posts 816c are always lower than the surface of the porous surface structure 811, the applicability of such a connected structure is not fundamentally affected.

Based on the embodiments shown in FIG. 5b and FIG. 5c, in another Embodiment (not shown schematically), a set position is selected along the height of each support posts, which is proud of the surface of the porous surface structure 811, and the portion of the support posts between the selected position and the top surface of the support posts is more porous than the rest of the support posts. The support posts overall are therefore not flush with the top surface of the porous surface structure shown in FIG. 5b. In this case, the positive electrode firstly contacts with the top porous portion of the support posts, leading to a small amount of sinking under contact pressure and resistance heating. The support posts collapses to the above set position to be basically flush with the adjacent porous surface structure (the maximum extent of sinking is up to the set position, and when the degree of sinking is not large, the sunk position is higher than the set position). In this case, the damage to the surface of the porous surface structure caused by resistive heat from contact with the positive electrode is completely avoided, and no additional processing is required to remove the excess portion of the support posts above the porous surface structure.

FIG. 5d shows a schematic diagram of the structure of the porous surface structure with a non-porous base plate. In one Embodiment , a plurality of support posts are provided within the porous surface structure, the top of each support post is lower than the top surface of the corresponding portion of the porous surface structure, and the support posts are hidden inside the porous surface structure 811. The non-porous base plate 812, the porous surface structure 811, and the support posts may be integrally formed, such as realized by a 3D printing additive manufacturing process, or a vapor phase deposition process, etc. Based on this integral forming process, a portion or all of the non-porous base plate 812 can be formed within the porous surface structure 811, with most of the porous surface structure 811 being located above the non-porous base plate 812 and a small portion of the porous surface structure 811 being located below the non-porous base plate 812, as shown in Figure 5d. The design principle that some or all of the non-porous base plate in this Embodiment can be formed within the porous surface structure is not limited to FIG. 5d, but also applies to embodiments in which the composite has a non-porous base plate and a porous surface structure, and will not be repeated here.

### Embodiment VI

For the above Embodiment III, a non-porous base plate 812 (or a low porosity porous structure) is provided between the porous surface structure 811 and the substrate 813, and a plurality of raised structures are prefabricated on the bottom surface of the non-porous base plate 812, and the raised structures are in contact with the top of the substrate 813, and a plurality of good conductive solid support posts 816a are provided on the surface of the non-porous base plate 812 facing the porous surface structure, being placed between the non-porous base plate 812 and the positive electrode 814.

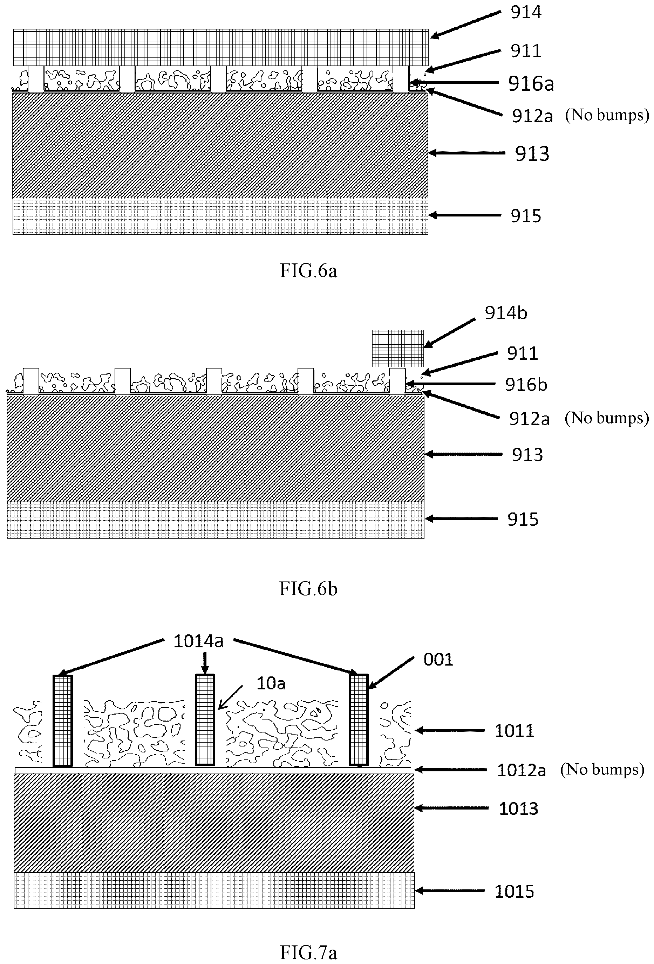

The main difference from Embodiment III is that the non-porous base plate 912a provided between the porous surface structure 911 and the substrate 913 in this Embodiment VI does not have raised structures (e.g., bumps) as described above on its bottom surface, but has a plurality of good conductive support posts 916a provided on its surface proximal to the porous surface structure, with the support posts 916a situated between the non-porous base plate 912a and the positive electrode 914, as shown in FIG. 6a. The bottom surface of the non-porous base plate 912a is primarily in a planar contact with the substrate 813.

Exemplarily, the support posts 916a are located inside the porous surface structure 911, their surfaces being substantially flush with the top surface of the porous surface structure, and the heights of the support posts 916a being substantially equal to the height of the porous surface structure, and similarly, the top of the negative electrode 915 being in direct contact with the bottom surface of the substrate 913.

Exemplarily, the non-porous base plate 912, the porous surface structure 911, and the support posts 916a are formed as an integral structure through processes such as 3D printing additive manufacturing or vapor phase deposition.

In this Embodiment VI, although the surface of the porous surface structure is still partially in contact with the positive electrode 914, the support posts 916a are solid structures with good conductivity, and the porous surface structure 911 has numerous pores, so that most of the current flows from the electrode to the support posts 916a of the solid structure with good conductivity and then to the non-porous base plate 912a and eventually to the substrate 913. Even if the bottom surface of the non-porous base plate 912a does not have prefabricated raised structures, this Embodiment has set up a plurality of good conductive pillar-shaped support posts 916a. Consequently, there is still enough electrical current flow and sufficient resistance heating to provide sufficient welding strength between the non-porous base plate 912a and the substrate 913, while reducing the thermal damage to the surface of the porous surface structure.

### Embodiment VII

As a variant of Embodiment VI, this variation Embodiment VII is that: as shown in Figure 6b, in addition to the features of Embodiment VI regarding a non-porous base plate (or a porous structure with low porosity) without a raised structures, in this Embodiment VII, all of the support posts 916b are specifically set with their top surfaces being proud of the adjacent porous surface structure and their heights more than the height of the adjacent porous surface structure, so as to completely avoid surface damage of the porous surface structure 911 due to contact resistance heat generation. The positive electrode in this Embodiment will firstly contact the support posts 916b, thus avoiding the positive electrode from making contact with the lower positioned porous surface structure 911. In addition, since the support posts 916b sit proud of the adjacent porous surface structure, in order to ensure the proper function of the overall connecting structure, this part of the support posts 916b above the porous surface structure 911 can be made flush by cutting or alternative means after the welding is completed. Further, as shown in FIG. 6b, the positive electrode may not only be a continuous large planar positive electrode as shown in FIG. 6a, but also a segmented plurality of positive electrodes 914b, with each segment of positive electrodes 914b being pressed on the upper end of the corresponding support posts 916b, and the positive electrodes 914b being connected in parallel to a large planar electrode or directly to the positive terminal of the power supply.

### Embodiment VIII

Unlike Embodiment I, the positive electrode 1014a in this Embodiment VIII is not a large planar electrode in direct contact with the porous surface structure 1011, but can be multiple positive electrodes 001 from dividing the positive electrode 54, with the positive electrodes 001 being inserted into the gaps 10a within the porous surface structure 1011 in the vertical direction, as shown in Figure 7a, on top of the non-porous base plate 1012a (or low porosity porous structure). Exemplarily, a plurality of the said positive electrodes 001 are connected in parallel and are all connected to a large planar electrode or directly to the positive terminal of the power supply, and the negative electrode 1015 is connected at the negative terminal of the power supply.

Optionally, the porous surface structure 1011 and the non-porous base plate 1012a are integrally formed, through processes such as 3D printing additive manufacturing, or vapor phase deposition etc.

As shown in FIG. 7a, the gaps 10a within the porous surface structure 1011 serve as insertion spaces for the corresponding positive electrodes 001, and the gaps 10a are prefabricated. The bottom end of the positive electrode 1014a in this Embodiment is not in contact with the top of the porous surface structure 1011 in order to avoid resistance heat damage to the surface of the porous surface structure 1011. In this case, the gaps 10a have clearance fits laterally with the positive electrodes 001, i.e., the gaps 10a are fabricated to ensure that the inserted positive electrodes 001 are not in contact with the porous surface structure of the adjacent part. This will avoid thermal damage to the porous surface structure.

Raised structures (e,g. bumps) are provided in Embodiment I to the non-porous base plate to achieve large contact resistance and hence to generate a large amount of resistance heat. In this Embodiment VIII, the bottom of the non-porous base plate 1012a is not provided with a raised structures, but the positive electrodes 1014a are in direct contact with the non-porous base plate 1012a, and each positive electrode 001 is connected to the power supply separately. Once connected, the current flows directly from the positive electrodes 001 to the non-porous base plate 1012a and the substrate 1013 (bypassing the porous surface structure 1011), thereby ensuring sufficient electrical current flow and resistance heating to achieve sufficient welding strength through effective welding of the non-porous base plate 1012a and the substrate 1013.

### Embodiment IX

As a variant of Embodiment VIII, the variation this Embodiment IX brings is that: a number of support structures 10b of good conductive solid structures are provided on the top surface of the non-porous base plate 1012b (or porous structure of low porosity), and the support structures 10b are placed in the gaps preformed inside the porous surface structure 1011, as shown in Figure 7b. The said support structures 10b are used to place and support individual positive electrodes 001 of the positive electrode 1014a, respectively, and the positive electrodes 001 are fitted into the recesses opened in the support structure 10b to ensure good contacts between all positive electrodes 001 and the corresponding support structure 10b.

Exemplarily, the non-porous base plate 1012b, the porous surface structure 1011, and the support structures 10b are formed into an integral structure through processes such as 3D printing additive manufacturing or vapor phase deposition etc.

Optionally, the top of the support structure 10b is substantially flush with the top of the porous surface structure 1011, and the height of the support structure 10b is substantially equal to the height of the porous surface structure 1011; or, the top of the support structure 10b is lower than the top of the porous surface structure 1011; or, the top of the support structure 10b is higher than the top of the porous surface structure 1011 and, with the aid of a subsequent cutting process, the top of the final support structure 10b is flush with the top of the porous surface structure 1011. Or, the top of the support structure 10b is higher than the top of the porous surface structure 1011 and with the subsequent cutting process, the top of the final support structure 10b is flush with the top of the porous surface structure 1011. The present invention does not limit the choice of the height design of the support structures. Similarly, in this Embodiment , even if the bottom of the non-porous base plate 1012b is not provided with raised structures, the positive electrode 1014a is connected to the non-porous base plate 1012b through the good conductive solid support structures 10b, and each of the positive electrodes 001 is respectively connected to the power supply, the current flows directly from the positive electrodes 001 and passes through the non-porous base plate 1012b and substrate 1013 (bypassing the porous surface structure 1011) so as to ensure sufficient electrical current flow and resistance heating and sufficient welding strength between non-porous base plate 1012b and substrate 1013.

### Embodiment X.

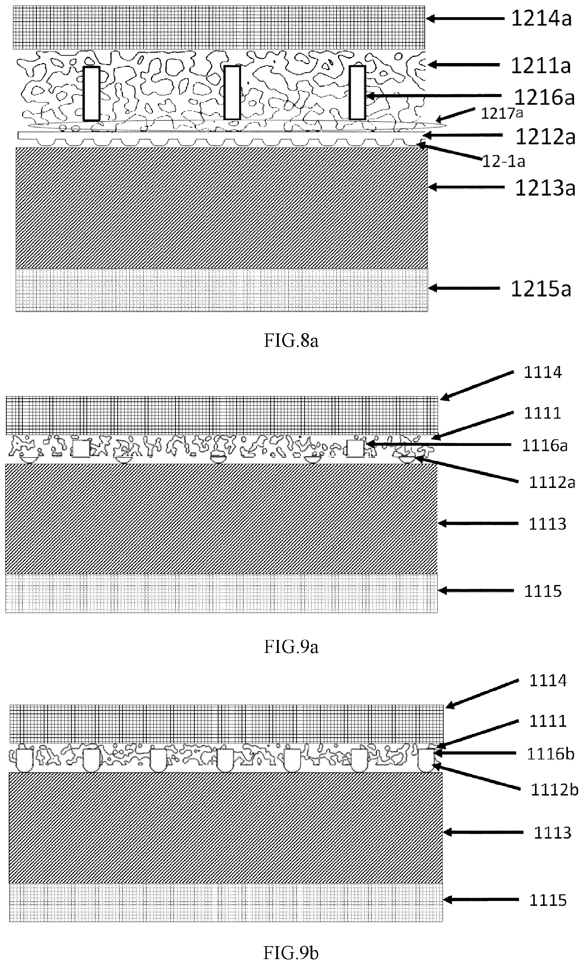

As shown in FIG. 8, this Embodiment X is based on Embodiment I. Further a number of support posts 1216 are provided between the non-porous base plate 1212 (or low porosity porous structure) and the positive electrode 1214, and the support posts 1216 are placed on the surface of the non-porous base plate 1212 proximal to the porous surface structure 1211. Optionally, the top surfaces of the support posts 1216 are lower than the top surface of the corresponding portion of the porous surface structure, the heights of the support posts 1216 are lower than the height of the porous surface structure, and the support posts 1216 are hidden inside the porous surface structure 1211. Similarly, the bottom side of the non-porous base plate 1212 in this Embodiment X has a plurality of prefabricated raised structures 12a in contact with the top surface of the base 1213.

In this Embodiment X, the positive electrode 1214 will firstly contact with the top surface of the porous surface structure 1211 , which will sink to a certain extent due to the contact resistance heat damage until reaching the top surfaces of the support posts 1216 (the maximum amount of the sinking possible), or a little above when the amount of sinking is less. As the support posts 1216 are solid structures, they play the position limit role to ensure that the height of the final porous surface structure to be no less than the heights of the posts, thereby avoiding the excessive compression of the porous surface structure.

Exemplarily, the support posts 1216 can be staggered or aligned with the corresponding raised structures 12a underneath. In addition, the present invention does not limit whether the support posts 1216 are conductive or not, so long as to ensure their position limit function for preventing the porous surface structure from excessive compression. When the support posts 1216 are good conductive structures, the current flows preferentially through them and then through adjacent porous structures to the raised structures 12a. In this way, the contact resistance heat damage to the surface of the porous surface structure can be minimized. When the support posts 1216 are non-conductive, the current flows from positive electrode 1214 to the porous surface structure 1211 and then directly to the raised structures 12a. In this case, even though a certain degree of thermal damage may still occur to the surface of the porous surface structure, because the support posts 1216 are always lower than the surface of the porous surface structure 1212, the basic function of the entire connecting structure is not affected in actual field of application.

As a variation of this Embodiment X, the variation is as follows:
As shown in FIG. 8a, the intermediate in the composite contains not only a non-porous base plate 1212a (or a porous structure with low porosity), but also an insertion portion, the said insertion portion being placed at any position within the porous surface structure 1211a. Wherein, the non-porous base plate 1212a is provided between the porous surface structure 1211a and the substrate 1213a, the said non-porous base plate 1212a having a plurality of raised structures 12-1a prefabricated on the bottom surface of the said non-porous base plate 1212a, the raised structures 12-1a being in contact with the top of the substrate 1212a.

Preferably, the said insertion portion comprises a solid structure and good conductive support post 1216a, the bottom end of the said support post 1216a is not in direct contact with the top of the substrate 1213a, a portion of the porous surface structure 1217a is distributed between the bottom end of the support post 1216a and the top of the substrate 1213a, the top of the negative electrode 1215a is in close proximity to the bottom of the substrate 1213a, and The bottom of the positive electrode 1214a is abutted against the top of the porous surface structure 1211a. Wherein, the top of the support post 1216a may be lower than or equal to or higher than the top of the porous surface structure 1211a, which is not limited by the present invention, with specific reference to the above, and will not be repeated herein.

This Embodiment can use the limiting effect of the support post 1116a to avoid excessive compression of the porous surface structure, because the positive electrode 1214a will contact with the top surface of the porous surface structure 1211a below it first, and then the surface of the porous surface structure 1211a will sink a little due to the damage caused by the heat generated by the contact resistance, until it sinks to the top of the support post (the maximum sinking degree can only sink to the top position). The support post 1216a is a solid structure, the support post 1216a plays a limiting role to ensure that the height of the final porous surface structure 1211a surface reaches the height of the support post.

Preferably, the non-porous base plate, the porous surface structure, and the support post are one-piece structures, such as achieved by a 3D printing additive manufacturing process, or a vapor phase deposition process, etc.

In addition, since the support post is a solid structure with good electrical conductivity, this Embodiment also enables the current to pass mostly preferably through the support post 1216a, then through a portion of the porous surface structure 1217a below the support post 1216a, and finally to the substrate 1213a, which also improves the damage to the surface of the porous surface structure caused by contact resistance heating, and also enhances the current conduction Role, a part of the porous surface structure 1217 below the support post 1216a and the substrate 1213a welding welding efficiency increased to ensure sufficient welding strength; the structure of the insertion part of this Embodiment is not limited to the above-mentioned support post, as long as the corresponding technical effect of the Embodiment of the structural form as the scope of protection of the present invention.

### Embodiment XI

Unlike Embodiment V, this Embodiment XI does not provide a non-porous base plate between the porous surface structure 1111 and the substrate 1113. The improvement is that: at least a part of bottom surface of the porous surface structure 1111 has raised structures of good conductive solid 1112a (such as bumps) and the raised structures 1112a are in contact with the top of the substrate 1113, as shown in Figure 9a. And good conductive solid support posts 1116a may be provided at any locations within the interior of the porous surface structure 1111.

The support posts 1116a and the raised structures 1112a in this Embodiment can be staggered (not aligned with each other), as shown in Figure 9a.

Exemplarily, the porous surface structure 1111, the raised structures 1112a, and the support posts 1116a are integrally formed through processes such as 3D printing additive manufacturing or vapor phase deposition etc.

Optionally, the support posts 1116a are concealed within the porous surface structure 1111, the top of each of the support posts 1116a is lower than the top of the porous surface structure 1111, and the bottom of the support posts 1116a is higher than the bottom of the porous surface structure 1111.

In this Embodiment , the position limit function of the support posts 1116a can be used to avoid excessive compression of the porous surface structure 1111, because the positive electrode 1114 will firstly contact with the top surface of the porous surface structure 1111 , which will sink to a certain extent due to the contact resistance heat damage until reaching the top surfaces of the support posts 1116a (the maximum amount of the sinking possible), or a little above when the amount of sinking is less. As the support posts 1116a are good conductive solid structures, the support posts 1116a play the position limit role to ensure that the height of the final porous surface structure to be no less than the heights of the support posts; in addition, as the support posts 1116a are good conductive solid structures, the current flows preferentially through the support posts 1116a and some through porous surface structure near the support posts 1116a to reach the raised structures 1112a. In this way, the contact resistance heat damage to the surface of the porous surface structure can be minimized. Furthermore, in this Embodiment , the raised structures 1112a are used to increase the contact resistance with the substrate 1113 so as to generate sufficient resistance heat and achieve sufficient welding strength between the raised structures 1112a and the substrate 1113. The raised structure in this Embodiment can be a solid body or a porous structure with high solid volume fraction (e.g. higher solid volume fraction than the porous surface structure), which is also applicable to the subsequent embodiments and will not be repeated in the present invention.

### Embodiment XII

The above Embodiment XI describes that the support posts 1116a are misaligned with the raised structures 1112a. As a variant of Embodiment XI, this Embodiment XII designs the raised structures 1112b to align with the support posts 1116b, with the two at least partially overlapping (e.g., partially overlapping or completely overlapping), as shown in Figure 9b.

Exemplarily, the porous surface structure 1111, the raised structures 1112b, and the support posts 1116b are integrally formed through processes such as 3D printing additive manufacturing or vapor phase deposition, etc.

In this Embodiment XII, the support posts 1116b are concealed inside the porous surface structure 1111, with their top surfaces being lower than the top surface of the porous surface structure 1111, and their heights being lower than the height of the porous surface structure. The said raised structures 1112b are in contact with the top of the substrate 1113.

In this Embodiment , it is also possible to use the support posts 1116b to avoid the excessive compression of the porous surface structure 1111, because the positive electrode 1114 will firstly contact with the top surface of the porous surface structure 1111 , which will sink to a certain extent due to the contact resistance heat damage until reaching the top surfaces of the support posts 1116b (the maximum amount of the sinking possible), or a little above when the amount of sinking is less. As the support posts 1116b are good conductive solid structures, the support posts 1116b play the position limit role to ensure that the height of the final porous surface structure to be no less than the heights of the support posts; in addition, as the support posts 1116b are good conductive solid structures, the current flows preferentially through the support posts 1116b to reach the raised structures 1112b. In this way, the contact resistance heat damage to the surface of the porous surface structure can be minimized. Furthermore, in this Embodiment , the raised structures 1112b are used to increase the contact resistance with the substrate 1113 so as to generate sufficient resistance heat and achieve sufficient welding strength between the raised structures 1112b and the substrate 1113. It is worth noting that the welding efficiency of this Embodiment XII is better than Embodiment XI because the raised structures 1112b align with the support posts 1116b and the current flows through the support posts 1116b and then directly to the raised structures 1112b, while in Embodiment XI the current flows through the support posts 1116b and then needs to pass through the porous surface structure before reaching the raised structures 1112a.

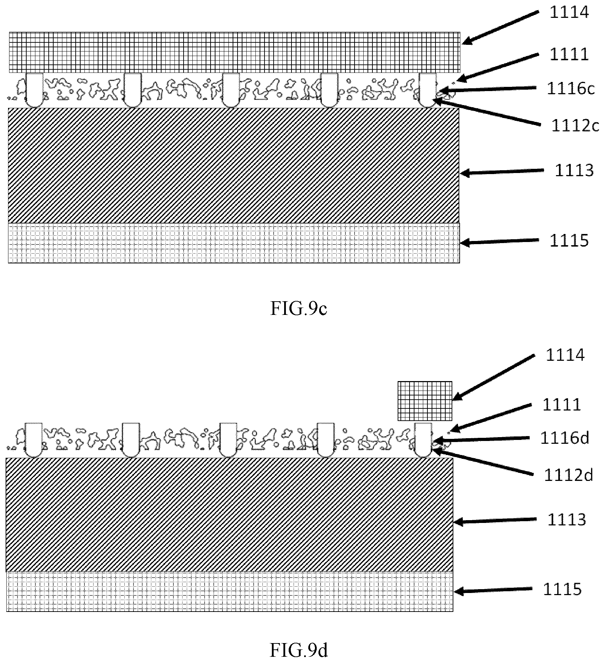

As a variant of this Embodiment XII, this variation is to change the above design where the top surfaces of the support posts are below the surface of the porous surface structure to the following: the support posts 1116c are located inside the porous surface structure 111 and their top surfaces are substantially flush with the top of the porous surface structure, and their heights are substantially equal to the height of the porous surface structure, and the raised structures 1112c align with the support posts 1116c with at least partial overlapping (e.g., partial overlapping or complete overlapping), as shown in FIG. 9c.

Similarly, as another variant of this Embodiment XII, this variation is to change the above design where the top surfaces of the support posts are below the surface of the porous surface structure to the following: the top surfaces of all the support posts 1116d are set above the top surface of the porous surface structure, as shown in FIG. 9d, and the heights of support posts 1116d are more than the height of the adjacent porous surface structure. And the raised structures 1112d also align with the support posts 1116d with at least partially overlapping (e.g., partially overlapping or completely overlapping), as shown in FIG. 9d. Exemplarily, the porous surface structure 1111, the raised structures 1112d, and the support posts 1116d are integrally formed with processes such as 3D printing additive manufacturing, or vapor phase deposition etc. Other elements of this variation can be referred to those in Embodiment IV and XII above, and will not be repeatedly described herein.

Embodiments of the present invention in which the intermediate does not contain a non-porous base plate are also shown in the following Embodiment s, in addition to those described in Embodiments XI and XII above.

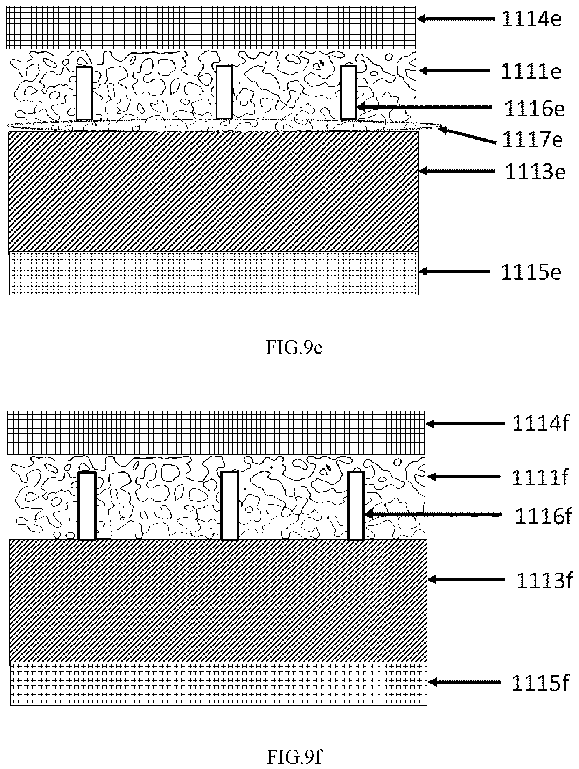

(1) In one Embodiment, as shown in FIG. 9e, a non-porous base plate is not provided between the porous surface structure 1111e and the substrate 1113e, and the intermediate comprises only an insertion portion, the said insertion portion being placed at any position inside the porous surface structure 1111e. Preferably, the said insertion portion comprises a good conductive solid support post 1116e, the bottom end of the said support post 1116e is not in direct contact with the top surface of the substrate, a portion of the porous surface structure 1117e is distributed between the bottom end of the support post 1116e and the top surface of the substrate 1113e, the top of the negative electrode 1115e is in close contact with the bottom of the substrate 1113e, the bottom of the positive electrode 1114e is in close contact with the top surface of the porous surface structure 1111e. Wherein, the top of the support post 1116e may be lower than or equal to or higher than the top of the porous surface structure 1111e, which is not limited by the present invention, and the details of which are referred to above and will not be repeated herein. As shown in FIG. 9e, the side of the support post 1116e proximal to the substrate 1113e is not provided with a raised structure, and the bottom end of the support post 1116e is higher than the bottom end of the porous surface structure.

This Embodiment can use the limiting effect of the support post 1116e to avoid the porous surface structure from being compressed excessively, because the positive electrode 1114e will contact with the top surface of the porous surface structure 1111e first, and then the surface of the porous surface structure 1111e may sink somewhat due to the damage caused by the heat generated by the contact resistance, until it sinks to the top of the support post (the thermal sinking is limited by the top surface of the support post). The support post 1116e is a solid structure, the support post 1116e plays a limiting role to ensure that the surface of the final porous surface structure 1111e is no lower than the top surface of the support post.

In addition, because the support post is a solid structure with good electrical conductivity, this Embodiment also enables the current to pass most preferably through the support post 1116e, then through a portion of the porous surface structure 1117e below the support post 1116e, and finally to the substrate 1113e, which also improves the damage to the surface of the porous surface structure caused by contact resistance heat generation, and also enhances the current conduction. A part of the porous surface structure 1116e around the support post 1117e and the substrate 1113e achieves high welding efficiency to ensure sufficient welding strength; the structure of the insertion portion of this Embodiment is not limited to the above-mentioned support post, as long as the corresponding technical effect of the Embodiment is achieved, all of which fall within the scope of protection of the present invention.

(2) In another Embodiment , as shown in FIG. 9f, a non-porous base plate is not provided between the porous surface structure 1111f and the substrate 1113f, and the intermediate comprises only an insertion portion, the said insertion portion being placed at any position inside the porous surface structure 1111f. The surface of support post 1116f proximal to the substrate 1113f is not provided with a raised structure. Preferably, the said insertion portion comprises a good conductive solid support post 1116f, the bottom end of the said support post 1116f is in direct contact with the top surface of the substrate 1113f, the top of the negative electrode 1115f is in close contact with the bottom of the substrate 1113f, the bottom of the positive electrode 1114f is in close contact with the top surface of the porous surface structure 1111f. Wherein, the top of the support post 1116f may be lower than or equal to or higher than the top of the porous surface structure 1111f, which is not limited by the present invention, and the details of which are referred to above and will not be repeated herein.

This Embodiment also makes use of the limiting effect of the support post 1116f to avoid excessive compression of the porous surface structure 1111f, as described above; although the porous surface structure 1111f is not provided with a raised structure between the porous surface structure 1111f and the substrate 1113f, as the support post 1116f is a good conductive solid structure, the electric current most preferably passes through the support post, and the current flowing through the support post 1116f to the porous surface structure adjacent to the bottom of the support post 1116f is high to ensure there is sufficient resistance heating for a sufficient welding strength to be achieved between the composite body and the substrate 1113f, while also reduce somewhat the thermal damage to the surface of the porous surface structure.

It should be noted that in the above EmbodimentsIII to XII, the support post is defined as part of the intermediate, and this is only one of the conceptual definition, but it should not be limited to this. Because the non-porous base plate, porous surface structure and the support post can be integrally formed, one can also define the support post in the above EmbodimentsIII to XII as a part of the porous surface structure. This also falls into the scope of protection of the present invention, the details of which can be referred above, and will not be repeated herein.

### Embodiment XIII

For example 1 above, the top of the negative electrode 25 is pressed against the bottom of the substrate 23, and the bottom of the positive electrode 24 is pressed against the top of the porous surface structure 21; optionally, the positive electrode 24 and the negative electrode 25 are large planar electrodes and the positive electrode 24 covers the top of the porous surface structure 21, and the negative electrode 25 is pressed against the bottom of the substrate 23. Since the large planar positive electrode 24 of Embodiment I is pressed on the top of the porous surface structure 21, the surface contact and compression between the large planar positive electrode 24 and the surface of the porous surface structure 21 lead to mechanical and thermal damage to the surface of the porous surface structure 21, such as darkening, denting, and pore collapsing.

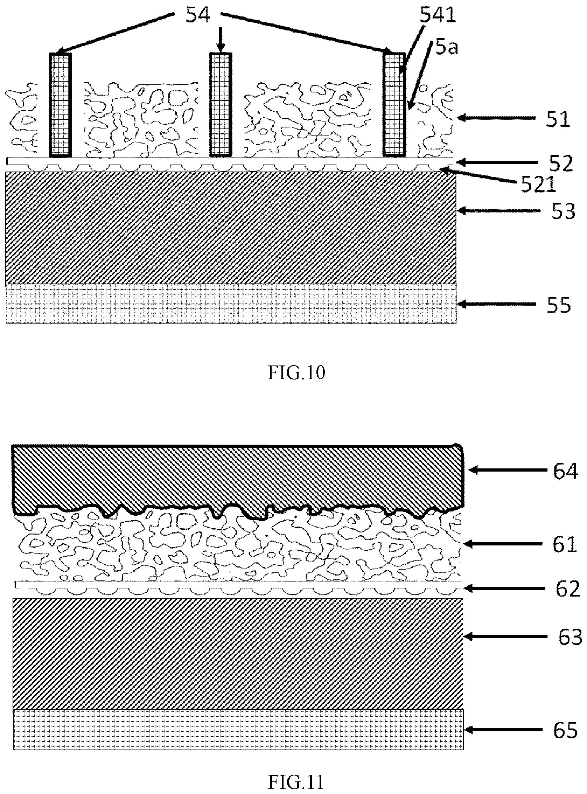

In order to protect the surface of the porous surface structure, the positive electrode 54 in this Embodiment XIII may not use a large planar electrode to be in contact with the porous surface structure 51 but instead the positive electrode is divided into multiple positive electrode monomers 541 which are inserted vertically into the gaps 5a inside the porous surface structure 51, and the positive electrode monomers 541 are placed on the top of the non-porous base plate 52 (as an intermediate structure), as shown in FIG. 10. Again, the porous surface structure 51 and the non-porous base plate 52 in this Embodiment are integrally formed, such as through 3D printing additive manufacturing process, or a vapor phase deposition process, etc. The materials and fabrication processes of the substrate 53, the non-porous base plate 52, and the porous surface structure 51 in this Embodiment can be found in Embodiment 1, and will not be elaborated in more details herein.

In this embodiment, the individual positive electrodes 541 are connected in parallel to the positive terminal of the power supply, and the negative electrode 55 is connected to the negative terminal of the power supply. As shown in FIG. 10, the bottom of the non-porous base plate 52 is pre-fabricated with a plurality of raised structures 521 that are in contact with the top of the substrate 53, which is in contact with the negative electrode 55. Optionally, the gaps 5a within the porous surface structure 51 serve as insertion spaces corresponding to the positive electrodes 541. The said gaps 5a are prefabricated as parts of the porous surface structure, and gaps start from the surface of the porous surface structure 51 and extend to the top of the non-porous base plate 52, such that the top of the non-porous base plate 52 is exposed within the gaps 5a for the bottom surfaces of the inserted positive electrodes 541 to come in direct contact with the top of the non-porous base plate 52.

The positive electrode 54 of this Embodiment XIII does not come into direct contact with the surface of the porous surface structure 51, thereby effectively resolving the problem of thermal damage to the surface of the porous surface structure due to resistance heat from its contact with the positive electrode. Exemplarily, the gaps 5a fit with the positive electrodes 541 , with lateral clearance fits, i.e., the gaps 5a need to ensure that after insertion the positive electrodes 541 are spaced apart from the adjacent porous surface structure to avoid resistance heat damage to the surface of the porous surface structure. Optionally, the positive electrodes 541 are columns or structures of other shapes. This Embodiment does not specifically limit in this respect, nor in other embodiments of the present invention.

Exemplarily, a plurality of raised structures 521 on the bottom of the non-porous plate 52 may be placed to correspond to the positions of positive electrode 541. For example, the contact position between each of the positive electrodes 541 and the top of the non-porous plate 52 is directly above each of the raised structures 521 or in the area adjacent to the raised structures 521. This is arranged so as to ensure smooth conduction of electric current to the non-porous substrate 52 and then to the area of contact between the raised structures 521 and the top of the substrate 53, and the adjacent region, which generates resistance heat to form welding nuggets between the top of the raised structures 521 and the substrate 23. The shape of the raised structures 521 of this Embodiment can be seen in Embodiment 1 and will not be elaborated further herein. The variations in Embodiment XIII applies not only to Embodiment I, but also all other Embodimentsand their variations and will not be further elaborated herein.

It is worth noting that the division of the positive electrode into a plurality of positive electrode monomers and the insertion of the positive electrode monomers into the gaps inside the porous surface structure in this Embodiment XIII are also applicable to Embodiment II in which the intermediate structure is a second porous structure (less porous than the said porous surface structure), where similarly the positive electrode 44 in Embodiment II are replaced with a plurality of positive electrodes inserted vertically into the gaps inside the porous surface structure 41. The pre-formed gaps start from the surface of the first porous structure and pass through the first porous structure up to the upper surface of the second porous structure or into the interior of the second porous structure, such that portions of the second porous structure are exposed within the gaps 5a and the bottom surfaces of the inserted positive electrodes are in direct contacts with portions of the second porous structure. Similarly, lateral clearance fits exit between the gaps and the inserted positive electrodes, and the gaps ensure that inserted positive electrodes are spaced apart from the porous surface structure to prevent the surface of the porous surface structure from damages by resistance heating. All other specific structures and processes are the same as in Embodiment XIII and will not be repeated here.

### Embodiment XIV

For the above Embodiment I, the positive electrode 24 and the negative electrode 25 may be made of conductive materials (e.g., metallic materials); the top of the negative electrode 25 are in direct contact with the bottom of the substrate 23 and the bottom of the positive electrode 24 are in direct contact with the top of the porous surface structure 21; the positive electrode 24 and the negative electrode 25 are large planar electrodes, and the positive electrode 24 covers the top of the porous surface structure 21 and the negative electrode 25 covers the bottom of the substrate 23.

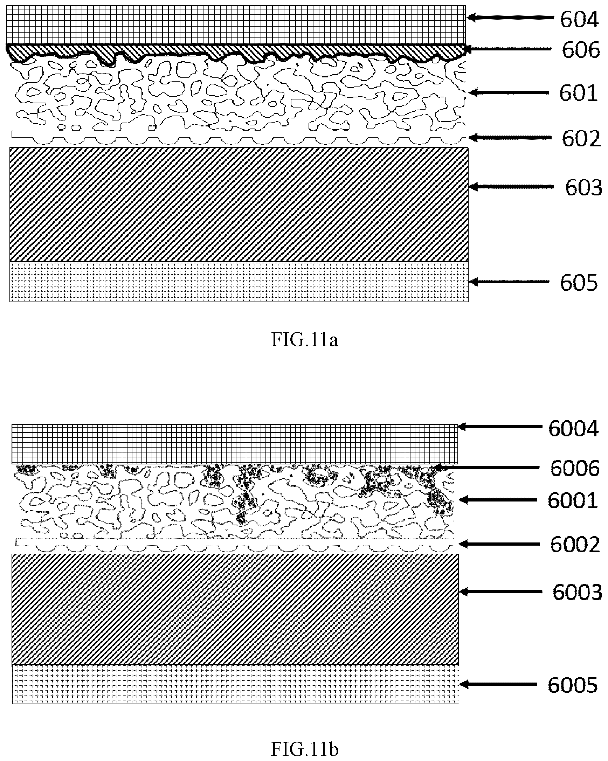

The main difference from Embodiment I is that the positive electrode in this Embodiment XIV is a flexible positive electrode 64, as shown in Figure 11. In this Embodiment XIV, the flexible positive electrode 64 is a large planar electrode and covers the top of the porous surface structure 61; the negative electrode 65 is in contact with the bottom of the substrate 63, and the non-porous base plate 62 is located between the porous surface structure 61 and the substrate 63. Exemplarily, the porous surface structure 61 and the non-porous plate 62 are integrally formed, For example, by a 3D printing additive manufacturing process, or a vapor phase deposition process, etc. The materials and fabrication processes of the substrate 63, the non-porous base plate 62, and the porous surface structure 61 of this Embodiment can be found in Embodiment 1 and will not be described in details here.

In this Embodiment XIV, since the flexible positive electrode 64 covers the top surface of the porous surface structure 61 and exerts a certain pressure on the surface of the porous surface structure 61, the flexible positive electrode 64 deforms flexibly under the applied pressure, which increases the contact area between the flexible electrode 64 and the top of the porous surface structure 61 (compared with the contact area between the rigid positive electrode and the top of the porous surface structure under the same conditions), which not only reduces the contact resistance between positive electrode 64 and porous surface structure 61, improves or avoids surface damage (such as denting, blackening, pore space reduction, etc.) of porous surface structure due to resistance heating, but also enhances current conduction, increases the welding efficiency between non-porous base plate 62 and substrate 63, and hence increases the welding strength.

Exemplarily, the flexible material is a conductive material, such as copper foil or tin foil, etc. This Embodiment is not limited to this or to other relevant Embodiment s, and can be designed according to the actual application. The variations in Embodiment XIV applies not only to Embodiment I, but also all other Embodimentsand their variations and will not be further elaborated herein.

The following is another variant of Embodiment XIV:
As shown in FIG. 11a, a deformable good conductive medium 606 is added between the bottom of the positive electrode 604 and the top of the porous surface structure 601, the said deformable good conductive medium 606 covering the top surface of the porous surface structure 601. Optionally, the said deformable good conductive medium 606 is in the form of a continuous solid film, such as a copper foil, etc. Similarly, the intermediate structure 602 is located between the porous surface structure 601 and the substrate 603, with the top of the negative electrode 605 in direct contact with the bottom of the substrate 603. The positive electrode 604 is a large planar electrode and covers the top surface of the deformable conductive medium 606. The contact area between the porous surface structure 601 and the porous conductive medium 606 increases due to the deformability of the porous conductive medium 606, which can not only reduce the contact resistance between the porous surface structure 601 and the positive electrode 604, hence reducing the resistance heat and surface damage of the porous surface structure 601, but also increase the current conduction, thereby increasing the welding strength between the intermediate structure 602 and the substrate 603.

Further variations to the above are as follows:
As shown in Figure 11b, the gap between the bottom of the positive electrode 6004 and the top of the porous surface structure 6001 can be filled with good conductive material powder 6006 (or good conductive wire or mesh material), which reduces the contact resistance between the positive electrode 6004 and the surface of the porous surface structure 6001, thus reducing the surface damage of the porous surface structure 6001, and also increasing the current conduction effect and the welding efficiency between the intermediate structure 6002 and the substrate 6003. Preferably, the material of the good conductive material powder 6006 (or good conductive wire/mesh) is the same as the material of the porous surface structure 6001, For example, titanium powder (or titanium wire/mesh). Similarly, as in FIG. 11b, the intermediate structure 6002 is disposed between the porous surface structure 6001 and the substrate 6003, with the top of the negative electrode 6005 in direct contact with the bottom of the substrate 6003. In a different Embodiment , the contact resistance between the electrode and the porous surface structure can likewise be reduced by spraying a conductive material on the surface of the porous surface structure, reducing surface damage to the porous surface structure, which will not be described in detail in the present invention.

The above deformable conductive medium 606, conductive material powder 6006 (or conductive wire/mesh), sprayed conductive material or liquid conductive medium, etc., need to be properly removed after the porous surface structure and the substrate are welded together to ensure that pores of the porous surface structure are open without blockages.

It should be noted that, after the porous surface structure is connected to the substrate in any of the above embodiments, the porous surface structure can be individually coated with a hydroxyapatite (HA) coating that is bioactive and biocompatible to facilitate the subsequent bone ingrowth process; alternatively, the porous surface structure can be individually coated with a coating containing antimicrobial silver ions or cell growth factors, etc.

Based on the above, the present invention also provides a variant Embodiment, as follows:
In order to avoid or lessen damage to the surface of a porous surface structure due to resistance heating, it is necessary to improve the electrical conductivity of the porous surface structure as much as possible for reducing the contact resistance between the porous surface structure and the electrode. In this variant Embodiment , a molten material (with superior conductivity) is poured into the porous surface structure and almost fills full the pores in a selected part (the upper part of a nonpermeable partition layer). This not only requires a low melting point of the said superior conductive material, but also needs to install a nonpermeable partition layer, preferably also made of conductive materials. The partition layer intends to prevent the molten material from penetrating downward and flowing into the intermediate structure below and incapaciting the effectiveness of resistance welding. After completing the resistance welding process, the whole assembly will be put into a high temperature environment. As the melting point of the specific conductive medium is lower than the porous surface structure and the substrate (such as titanium alloy), the high temperature environment will not affect the substrate, but the conductive medium with a low melting point will be melted, and the low melting point of the added conductive medium can be removed by some of the processes of the prior art.

### Embodiment XV

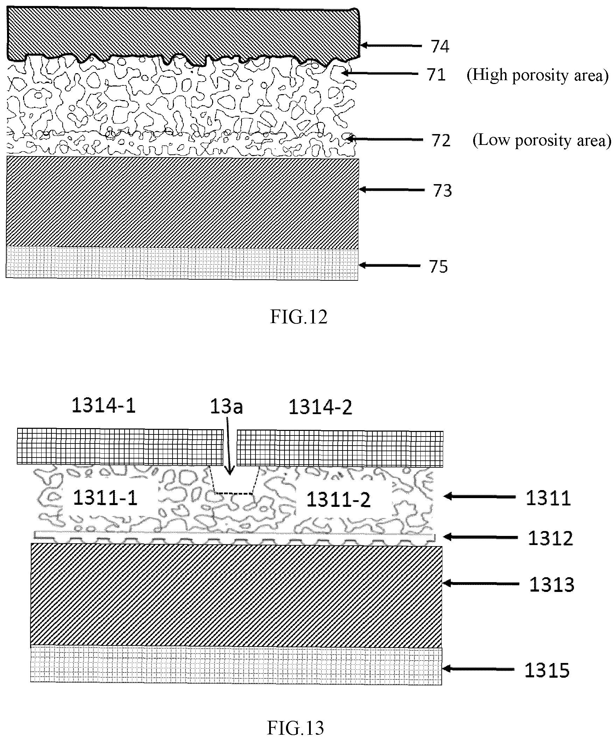

For the above-mentioned Embodiment II, the positive electrode 24 and the negative electrode 25 are made of conductive material (metallic material), the top of the negative electrode 45 is in direct contact with the bottom of the substrate 43, and the bottom of the positive electrode 44 is in direct contact with the top of the first porous structure 41 with high porosity. The main difference from Embodiment II is that the positive electrode of this Embodiment XV is a flexible positive electrode 74 made of a flexible material, not a metallic material as in the above embodiments, as shown in Figure 12.

In this Embodiment XV, the flexible positive electrode 74 is a large planar electrode and covers the top of the first porous structure 71 with high porosity, and the negative electrode 75 is in direct contact with the bottom of the substrate 73, and the second porous structure 72 with low porosity is located between the first porous structure 71 with high porosity and the substrate 73. Optionally, the first porous structure 71 and the second porous structure 72 are integrally formed, For example, by a 3D printing additive manufacturing process, or a vapor phase deposition process, etc. The materials and fabrication processes of the substrate 73, the second porous structure 72, and the first porous structure 71 in this Embodiment can be found in Embodiment 2, and will not be described in detail herein.

In this embodiment, the flexible positive electrode 74 covers the top surface of the first porous structure 71 and generates a certain pressure on the top surface of the first porous structure 71, at which time the flexible material of the flexible positive electrode 74 will produce a certain flexible deformation under the pressure, which increases the contact area between electrode 74 and the top of the first porous structure 71 (compared with the rigid electrode case) , thereby not only reducing the contact resistance between the positive electrode 74 and the porous surface structure 71, reducing or avoiding surface damage (e.g., denting, darkening, pore space reduction, etc.) on the porous surface due to resistance heating, and protecting the surface of the porous surface structure, but also enhancing current conduction, increasing the welding efficiency between the non-porous base plate 72 and the substrate 73, and increasing the welding strength.

Exemplarily, the flexible material is a conductive material, such as copper foil or tin foil, etc. This Embodiment is not limited to this or to other specific relevant Embodiment s, and can be designed according to the actual application.

Similar to Embodiment II, the lower surface of the low-porosity base plate 72 can have bumps to increase resistance welding efficiency. The variations in Embodiment XIV applies not only to Embodiment II, but also all other Embodimentsand their variations and will not be further elaborated herein.

### Embodiment XVI

In the present invention, the porous surface structure and the substrate are connected with resistance welding (e.g., projection welding). When the area of the workpiece to be welded is too large, a larger number of raised structures are required. The total current from the electrode needs to be increased to ensure the welding strength between each of the raised structures and the substrate, which may lead to an increase in the cost of the power supply equipment, electrode damage and thermal damage to the surface of the porous surface structure. In this case, the workpieces should be welded in divided regions and multiple batches.

In this Embodiment XVI, the porous surface structure 1311 is resistance welded with the substrate 1313 in different regions and batches, as shown in FIG. 13. A first positive electrode 1314-1 is in contact with the the first region of the porous surface structure 1311-1; and similarly a second positive electrode 1314-2 with the second region of the porous surface structure 1311-2. The top surface of the negative electrode 1315 is in close contact with the bottom of the substrate 1313. A non-porous base plate 1312 (or a porous structure with low porosity) is placed between the porous surface structure 1311 and the substrate 1313, with a plurality of raised structures prefabricated on the bottom surface of the non-porous base plate 1312 and the raised structures being in contact with the top of the substrate 1313.

This Embodiment entails partitioned resistance welding where the surface of the porous surface structure 1311 is divided into multiple regions and separate welding occurs in each of the partitioned regions. The positive electrode may not be able to completely cover the particular region of the porous surface structure. For example, the edge formed between two adjacent regions may not be completely covered by the electrode, hence after welding is completed the edge of each partitioned welded region may be slightly higher than the welded region covered by the electrode (i.e., forming a convex edge). Then the surface of the porous surface structure 1311 after welding will be uneven, and the basic function of the connected structure for the actual application (e.g., bone ingrowth) may be adversely affected.

To resolve the above issue, the porous surface structure 1311 of this Embodiment XVI is provided with recesses 13a, which divide the top of the porous surface structure 1311 into multiple regions, such as the the first region of the porous surface structure 1311-1 and the second region of the porous surface structure 1311-2 in the figure. Each of the recesses 13a is in the form of an elongated strip, and the first region of the porous surface structure 1311-1 and the second region 1311-2 are located on each side of each of the elongated grooves 13a. The top surfaces of the recesses 13a are lower than the top of the porous surface structure 1311. The heights of the recesses 13a are less than the height of the porous surface structure 1311.

Exemplarily, the main body of the non-porous base plate 1312, the recesses 13a, and the porous surface structure 1311 are integrally formed through processes such as 3D printing additive manufacturing or vapor phase deposition etc. The said recesses 13a may also be formed by machining.

Figure 13 shows that there is a gap between the first positive electrode 1314-1 and the second positive electrode 1314-2. The first positive electrode 1314-1 and the second positive electrode 1314-2 can be used sequentially, when Figure 13 only represents the position schematics, or alternatively the first positive electrode 1314-1 and the second positive electrode 1314-2 are used simultaneously by coming into contact with corresponding regions of the porous surface structure 1311-1; and the positive electrodes are bigger in size than corresponding regions of the surface area of the porous surface structure 1311-1.

In this embodiment, a recess 13a is designed, where one side of the recess 13a near the first positive electrode 1314-1 is denoted as the first side, and the other side of the recess 13a near the second positive electrode 1314-2 is denoted as the second side.

In this embodiment, the first region of the porous surface structure 1311-1 is first connected to the substrate 1313 by resistance welding: the bottom surface of the first positive electrode 1314-1 covers the corresponding region of the porous surface structure 1311-1, and the part of the first positive electrode 1314-1 beyond the welding area does not exceed the edge of the second side of the recess 13a, and the first positive electrode 1314-1 and the first region of the porous surface structure 1311-1 are resistance welded, resulting in a small amount of resistance heating sinkage of the first region of the surface of the porous surface structure 1311-1 without forming convex edges. Subsequently, the second region of the porous surface structure 1311-2 is connected with the substrate 1313 by resistance welding: the bottom surface of the second positive electrode 1314-2 covers the corresponding region of the porous surface structure 1311-2, and the part of the second positive electrode 1314-2 beyond the welding area does not exceed the edge of the side of the first positive electrode 1314-1 proximal to the second positive electrode 1314-2, and the resistance heating between the second positive electrode 1314-2 and the second region of the porous surface structure 1311-2 results in the sinkage of the second region of the surface of the porous surface structure 1311-2 without forming a convex edge. When the first positive electrode 1314-1 and the second positive electrode 1314-2 are sequentially used for welding, the first positive electrode 1314-1 and the second positive electrode 1314-2 may be the same electrode.

Alternatively, the first positive electrode 1314-1 and the second positive electrode 1314-2 are pressed simultaneously onto the porous surface structure of the corresponding regions, then the first region of the porous surface structure 1311-1 and the second region of the porous surface structure 1311-2 complete simultaneously resistance welding with the substrate, and the part of the first positive electrode 1314-1 beyond the welding area does not exceed the edge of the second side of the recess 13a; the bottom surface of the second positive electrode 1314-2 covers the corresponding region of the porous surface structure 1311-2, and the part of the second positive electrode 1314-2 beyond the welding area does not exceed the edge of the first side of the recess 13a. In this way, the issue of convex edges from partitioned welding is resolved. The process requires that the sinkage of the porous surface structure to be above the top surface of the recess 13a.

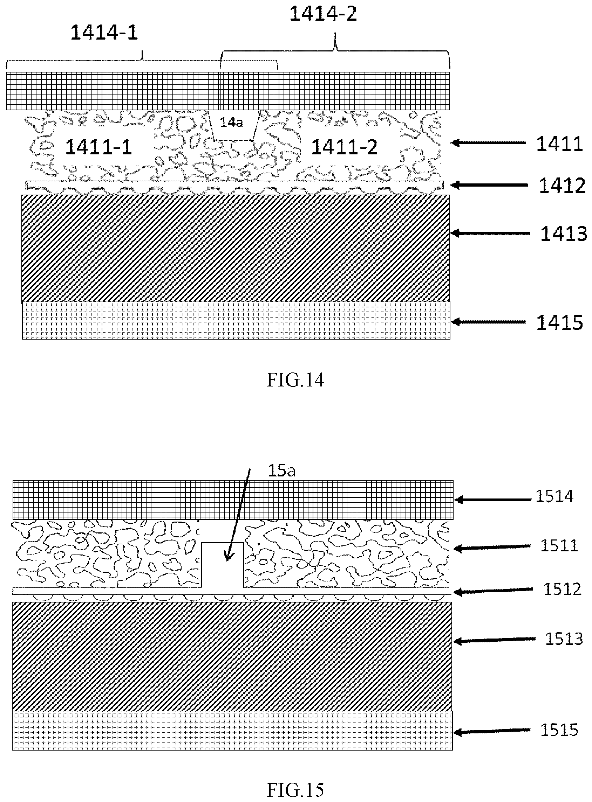

A variant of this Embodiment XVI is as follows:
As shown in FIG. 14, a first positive electrode 1414-1 is in contact with the first region of the porous surface structure 1411-1, and similarly a second positive electrode 1414-2 with the second region of the porous surface structure 1411-2. The top of the negative electrode 1415 is in close proximity to the bottom of the substrate 1413, and between the porous surface structure 1411 and the substrate 1413 is provided with a non- porous base plate 1412 (or a low porosity porous structure), and a plurality of raised structures are prefabricated on the bottom surface of the base plate 1412, and the raised structures are in contact with the top of the substrate 1413.

The porous surface structure 1411 is provided with recesses 14a, which divide the top of the porous surface structure 1411 into a plurality of regions, such as the first region of the porous surface structure 1411-1 and the second region of the porous surface structure 1411-2 in the figure. Each of the recesses 14a is in the form of an elongated strip, with the first region of porous surface structure 1411-1 and the second region of the porous surface structure 1411-2 located on each side of the recess 14a. The top of the recess 14a is lower than the top of the porous surface structure 1411. The height of the recess 14a is less than the height of the porous surface structure 1411. This Embodiment contains the recess 14a, with one side of the recess 14a proximal to the first positive electrode 1414-1 denoted as the first side, and the other side of the recess 14a proximal to the second positive electrode 1414-2 denoted as the second side.

As shown in Figure 14, there is an overlap portion between the first positive electrode 1414-1 and the second positive electrode 1414-2 (the first positive electrode 1414-1 and the second positive electrode 1414-2 are used sequentially, and Figure 14 only represents the positions schematically).

The first positive electrode 1414-1 is in contact with the first region of the porous surface structure 1411-1 and extends beyond a part of the recess 14a (i.e., the first positive electrode 1414-1 spans the first side of the recess 14a but does not extend beyond the second side of the groove 14a) and further contacts a part of the second region of the porous surface structure 1411-2. After the welding process is completed, the second region of the surface of the porous surface structure 1411-2 has a small amount of sinking and the has an indented raised edge. Then the second positive electrode 1414-2 contacts the second region of the porous surface structure 1411-2 and spans the remaining portion of the recess 14a or spans the entire recess 14a, and covers the above indentation raised edge to ensure that the second positive electrode 1414-2 presses and flatten out the indentation raised edge on the the above porous surface structure 1411-2.

Alternatively, the first positive electrode 1414-1 contacts the first region of the porous surface structure 1411-1 and does not extend beyond the first side of the recess 14a. After the welding process is completed, the first region of the surface of the porous surface structure 1411-1 has a small amount of sinking and the surface will have an indented raised edge. And then the second positive electrode 1414-2 contacts the second region of the porous surface structure 1411-2, and spans across both the entire recess 14a and the indented raised edge produced earlier on the surface of the first region of the porous surface structure. The second positive electrode 1414-2 is ensured to press on and flatten out the indentation raised edge on the surface of the first region of the porous surface structure 1411-1. The process requires the controlling of resistance heating sinkage of the porous surface structure to be above or substantially flush with the top of the recess 14a.

### Embodiment XVII

This Embodiment XVII is similar to Embodiment XVI above in that both use partitioned resistive welding. However, in order to solve the problem of indentation raised edges caused by partitioned welding, the porous surface structure of this Embodiment XVII (no schematics shown) does not contain recesses. In this Embodiment , when the porous surface structure of two adjacent partitioned regions are resistance welded sequentially, the bottom surface of the first positive electrode is smaller in size than the corresponding first region of the porous surface structure. Hence, an indentation raised edge is formed on the first region of the porous surface structure after the welding (where the part of the edge that is not indented is higher relative to the indented part). It is necessary to ensure that the second positive electrode for resistance welding can cover and flatten out the raised edge of the first region of the porous surface structure, thus avoiding the problem of raised edges of edges caused by partitioned welding.

### Embodiment XVIII

As shown in FIG. 15, the top surface of the non-porous base plate 1512 of this Embodiment XVIII is provided with a border structure 15a in the form of an elongated strip that can be used as a reference for the regional division, and the said border structure 15a is provided at the border of any two adjacent areas. The top of the border structure 15a is lower than the top of the porous surface structure 1511, and the height of the border structure 15a is less than the height of the porous surface structure 1511. Exemplarily, the body of the non-porous base plate 1512, the border structure 15a, and the porous surface structure 1511 are integrally formed with processes such as 3D printing additive manufacturing, or vapor phase deposition, etc.

The limiting structure 15a in this Embodiment is a solid structure or a porous structure with lower porosity than the porous surface structure 1311-1 and porous surface structure 1311-2.

The positive electrodes of this Embodiment XVIII can be a large planar electrode 1514, covering multiple regions of the porous surface structure shown in FIG. 15, or the first positive electrode 1314-1 and the second positive electrode 1314-2 in FIG. 13 where gaps exist in between, or the first positive electrode 1414-1 and the second positive electrode 1414-2 in FIG. 14 that at least partially overlap. With above processes, the composite structure of the porous surface structure 1511 and the non-porous base plate 1512 is resistance welded with the substrate 1513, without forming raised edges in any region on any surface of the porous surface structure 1511 with a small amount of sinking. The limit structures 15a achieve the purpose of position limit to restrict the amount of sinking. This method not only solves the problem of raised edges from partitioned welding, but also limits the final position of the porous surface structure after resistive welding heat sinkage.

### Embodiment XIX

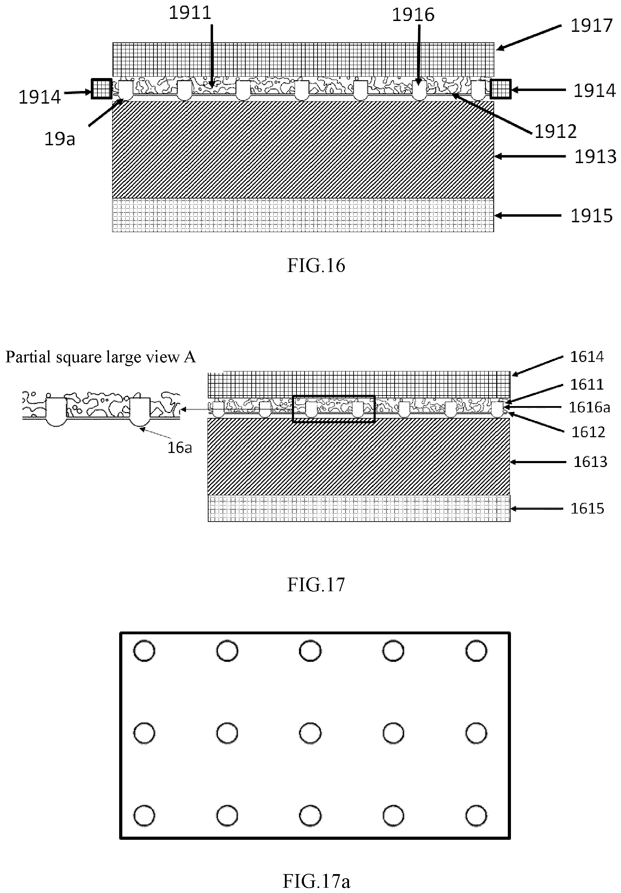

Based on Embodiment V, some variations of this Embodiment XIX are made, as described below: As shown in FIG. 16, the non-porous base plate 1912 is set between the porous surface structure 1911 and the substrate 1913, and the bottom surface of the non-porous base plate 1912 is prefabricated with a plurality of raised structures 19a, which are in contact with the top of the substrate 1913. The support post 1916 is set anywhere within the porous surface structure 1911, the top of the support post 1916 is lower than the top surface of the corresponding portion of the porous surface structure, and the height of the support post 1916 is lower than the height of the porous surface structure, i.e., the support post 1916 is hidden inside the porous surface structure 1911. The top of the negative electrode 1915 is also in close proximity to the bottom of the substrate 1913. In particular, the positive electrode 1914 in this Embodiment XIX is not in contact with the upper surface of the porous surface structure 1911, and the upper surface of the porous surface structure 1911 is in contact with a load applicator 1917, which provides pressure to press the porous surface structure 1911 and the substrate 1913 towards each other, facilitating the welding between the raised structure of the non-porous base plate 1912 and the substrate 1913.

In one Embodiment , the positive electrode 1914 is in contact with the side of the porous surface structure 1911, and when an electric current is applied, the current flowing from the positive electrode 1914 first flows through the said porous surface structure 1911, and since the support post 1916 is a good conductive solid structure, most of the current then passes preferentially through the support post 1916 and then flows to the contact surface and adjacent area between the raised structure 19a of the non-porous base plate 1912 and the substrate 1913, to form the welding connection between the raised structure 19a and the top of the substrate 1913.

In another Embodiment , the positive electrode 1914 is in contact with the side of the support post 1916, and when an electric current is passed, the current flowing from the positive electrode 1914 flows first through the support post 1916 and then to the contact surface and adjacent area between the raised structure 19a of the non-porous base plate 1912 and the top of the substrate 1913, causing the raised structure 19a to be welded to the top of the substrate 1913.

In another Embodiment , the positive electrode 1914 is in contact with the side of the non-porous base plate 1912, and when current is passed, the current flowing from the positive electrode 1914 flows through the non-porous base plate 1912 and reaches the contact surface and adjacent area between the raised structures 19a of the non-porous base plate 1912 and the top of the substrate 1913, causing the raised structures 19a to be welded to the top of the substrate 1913.

In another Embodiment , the positive electrode 1914 is in contact with two or three of the side of the porous surface structure 1911, the side of the support post 1916, and the side of the non-porous base plate 1912, and the current flowing from the positive electrode 1914 flows to the contact surface and adjacent area between the raised structure 19a of the non-porous base plate 1912 and the top of the substrate 1913, causing the raised structure 19a to be welded to the the top of the substrate 1913.

Based on the above Embodiment , the positive electrode 1914 is in contact with the side of the composite and is not in direct contact with the upper surface of the porous surface structure 1911. Both avoid damage to the surface of the porous surface structure 1911 due to resistance heating.

The method of energizing the positive electrode from the lateral direction into the composite body in this Embodiment XIX (the polarity of the positive electrode and the negative electrode in this Embodiment is interchangeable) is not only applicable to the embodiments with support posts in the porous surface structure, such as EmbodimentsIII to VII and IX to XII, but also applicable to other embodiments without support posts in the porous surface structure, such as EmbodimentsI, II, VIII and XIII, which will not be described in detail in the present invention; In addition, when the positive electrode is connected to the composite from the lateral direction for energization in this Embodiment XIX, the following situations exist: (a) the upper surface of the porous surface structure 1911 is not connected to any positive electrode, the details of which can refer to the corresponding Embodiment above; (b) part or all of the upper surface of the porous surface structure 1911 is in contact with a part or all of the upper surface of the porous surface structure 1911 is in contact with one positive electrode, the details of which can refer to the corresponding Embodiment above, and each positive electrode is connected in parallel to the positive end of the power supply; all of the above cases are covered by the scope of protection of the present invention.

### Embodiment XX.

It is worth stating that the present invention is not limited to the projection resistance welding method alone in any of the above embodiments, but can also employ the spot resistance welding method alone or use in combination the projection resistance welding method and the spot resistance welding method to connect the intermediate structure with the substrate. Specifically, the spot resistance welding method differs from the projection resistance welding method with raised structures. Spot resistance welding does not create raised structures such as bumps and uses a single electrode while moving the workpiece (e.g., the composite and the substrate) or the electrode to achieve welding at one spot at a time until a set number of welded joints are completed and adequate welding strength is ensured between the intermediate structure and the substrate. In addition, the invention can also use projection resistance welding and spot resistance welding in combination. For example, after the completion of the projection resistance welding as described in any of the above embodiments, spot resistance welding can be subsequently used to strengthen the weld strength between the intermediate structure and the substrate.

The method of projection resistance welding of the present invention can weld multiple joints simultaneously within one welding cycle, with high productivity and without shunt effect; at the same time, because the current solid volume fraction is concentrated in the bumps, the current solid volume fraction is high, so a smaller current can be used for welding and a smaller welding nugget can be reliably formed, overcoming the nugget offset phenomenon of spot resistance welding; the bump position and size of the projection resistance welding are accurately controlled with relatively uniform welding strength. Therefore, for a given bonding strength, the size of a projection welding nugget can be smaller than the spot welding; in addition, because of the use of large planar electrodes, and the bumps are set on the intermediate structure, so the indentation on the exposed surface of the substrate can be minimized, while the current solid volume fraction of large planar electrodes is small with good heat dissipation, the electrode wear is much less than the spot welding electrodes, thus greatly reducing the electrode maintenance and repair costs.

For any of the above embodiments of projection resistance welding processes, mainly the central portion of the raised structure are pressed against the substrate to generate resistance heating for forming welding connection, and the side surfaces of the raised structures are not in sufficient contacts with the substrate to achieve effective connection. It can therefore be beneficial to perform resistance welding to the intermediate structure in multiple times and from multiple directions by rotating one or more of the electrodes, or the substrate, or the intermediate structure, or combinations of thereof, to ensure that the raised structures are welded to the substrate from all directions. This can further improve the bonding strength between the raised structures of the intermediate structure and the substrate,

In addition, an extension of the above-mentioned Embodiment is that: in some of the above-mentioned embodiments, the porous surface structure 21 is in contact with a large planar positive electrode 24, and the surface of the porous surface structure may be resistance heat damaged (dented, darkened), which can be overcome by covering the surface of the porous surface structure with an insulating member with multiple openings through which positive electrodes or good conductive support posts can be inserted, etc., thereby preventing the porous surface structure covered by the insulating member from thermal damage. Wherein the thickness of the said insulating member needs to be optimal, to ensure a complete conduction of the current circuit and smooth welding process.

### Embodiment XXI

In the above Embodiment V, when the stiffness of the non-porous base plate 812 in the composite body is too high, it may lead to difficulties in fitting the raised structure and the substrate for intimate contacts, which in turn affects the welding effect between the non-porous base plate and the substrate. Therefore, the present invention needs to ensure that the entire connected structure has a suitable range of stiffness, so as to ensure the basic strength of the connected structure, but also enable the intermediate and the substrate to be in close contact and enhance the welding effect between the said non-porous base plate and the substrate.

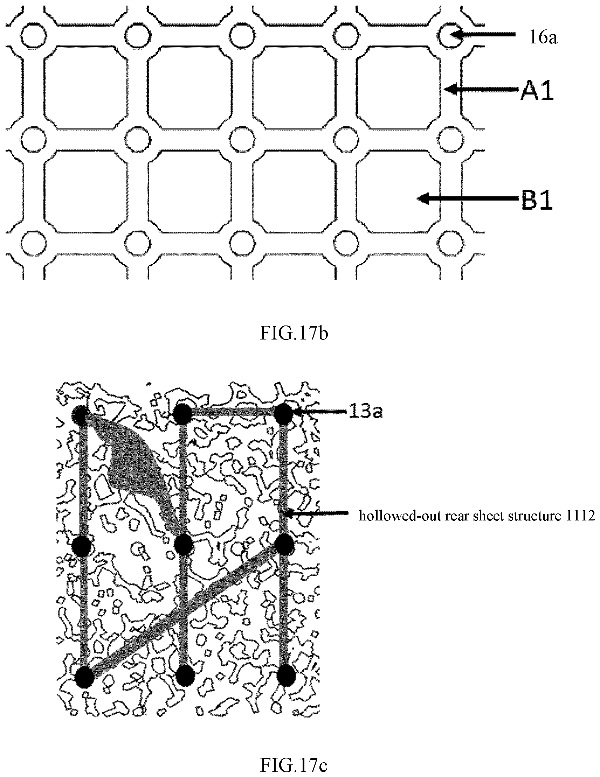

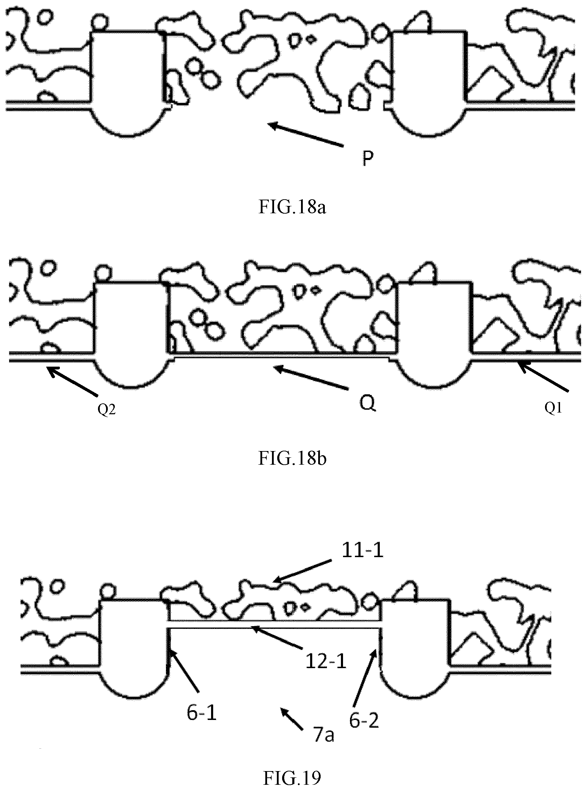

As shown in combination of FIG. 17, FIG. 17a-c, and FIG. 18a-b, this Embodiment XXI builds on Embodiment V by making the stiffness value of a certain portion of the composite region in the composite containing the porous surface structure 1611 and the intermediate 1612 smaller, For example, by making the thickness of the composite in that composite portion of the region smaller. The composite region referred herein is the area or at least part of the area occupied by the composite containing the non-porous base plate and porous surface structure. The thickness of the composite body can be reduced by: (a) reducing the thickness of the non-porous base plate 1612 in the intermediate at the corresponding location; and (b) reducing the thickness of the porous surface structure at the corresponding location. In addition, this Embodiment XXI can also design the non-porous base plate in Embodiment V as a hollowed-out non-porous base plate 1612 to achieve a smaller stiffness value in the corresponding composite region.

As shown in FIG. 17, the non-porous base plate 1612 in this Embodiment is set between the porous surface structure 1611 and the substrate 1613, a plurality of raised structures 16a are prefabricated on the bottom of the non-porous base plate 1612, and the raised structures 16a are in contact with the top of the substrate 1613, and a number of support posts 1616a, the said support posts 1616a being between the non-porous base plate 1612 and the positive electrode 1614. Exemplarily, the said support posts 1616a are located inside the porous surface structure 1611 and the top of the negative electrode 1615 is in direct contact with the bottom of the substrate 1613. This Embodiment is not only applicable to a non-porous base plate of the intermediate but also to a porous structure of the intermediate with a high solid volume fraction. For the sake of convenience of presentation, this Embodiment is mainly illustrated for a non-porous substrate 1612 with a solid structure.

### (i) Reducing the thickness of the non-porous base plate 1612:.

In one Embodiment , the thickness of the non-porous base plate 1612 in Region P is reduced to zero, i.e., there is no intermediate at Region P, forming a hollowed-out structure, as shown in Figure 18a.

In another Embodiment, the thickness of the non-porous base plate 1612 of region Q is reduced to below those of other regions (e.g., Regions Q1 and Q2). For example, the upper or lower part of the said non-porous base plate 1612 of region Q is made thinner relative to the non-porous base plate at regions Q1 and Q2, even though the reduced thickness value is still greater than zero, i.e., there is still the intermediate in Region Q, as shown in Figure 18b. (ii) Reducing the thickness of the porous surface structure 1611.

As shown in Figure 19, while the thickness of the non-porous base plate 1612 in region 7a is not reduced, the position of the non-porous base plate 12-1 in region 7a is raised upward, i.e., the bottom end of the nonporous base plate 12-1 is moved further away from the substrate, and the position of the bottom end of the porous surface structure 11-1 corresponding to region 7a is also raised upward, i.e., the thickness of the porous surface structure 11-1 becomes smaller, then The stiffness of the composite formed by the non-porous base plate 12-1 and the porous surface structure 11-1 at area 7a becomes smaller, which improves the welding efficiency between the non-porous base plate and the substrate, and at the same time, the gap distance between the bottom end of the non-porous base plate 12-1 in area 7a and the substrate increases, then eventually the non-porous base plate 12-1 in this area, the support posts 6-1 and 6-2 on both sides adjacent to the non-porous base plate 12-1 and the substrate together form a pocket structure, which facilitates the expansion of the use of this connected structure in future.

The above Embodiment describes the case where the thickness of the non-porous base plate 1612 of the region 7a is kept constant, i.e., only the thickness size of the porous surface structure 11-1 corresponding to the region 7a is reduced, but the present invention can also reduce the thickness of the non-porous base plate 1612 while simultaneously reducing the thickness of the porous surface structure 11-1 of the region, so that the reduction of the stiffness of the composite in the region is even greater.

The thickness reduction of the composite body in this Embodiment can be achieved in any one or a combination of the following ways: (1) by machining operations: e.g., by hollowing out a non-porous base plate with reduced thickness or hollowed out plate body on an unhollowed out prefabricated uniform thin plate solid with a raised structure; (2) by integral forming of a non-porous base plate with a porous surface structure, etc.: e.g., by 3D printing additive manufacturing process, or vapor phase deposition process, etc. to obtain a composite body with reduced thickness.

As shown in FIG. 17b, the non-porous base plate 1612 of this Embodiment is divided into raised structures 16a, region B1 with reduced stiffness, and region A1 without stiffness reduction. The present invention does not limit the shape of the stiffness reduction region B1 of the non-porous base plate, as long as it contains a portion of the stiffness reduction region, wherein the stiffness reduction region B1 may comprise plurality of localized regions.

When the stiffness value of all areas of the composite body is reduced in this Embodiment XXI compared to Embodiment V, and the thickness of the intermediate in all areas of the composite body is reduced to zero, only the raised structure 16a remains in the main body of the intermediate, i.e., this Embodiment XXI becomes the case in Embodiment XII above. The non-porous base plate in this Embodiment is not only applicable to Embodiment V, but also applicable to EmbodimentsIII, IV, VI, XII, and also applicable to other variations of EmbodimentsIII~XII and any other embodiment. The present invention will not descrived these in detail. In addition, the present embodiments are not only applicable to each of the above embodiments in which the composite body contains a support post or a raised structure, but the design method of this Embodiment XXI is also applicable to other embodiments of the present invention in which the composite body is not provided with support posts and/or no raised structure is provided, as well as their variations and any other embodiments, as long as the thickness of a corresponding composite area of the composite body can be made smaller so that its stiffness is reduced. The invention is not limited by the fact that the basic strength of the joint structure can be guaranteed and the intermediate and the substrate can be tightly welded. Again, no detailed description will be provided in the present invention.

The intermediate in this Embodiment is not limited to solid structure, and can be a porous structure. When the intermediate is a high-solid volume fraction porous structure and the thickness of the intermediate is reduced to zero in any of the above-mentioned local areas of the stiffness reduction area B1, the pores of the porous structure as an intermediate should be smaller than the size of the hollowed-out area.

In summary, the present invention can not only ensure the strength of the entire connected structure, but also avoid to some extent the problem of poor contact between the raised structure and the substrate due to too much stiffness, thus enhancing the welding efficiency between the said non-porous base plate and the said substrate.

It is worth stating that the size and shape of the gap left between the non-porous base plate and the substrate with a corresponding pocket structure in this Embodiment can be designed according to the actual application. For example, they can be adjusted according to the shape of the substrate or specific requirements for the whole connected structure. The present invention does not set limitations in this respect. It should be noted that the design of the pocket structure in the present Embodiment is not only applicable to embodiments III, IV, V and X with supporting posts and raised structures, but also to other embodiments with non-porous base plates (or porous intermediates) without supporting posts or raised structures, as long as a certain gap is retained to form a pocket structure between the non-porous base plates (or porous intermediates) and the substrate. The present invention will not describe these situations in detail. Of course, the pocket structure design in this Embodiment is also applicable to each Embodiment with a non-porous base plate (or porous intermediate) and any other embodiments, any variations of the Embodiment XXI. The present invention will not describe these situations in detail herein.

### Embodiment XXII

Referring to EmbodimentsIII, IV, V and X above, the present invention not only provides a non-porous base plate between the porous surface structure and the substrate, and a plurality of raised structures prefabricated on the bottom surface of the non-porous base plate, with the raised structures in contact with the top of the substrate, but also provides a number of support posts on the surface of the non-porous base plate near the side of the porous surface structure (e.g., support posts 816a of FIG. 5a, 816b of FIG. 5b, 816c of FIG. 5c, 1216 of FIG. 8, etc.), as the support post is a solid structure with good electrical conductivity, and the porous surface structure has pores, most of the current flowing from the electrode passes through the solid structure of the support post with good electrical conductivity, which can greatly reduce the surface damage of the porous surface structure due to resistance heating, and can also enhance the current conduction effect, enhancing the welding efficiency between the non-porous base plate and the substrate with increased welding strength. It can be seen that the welding strength can be directly enhanced by increasing the amount of current conducted, For example, by increasing the volume or diameter of the support post or by increasing the number of support posts.

If the number of support posts is too many, there will be some problems despite of the increase in current conduction: (1) the support posts generally correspond to the raised structures, and when the number of support posts increases, the number of raised structures will also increase,. But if the number of raised structures is too many, it will affect the welding uniformity and welding strength between the non-porous substrate and the substrate; (2) if the number of support posts is too many, it will also affect the welded (2) If the number of support posts is too many, it will also affect the visual appearance after welding, because even if there is a porous surface structure above the support posts, but the porous surface structure has a certain thickness and there are pores, and the traces of multiple support posts being pressed can still be visible; therefore, it is not appropriate to design too many support posts in this invention. In addition, increasing the diameter of the support post at a certain location can increase the electric current passing through, thus enhancing the welding strength; but the large the diameter of the support post becomes, the more obvious the visible indentation of the support post after welding becomes; in addition, the stiffness of the whole structure increases with larger diameters of the support posts.

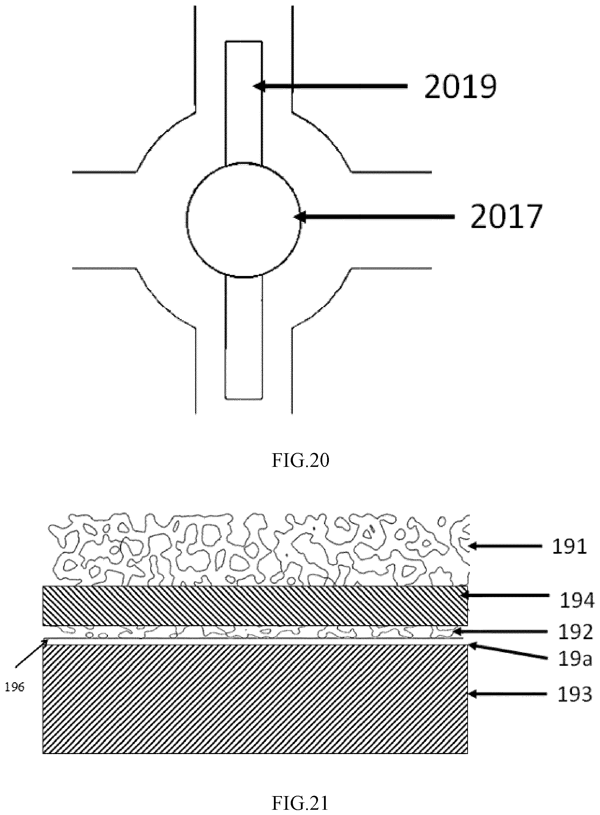

For the above reasons, this Embodiment XXII provides accessory structures 2019 to the top surfaces of the support posts 2017, which are placed on the top surface of the non-porous base plate, as shown in FIG. 20. The said accessory structures 2019 are conductively connected to the said support posts 2017, so that most of the current flowing from the electrode is preferentially passed through the good conductive support post 2017 connected with the accessory structure 2019, which greatly reduces the surface damage to the porous surface structure from resistance heating, and enhances current conduction to ensure sufficient welding strength without creating the problem of unnecessary indentation caused by increasing the number of support posts.

Exemplarily, the said support post 2017 is a solid structure. Preferably, the said accessory structure 2019 is a solid structure of a good conductor or a porous structure with a high solid volume fraction; wherein, when the said accessory structure 2019 is porous, its solid volume fraction is higher than that of the porous surface structure. In this embodiment, one or more accessory structures 2019 are provided on any one of the support posts 2017.

As shown in FIG. 20, the accessory structure 2019 is in the shape of "I" (e.g. a long bar or a round rod), or "+"; the said accessory structure 2019 may also be a structure of any shape such as a bent shape or a curved shape, and the present invention does not limit this. The accessory structure 2019 may be provided at any height position on the support post 2017, and the accessory structure 2019 may be provided at any position on the lateral surface on the support post 2017, and the accessory structure of the present invention is applicable to any of the above embodiments provided with a support post, and the said accessory structure may be located within the porous surface structure, or may be located between the porous surface structure and the non-porous base plate. The structure can be located between the porous surface structure and the substrate. Preferably, based on Embodiment XXI, the accessory structure 2019 is staggered from the stiffness reduction region B1 of the composite body, i.e. the said accessory structure 2019 corresponds to the stiffness unchanged region A1 of the composite body, as shown in Figure 20. As can be seen from the above, the present invention is effective as long as the accessory structure made of conductive material is conductively connected to the support post to achieve enhanced current conduction. Wherein, the said accessory structure 2019 may or may not be connected to the said non-porous base plate, and the said accessory structure 2019 is connected to a porous surface structure. The non-porous base plate in this Embodiment can be the non-hollowed structure (the thickness of the composite body is not reduced) in Embodiment III, IV, V and X, etc., or is a hollowed-out structure (the thickness of the composite body is reduced) or a pocket structure in Embodiment XXI. These can be designed according to the actual application, and the present invention does not limit this.

Preferably, the non-porous base plate, the porous surface structure, the support post, and the accessory structure are integrally formed as one structure, such as achieved by a 3D printing additive manufacturing process, or a vapor phase deposition process, etc.

In one Embodiment , in order to ensure uniformity of welding between each support post on the non-porous base plate and the substrate, all support posts on the non-porous base plate are uniformly provided with the same structural shape and the same number of accessory structures.

In another Embodiment , in order to ensure the uniformity of welding between each support post on the non-porous base plate and the substrate, but the structure type, shape and size of each support post of the non-porous base plate are different, it is necessary to design the structure, number and size of the corresponding accessory structure of each support post according to the specific situation of each support post, and finally ensure the welding strength between each support post and the substrate is uniform and optimal.

In summary, this Embodiment XXII utilizes conductive accessory structures as additional design to the support posts so that most of the current flowing out of the electrode preferentially passes through the good conductive support post with accessory structures, greatly reducing surface damage to the porous surface structure due to resistance heating, and can enhance the current conduction effect, to ensure adequate welding strength, and will not lead to unnecessary surface indentation or excessive structure stiffness from using large number or large diameter of support posts. The present invention is not only applicable to the above-mentioned Embodiment III, IV, V and X, but also applicable to other embodiments with support posts, as well as their variations or any other embodiments, which will not be detailed in the present invention.

### Embodiment XXIII

The welding method between the non-porous base plate (or porous structure) and the substrate in EmbodimentsIII ~ XXII can utilize the design of the hollowed-out structure in Embodiment XX above, and is mainly the resistance welding method. However, the hollowed-out design of the non-porous base plate of the present invention is not limited to the resistance welding method, but can also be applied to the laser welding method pointed out in this Embodiment XXIII to achieve connection of a porous surface structure, intermediate, and the substrate. Specifically, the porous surface structure and the non-porous base plate are formed as a composite, and the composite is connected to the substrate by laser welding between the non-porous substrate and the substrate. In this case, there are multiple welding points between the non-porous base plate and the substrate, and the location of the welding points is freely chosen according to the application needs.

When the non-porous base plate in this Embodiment uses the hollowed-out structure design in Embodiment XXI, the non-porous base plate is divided into a welding portion, a solid area and a hollowed-out area, and the said solid area serves as a linkage structure for each laser welding area. This Embodiment of the solid area and hollowed-out area of the specific content can be referred to Embodiment XXI, which will not be repeated herein. The present invention does not specifically limit the location of the contact surface between the non-porous base plate and the substrate, welding position, shape (such as curved surface), size, etc., and can be designed according to the actual application.

### Embodiment XXIV

This Embodiment XXIV may be used as follows: a pre-connected porous structure 192 is provided at the top of the substrate 193, and the said pre-connected porous structure 192 is first effectively bonded to the substrate 193 in advance by various methods, including laser welding, resistance welding, etc., as shown in FIG. 21. In the case of resistance welding, the pre-connected porous structure 192 can be resistance welded in direct contact with the substrate 203, or the pre-connected porous structure 192 can be bonded to the substrate 193 through the intermediate 196 using the resistance welding method.

The pre-connected porous structure 192 is pre-connected with the said intermediate 196 to form a composite body, and the intermediate 196 is effectively bonded with the substrate 193 by resistance welding method so that the composite body is connected with the substrate 193, which can be referred to the above-mentioned EmbodimentsI to XXII, and the present invention will not describe those details herein. In addition, when the laser welding method is used to connect the pre-connected porous structure 192 and the substrate 193 in advance, it can be referred to the above-mentioned Embodiment XXIII, and the present invention will not repeat the details herein.

As shown in FIG. 21, through an intermediate 196 (such as the non-porous base plate 196 of FIG. 21) located between the pre-connected porous structure 192 and the substrate 193, the pre-connected porous structure 192 is connected to the substrate 193 with a projection resistance welding method, and a connection interface 19a is created between the two; then a gap is left between the pre-connected porous structure 192 and the porous surface structure 191 (for bone ingrowth) above 192, and a molten polymer is injected into the gap and penetrates into both the porous surface structure 191 and the pre-connected porous structure 192.. Alternatively, the polymer material is placed in the gap and melted at when heated to a high temperature so that the molten polymer penetrates both the porous surface structure 191 and the porous structure of the pre-connected porous structure 192, and finally a polymer material intermediate layer 194 is formed. The polymer intermediate layer 194 is tightly bonded to both the porous surface structure 191 and the pre-connected porous structure 192, thus realizing a tight connection between the porous surface structure 191 and the substrate 193, as shown in Figure 15.

This Embodiment can avoid debonding between the substrate and the combined structure of the polymer material layer and the porous structure above it. In addition, this Embodiment can appropriately reduce the thickness of the substrate layer by adding an intermediate polymer material to the connected structure between the porous surface structure and the substrate, which can both ensure the basic mechanical strength of the said connected structure and improve the stress shielding phenomenon. Stress shielding effect means that: when two materials with different elastic moduli are stressed together, the one with the larger elastic modulus will bear more stress; when the modulus of elasticity of the substrate is much larger than that of the bone, the bone will bear less stress, which will lead to postoperative osteolysis in severe cases. Since the present invention incorporates polymeric poly material with elastic modulus smaller than that of the substrate, the phenomenon of stress shielding effect will be improved accordingly.

The pre-connected porous structure 192 and the porous surface structure 191 of the present invention are independent structures. The polymeric material intermediate layer 194 is filled within at least a portion of the pores in the porous surface structure 191 and within at least a portion of the pores in the pre-connected porous structure 192, respectively. In addition, the present invention does not limit the method of using the pre-connected porous structure 192 in effective combination with the substrate 193, as long as the two structures can be connected effectively.

### Embodiment XXV

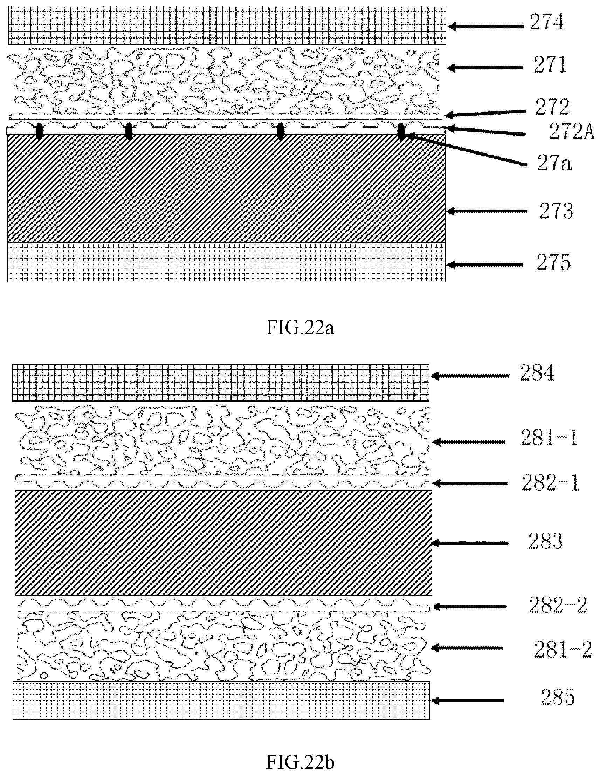

As shown in Figure 22a, the following improvements can also be made based on Embodiment I & II: transforming the non-porous base plate or low porosity area in EmbodimentsI and II by removing the raised structures into a (non-porous or low porosity) intermediate plate structure 272 without a raised structure, and changing the substrate 273 into a substrate composite comprising substrate body 273 and a separate substrate structure 272A with raised structures on the top surface of the substrate composite, the said substrate with raised structure 272A being pre-connected (e.g., resistance welding/laser welding) to the said substrate body 273, the raised structure of substrate structure 272A being in contac with the side of the intermediate plate structure 272 without bumps. The surface composite formed by the porous surface structure 271 and the intermediate plate structure 272, and the substrate composite formed by the substrate raised structure 272A and the substrate body 273, are pressed between the positive electrode 274 and the negative electrode 275. When an electric current is passed, the current flows through the porous surface structure 271, the intermediate plate structure 272 up to the bumps of the substrate raised structure 272A, generating resistance heating due to contact resistance thereby heating the bumps of the substrate raised structure 272A and the bottom of the intermediate plate structure 272 to a molten or plastic state, and finally achieving a welding connection between the intermediate plate structure 272 and the substrate raised structure 272A, thereby causing the said surface composite body is tightly bonded to the said substrate composite body. The numerical mark 27a in the figure indicates the welding point. The substrate body 273 and the substrate raised structure 272A can be provided separately or simultaneously; when the substrate body 273 and the substrate raised structure 272A are provided separately, they are pre-bonded together; when the substrate body 273 and the substrate raised structure 272A are provided simultaneously, the substrate raised structure 272A can be formed on the top surface of the substrate body 273 toward the side of the intermediate plate structure 272. In another Embodiment , the surface composite body still adopts the structure 272 with bumps in Embodiments1 and 2, with the raised structure facing the side of the substrate body 273, and has the raised structure of the intermediate plate structure 272 (not shown, referring to the above-mentioned Embodiment s) staggeredly arranged with the bumps of the substrate raised structure 272A above the substrate, so that the said surface composite body and the said substrate composite body can also be tightly combined in the end. The above improvements of this Embodiment are not limited to the basis of Embodiment I, but can also be applied to any of the above embodiments, which will not be repeatedly described in this invention. The substrate raised structure of this Embodiment XXV is not limited to contact with the intermediate of the composite (e.g., the intermediate plate of Embodiment I), but it may also be in contact with the porous surface structure of the composite, e.g., when applicable to Embodiment XI, the bumps of the substrate raised structure 272A are staggeredly arranged with the raised structure 1112a of the composite, and the bumps of the substrate raised structure 272A may be in contact with the porous surface structure 1111 of the composite. Porous surface structure 1111 of the composite body or the bumps of the substrate raised structure 272A are in contact with the support post 1116a of the composite body, and the other contents are referred to this Embodiment and will not be described herein. The substrate raised structure of the present invention is provided on a substrate surface attachment layer that is additionally bonded to the substrate body, or the substrate raised structure is provided directly to the surface structure of the substrate body (e.g., by conventional machining or precision casting/precision forging), and the above-mentioned base surface attachment layer can be dispensed with.

As shown in FIG. 22b, based on Embodiment I, this Embodiment can also be done using the following scheme: first, as in Embodiment I, a first composite body formed by a first porous surface structure 281-1 pre-connected to a first non-porous base plate 282-1 is provided on the top side of the substrate 283, the first composite body is placed between the positive electrode 24 and the top surface of the substrate 283, and at least a portion of the first porous surface structure 281-1 is in contact with the positive electrode 24; the bottom of the first non-porous base plate 282-1 is prefabricated with a plurality of first raised structures which are in contact with the top of the substrate 283. Further, a second composite body formed by a second porous surface structure 281-2 pre-connected to the second non-porous base plate 282-2 is provided on the bottom side of the substrate 283, the second composite body being placed between the bottom surface of the substrate 283 and the negative electrode 285, and at least a portion of the bottom of the second porous surface structure 281-2 is in contact with the negative electrode 285. Wherein, the second composite body has the same structure as the said first composite body and is axisymmetric about the substrate 283. In accordance with the principle of Embodiment 1, the first composite body and the second composite body are resistance welded to the upper and lower surfaces of the substrate 283 simultaneously to achieve the connection of the first composite body, the second composite body, and the substrate; or, in accordance with the principle of Embodiment 1, the first composite body is resistance welded to the upper surface of the substrate first, and then the second composite body is resistance welded to the lower surface of the substrate that has been welded with the first composite body. Finally realize the first composite body, the second composite body, the complete connection of the substrate; or, in accordance with the principle of Embodiment 1, the second composite body and the lower surface of the substrate first resistance welding, and then the first composite body and has been welded with the second composite body of the upper surface of the substrate resistance welding, and finally realize the first composite body, the second composite body, the complete connection of the substrate. This variation is also applicable to any one of the above embodiments and its variation from Embodiment I to Embodiment twenty-four, and the invention will not be repeated.

### Embodiment XXVI

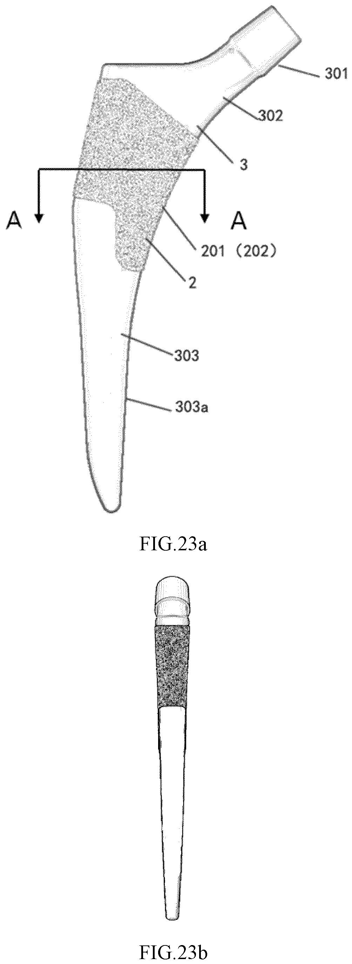

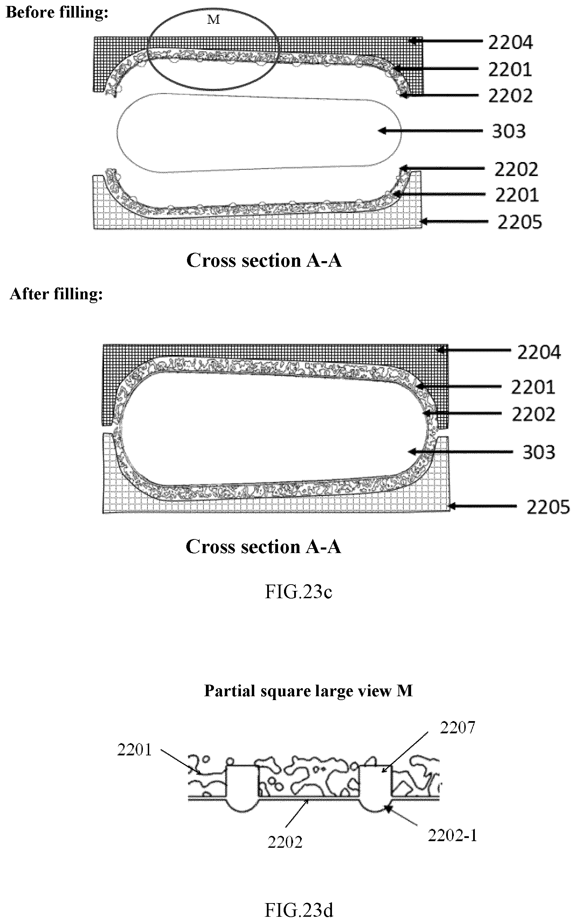

As shown in combination with FIGS. 23a-FIG. 23c, this Embodiment provides a prosthetic implant, preferably an orthopedic prosthesis; made with any one or more of the connected structures and methods described in embodiments I through XXV above, and their respective variants. The prosthetic body 1 corresponds to the substrate in the connected structure, and at least a portion of the surface of the prosthetic body 1 serves as an connection region, which is connected to a composite 2 comprising an intermediate structure and a porous surface structure. The porous surface structure is connected to the substrate through the connection of the intermediate and the substrate (by means of projection resistance welding and/or spot resistance welding) of the intermediate structure to the substrate to form a surface coverage of the connection area on the prosthesis.

In combination with the structures and methods of EmbodimentsI-XXV or variations thereof, the prosthetic implant is provided with a porous surface structure as the outer layer and an intermediate structure as the inner layer in contact with and connected to the connection area of the prosthetic body by resistance welding, achieving the connection of the porous surface structure to the prosthetic body and form a surface coverage of the connection area on the prosthetic body. The structure and method can be used for various implantable prostheses such as joint implants and other types of orthopedic implants such as femoral stems, acetabular cups, femoral condyles, tibial trays, etc., with specific reference to the detailed descriptions in subsequent EmbodimentsXXVI-XXIX.

Use the artificial hip joint for illustration. The artificial hip joint contains a femoral stem, a femoral head (not shown schematically), an acetabular cup, and a cup liner (not shown schematically), all of which are prostheses made of implantable medical materials such as metallic materials including titanium, cobalt-chromium-molybdenum and stainless steel, and polymers such as ultra-high-molecular-weight polyethylene, ceramics, etc.

The said femoral stem 3 (FIGS. 23a-FIG. 23c) comprises a tapered end 301, a neck 302, and a stem body 303, which may be integrally formed or assembled. The tapered end 301 of the femoral stem 3 is a taper structure, and the first end is connected to the stem body 303 through the neck 302, with the tapered end 301 and the neck 302 having a certain angle with respect to the stem body, arranged in an inclined form with respect to the stem side. The lower part of the stem body 303 is inserted into the femoral intramedullary cavity. The lower part of the stem body 303 may be provided with a number of longitudinal grooves. The surface of the stem body 303, preferably the upper surface of the stem body 303, is porous; the lower part of the stem body 303 may have a smooth surface.

The second end 301 of the femoral stem 3 is inserted into the inner taper of the femoral head; the acetabular component fits with the outer surface of the femoral head, and the femoral head is in contact with the inner concave surface of the acetabular liner so that the femoral head can freely rotate against the liner. In some Embodiment s, the acetabular cup is a partially spherical (e.g. hemispherical) dome; the acetabular cup is provided with a mating liner; the femoral head is in contact with the inner concave surface of the liner so that the femoral head can be rotated therein. The acetabular cup may be provided with through-holes for fixation devices (screws, etc.) to fix the acetabular cup to the acetabular bone; the liner may be provided with or without corresponding through-holes. The inner concave surface of the liner is in contact with the femoral head; the liner may be made of metallic or of non-metallic materials (e.g., polyethylene or ceramic, etc.) to reduce wear of the artificial joint. The acetabular shell is usually made of metallic materials. The outer peripheral surface of the acetabular cup, preferably, uses a porous structure.

The use of porous structures on the upper surface of the femoral stem body 303 and on the outer surface of the acetabular cup shell can increase the surface roughness and also encourage the osteoblastic bone ingrowth into the pores, thus effectively connecting the femoral stem to the femur and the acetabular cup to the acetabulum. This forms a good long-term biological fixation and enhances the stability of the interface between the joint implant and the host bone tissue.

The femoral stem 3 can use the structure and method of EmbodimentsI to XXV or variant Embodimentsthereof, as described above, without further descriptions in detail herein. Please refer to the contents of the corresponding embodiments described above. Wherein, the stem body 303 of the said femoral stem 3 is the substrate in the connecting structure; A composite body comprising of an intermediate structure (such as a non-porous base plate, or a low porosity region of the porous structure, depending upon specific embodiments) and a porous surface structure is the stem body shell 2, which covers the connection region of the stem body 303a (upper surface). Through the welding between the intermediate structure and the substrate, the porous surface structure 201 is connected with the substrate to achieve the coverage of the connection region with a porous surface structure on the top of the femoral stem body 303.

In some Embodiment s, the stem body 303a is manufactured by forging, casting, or machining etc, and is preferably a solid structure that is easy to machine and has high strength; or the stem body 303a may be a porous structure with high solid volume fraction; the intermediate structure may be solid or a porous structure with a higher solid volume fraction than the porous surface structure. When the stem body 303a and the intermediate structure 202 both are porous, the solid volume fraction of intermediate structure 202 lies between those of stem body 303a and porous surface structure 201. The intermediate structure 202 and the porous surface structure 201 of the stem shell 2 are preferably manufactured with 3D printing additive manufacturing process, which can readily form specific pores according to the design requirements, etc. The stem body 303a is effectively connected to the intermediate structure 202 of the stem body shell 2 with resistance welding, avoiding the current problem of significant reduction in overall strength when the porous structure is connected onto the surface of the femoral stem 3 with high temperature processes (e.g., diffusion bonding), etc.

In another Embodiment , as shown in FIG. 23c-d in correspondence with FIG. 16 in Embodiment XXI above, the inner structure stem body 303 is the substrate in the connected structure, and the outer porous structure 2201 is the porous surface structure of the connected structure, and an intermediate structure (e.g. non-porous base plate 2202 with the hollowed-out design in Embodiment XXI) is provided between the porous structure 2201 and the stem body 303. The composite comprising the porous structure 2201 and the non-porous base plate 2202, and the stem body 303 are pressed together between the positive electrode 2204 and the negative electrode 2205. When an electric current is provided, the current flows through the porous structure 2201, to the non-porous base plate 2202 and then to the contact surface with the outer surface of the stem body 303 and the adjacent area, generating resistance heat to melt or plasticize the welding areas, causing the non-porous base plate 2202 to form strong welding with the stem body 303, hence rendering the porous structure 2201 firmly bonded with the stem body 303. Wherein, between the stem body 303 and the porous structure 2201 is a hollowed-out non-porous base plate 2202, the bottom of the hollowed-out non-porous base plate 2202 is prefabricated with a plurality of raised structures 2202-1, the raised structures 2202-1 are in contact with the stem body 303, and a plurality of support posts 2207 are provided on the surface of the non-porous base plate 2202 near the side of the porous structure 2201, said support posts 2207 are located between the non-porous base plate 2202 and the positive electrode. Other elements concerning femoral stem applicable to Embodiment XXI are not described in detail herein.

In a specific Embodiment , the upper part of the stem body 303a of the femoral stem 3 is provided with a connection area; for the convenience of description, the side towards which the proximal portion 301 and the neck 302 of the femoral stem 3 incline is denoted as the medial side of the femoral stem 3, and the other sides of the stem body 303a are denoted as the posterior, lateral and the anterior side, respectively, with reference to Figure 23a (anti-clockwise), with the medial and lateral sides facing each other, and the anterior and posterior sides facing each other. Figure 23a shows the anterior view and Fig. 23b shows the lateral view.

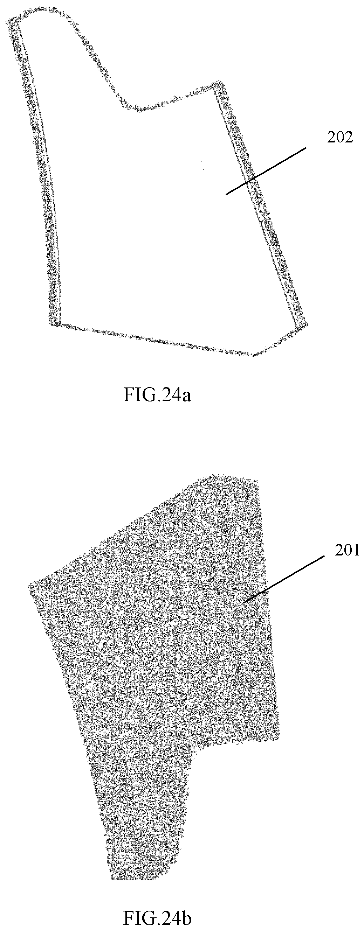

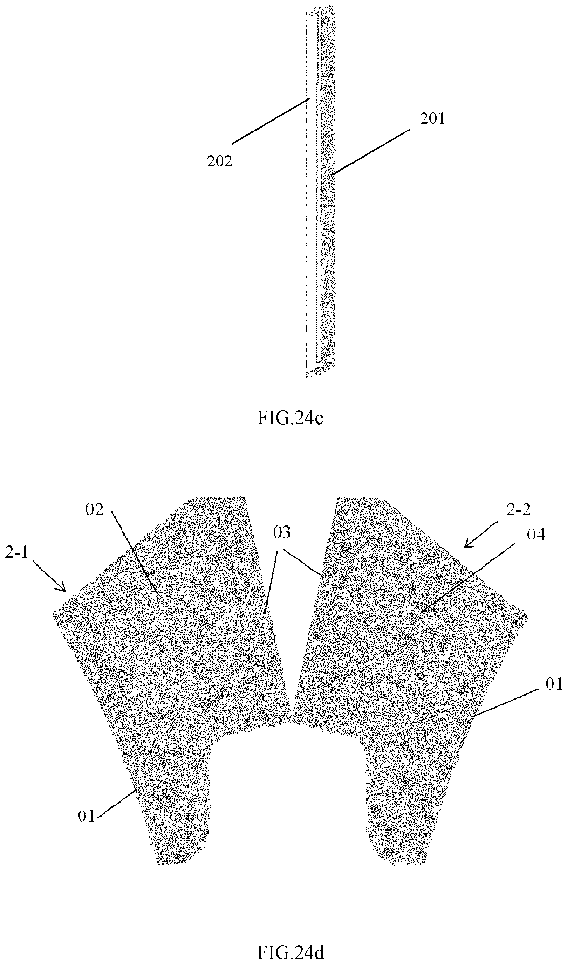

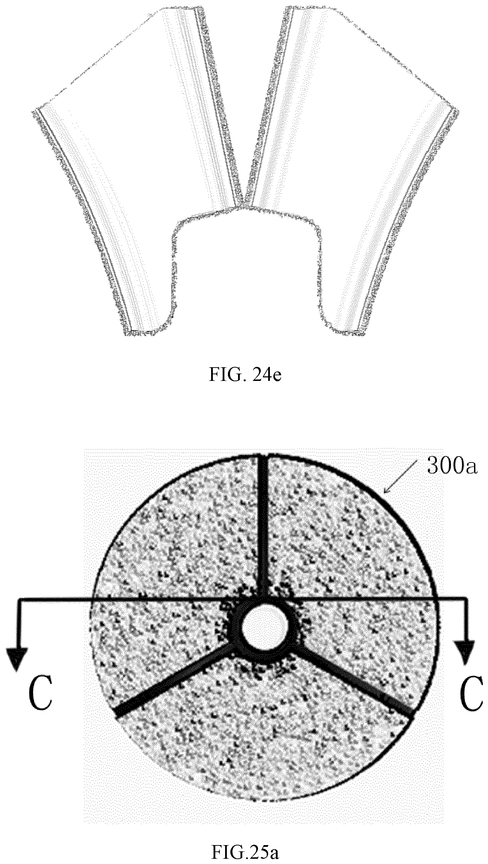

In this Embodiment , the connection area of the femoral stem 3, includes the medial, posterior, lateral, and anterior surfaces of the upper part of the stem body 303a. As shown in FIGS. 24a-e, the stem shell 2 contains two shell pieces: one shell piece 2-1 corresponding to a portion of the medial surface 01, the posterior surface 02, and a portion of the lateral surface 03 of the upper part of the stem body 303a; the other shell piece 2-2 corresponding to the remaining portion of the medial surface 01, the anterior surface 04, and the remaining portion of the lateral surface 03 of the upper part of the stem body 303a. After the two shell pieces are brought together, they are welded to the corresponding area of the the said connection area on the upper part of the stem body. The inner layer of each shell piece is the intermediate structure 202 and the outer layer is all or most of the porous surface structure 201.

As shown in FIGS. 24d-e, the two shell pieces may be symmetrical (or staggered, not shown schematically). Exemplarily, the said shell pieces may have adjacent non-connected edges after both have been welded to the stem body. Alternatively, the neighboring edges of the two shell pieces may be connected at one side (e.g., lateral side 03) after welding but not connected on the other side. Alternatively, the adjacent edges of the two shell pieces are separated from each other at the time of welding, and subsequently connected (e.g., with welding or connectors or other means of joining). The said adjacent edges mean the edges of the shell pieces when they are brought close to each other through their welding to the stem body. The connection along the adjacent edges may be between the inner intermediate structures and/or outer porous surface structures of the shell pieces.

### Embodiment XXVII

In this embodiment, the porous structure of the outer surface of the acetabular cup 300a can be achieved similarly using the structures and methods of EmbodimentsI to XXV above or variations thereof.

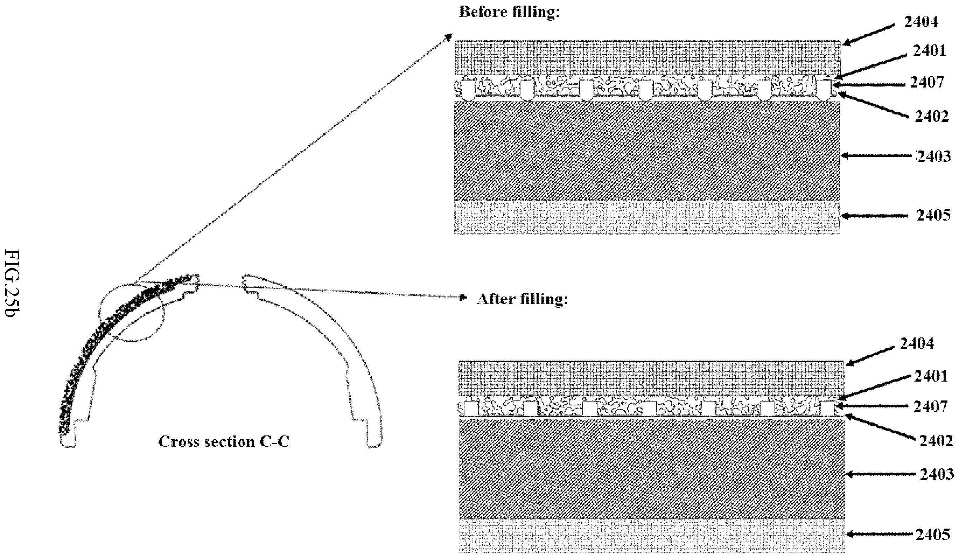

In a specific Embodiment , as shown in Figures 25a-b, corresponding to Figure 16 in Embodiment XXI above, the said acetabular shell contains the cup body as the substrate 2403 in the connected structure, the outer porous structure 2401 as the porous surface structure of the connected structure. An intermediate structure (e.g. non-porous base plate 2402 with hollowed-out design in Embodiment XXI) is provided between the porous structure 2401 and the substrate 2403. The porous structure 2401 and the non-porous base plate 2402 form the composite, which situates on the outer side of the cup body 3-3 and covers the connection area for resistance welding with the substrate 2403. The composite and the substrate are pressed together between the positive electrode 2404 and the negative electrode 2405. When an electrical current is provided, the current flows through the porous structure 2401, the nonporous base plate 2402 and then to the contact surface with the outer surface of the substrate 2403 and the adjacent area, generating resistance heat thereby heating them to a molten or plastic state, causing the nonporous base plate 2402 to form a welding bond with the substrate 2403, and realizing the connection between the nonporous base plate 2402 and the substrate 2403, thereby enabling the strong bonding of the composite of porous surface structure 2401 and non-porous base plate 2402 to the substrate 2403, thus forming a coverage of the attachment area on the body of the cup and obtaining a porous structure on the outer surface of the acetabular cup (shell). Wherein, between the substrate 2403 and the porous structure 2401 is a hollowed-out non-porous base plate 2402 prefabricated with a plurality of raised structures on its bottom, which are in contact with the cup body, and wherein a number of support posts 2407 are provided on the surface of the non-porous base plate 2402 near the side of the porous structure 2401. The support posts 2407 are located between the non-porous base plate 2402 and the positive electrode 2404. The cup body of the acetabular cup of the present invention is matched with the composite (or the intermediate contained therein) at the site of contact and connection. Other elements concerning acetabular cup applicable to Embodiment XXI are not described in detail herein, as well as specific elements concerning acetabular cup applicable to other embodiments are not described in detail herein.

In some Embodiment s, the cup body is manufactured by forging, casting, or machining etc, and is preferably a solid structure that is easy to machine and has high strength; or the cup body may be a high porosity structure; the intermediate structure may be solid or a porous structure with a higher solid volume fraction than that of the porous surface structure. When the cup body and the intermediate structure both are porous, the solid volume fraction of intermediate structure lies between those of cup body and porous surface structure. The intermediate structure and the porous surface structure are preferably manufactured with 3D printing additive manufacturing process, which can readily form specific pores according to the design requirements, etc. The cup body is effectively connected to the intermediate structure with resistance welding, avoiding the current problem of significant reduction in overall strength with high temperature processes (e.g., diffusion bonding), etc.

Exemplarily, the entire outer surface of the cup body is a single connection area, with a single composite body made to be in contact therewith and welded with the said connection area through the intermediate structure of the said composite body. Alternatively, the entire outer surface of the cup body is divided into a plurality of separate connection areas and a plurality of composite bodies (each of which may be in the form of a sheet or other shape matching the dome shell of the cup) are provided, each of which is in contact and welded with a corresponding connection area through a corresponding intermediate structure of the said composite body. Wherein, the inner layer of each composite body is an intermediate structure and all or most of its outer layer is a porous surface structure.

### Embodiment XXVIII

The proximal tibia and the distal femur form the knee joint. The proximal tibia and the distal femur are in contact with each other through bearing surfaces, which are important load-bearing structures of the knee joint. In a prosthetic knee implant, the component replacing the distal femoral bone is called femoral condyle and that replacing the proximal tibial bone is called tibial tray. There is a polyethylene insert placed between the femoral condyle and the tibial tray, for the purpose of wear reduction and functional restoration.

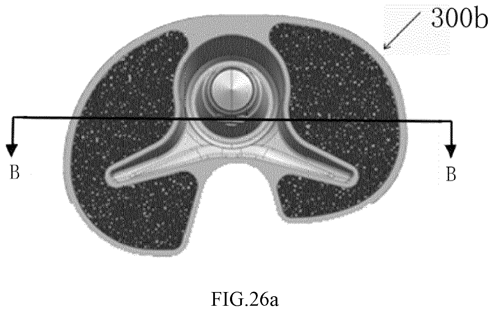

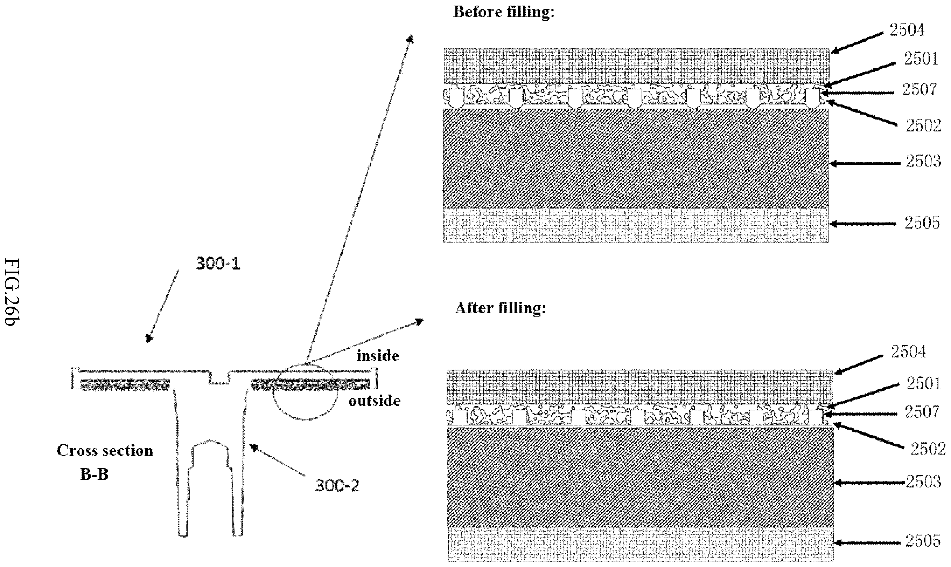

As shown in Figures 26a-b, the tibial tray 300b has a T-shaped structure containing the upper tibial tray 300-1 and the lower support structure 300-2. The lower surface of the tibial tray 300b uses a porous structure to increase the roughness, and to encourage osteoblastic bone ingrowth for effective fixation of the tibial prosthesis into the host tibial bone. The tibial tray 300b is designed to provide a good long-term biological fixation while transmitting joint loads, providing ambulation and resisting wear. The porous structure of the lower surface of the tibial tray 300b may be similarly implemented using the structures and methods of EmbodimentsI through XXV above or variations thereof.

Exemplarily, as shown in FIG. 26a-b, the lower surface of the tibial tray 300-1 corresponds to the porous surface structure 2501 of the connected structure, and the upper end of the tibial tray 300-1 corresponds to the substrate 2503 on the inner side of the connected structure; an intermediate structure (e.g. non-porous base plate 2502 with hollowed-out design according to Embodiment XXI) is provided between the porous structure 2501 and the substrate 2503. A composite body is formed by the porous structure 2501 and the non-porous base plate 2502. The said composite body and the substrate 2503 are pressed between the positive electrode 2504 and the negative electrode 2505. When an electrical current is provided, the current flows through the porous structure 2501, to the nonporous base plate 2502, and then to the surface and the adjacent area at contact with the substrate 2503, generating resistive heat thereby melting or plasticizing contact areas between the nonporous base plate 2502 and the substrate 2503 and welding them together. The composite body of the porous structure 2501 and the non-porous base plate 2502 is tightly bonded to the substrate 2503. The composite body is formed on the lower part of the tibial tray and covers its connection area for welding. Other elements related to the tibial tray applicable to Embodiment XXI are not described in detail herein. And the specifics regarding the tibial tray as applied to other embodiments are not repeatedly described herein.

### Embodiment XXIX

The knee prosthesis includes a femoral condyle, a tibial tray, and a tibial insert between the two, and a patellar prosthesis. The femoral condyle is connected to the distal femur and the tibial tray is connected to the proximal tibia. The tibial insert is connected to the tibial tray, and the femoral condyle is in contact with the tibial insert. The lower part of the tibial insert is in contact with the upper surface of the tibial tray, and the convex surface of the femoral condyle is in contact with the upper part of the tibial insert and the articular surface of the patellar prosthesis, allowing for flexion, extension, sliding, rotation, and other activities within a defined range.

In this regard, the outer convex surface of the femoral condyle 300c body is usually very smooth to reduce wear between it and the tibial insert; while the femoral condyle body will be on its inner concave surface, matching and in contact with the osteotomy section formed at the distal femur, thus preferably forming a porous structure on the inner concave surface of the femoral condyle body (such as the medial condyle fixation surface) to help the bone ingrowth and achieve a tight bond between the prosthesis and the bone tissue, reducing the postoperative loosening of the prosthesis. This reduces the risk of post-operative loosening of the prosthesis, which can lead to failure of the joint replacement surgery. In this embodiment, the use of porous structures on the concave surface of the femoral condyle 300c increases the roughness to enhance the initial postoperative stability of the prosthesis; on the other hand, it promotes bone ingrowth and effectively connects the femoral condyle prosthesis to the human femoral condyle. The tibial insert is located between the femoral condylar prosthesis and the tibial tray prosthesis and is designed to withstand joint loads, meet the kinematic and wear resistance requirements of the joint.

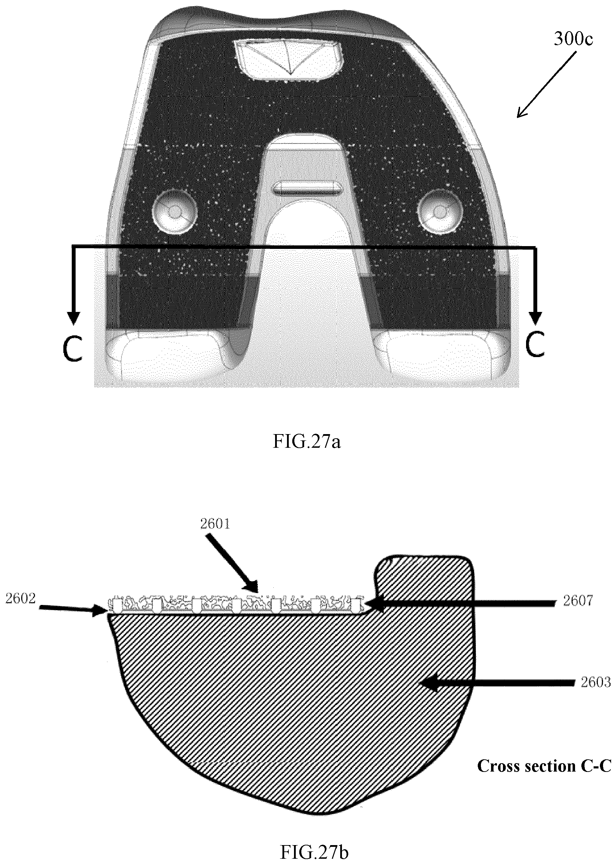

The said porous structure on the inner surface of the femoral condyle 300c may be achieved similarly using the structures and methods of EmbodimentsI to XXV above or variations thereof. In one Embodiment , the medial surface of the femoral condyles is shown in FIG. 27b corresponding to FIG. 16 in Embodiment XXI above. The medial surface of the femoral condyles corresponds to the porous structure 2601 of the connected structure, the intermediate (e.g., the non-porous base plate 2602 of the same hollowed-out design as described in Embodiment XXI) and the substrate 2603 in order from the outside to the inside. The medial condylar fixation surface of the femoral condyle 300c uses a porous surface structure 2601. due to the composite formed by the porous structure 2601 and the non-porous base plate 2602, and the substrate 2603 is pressed between the positive and negative electrodes. When an electric current is passed, the current flows through the porous structure 2601, the nonporous base plate 2602 up to the contact surface and adjacent areas on the outside of the base plate 2603, generating resistance heating thereby heating them to a molten or plastic state, causing the nonporous base plate 2602 to form a bond with the base plate 2603, achieving a solidifying effect between the nonporous base plate 2602 and the base plate 2603, thereby causing the porous structure 2601 and the non-porous base plate 2602 form a composite body tightly bonded to the substrate 2603. Wherein, between the substrate 2603 and the porous structure 2601 is a hollowed-out non-porous substrate 2602, the bottom of the hollowed-out non-porous substrate 2602 is prefabricated with a plurality of raised structures, the raised structures are in contact with the substrate 2603, and a plurality of support posts 2607 are provided on the surface of the non-porous substrate 2602 near the side of the porous structure 2601. the porous surface structure in this Embodiment and intermediate form a composite that is formed on the inner concave surface of the femoral condyle and covers the connection area of the femoral condyle. Other aspects of the femoral condyles applicable to Embodiment XXI are not described herein. The details of the femoral condyles as applied to other embodiments are not repeated herein. Likewise, the patellar prosthesis may also use the structure and method of any of the above embodiments or variations thereof to add a porous structure to the surface in contact with the bone.

EmbodimentsI to XXV of the present invention or variations thereof are not limited to the above-mentioned prosthesis Embodiment s, but can also be applied to, For example, spinal prostheses, ankle joints, shoulder joints, elbow joints, finger joints, toe joints, small intervertebral joints, jaw joints, wrist joints, dental implants (e.g., dental implants are implanted in the alveolar bone and then porcelain teeth installed above the dental implants), and so on, with reference to the above-mentioned structures and principles. The invention will not be repeated here.

Although the contents of the present invention have been described in detail by the above preferred embodiments, it should be recognized that the above description should not be considered a limitation of the present invention. A variety of modifications and alternatives to the present invention will be apparent to those skilled in the art after reading the foregoing. Accordingly, the scope of protection of the present invention shall be limited by the Claims.

## Claims

1. A connected structure of a porous surface structure and a substrate, **characterized in that** it comprises: a pre-connected or integrally formed composite body of a porous surface structure and an intermediate; and a substrate, which is connected to the said intermediate and/or the said porous surface structure to achieve the connection of the said composite body to the said substrate; the said composite body comprising a first composite region corresponding to a first stiffness; a remaining composite region in the composite body other than the first composite region, which at least contains a second composite region corresponding to a second stiffness; and the first stiffness is less than the second stiffness.

2. The connected structure as in Claim 1, **characterized in that** the thickness of the said composite body at the said first composite region is less than the thickness of the said composite body at the said second composite region.

3. The connected structure as in Claim 1, **characterized in** any one of (a)-(c) below or any combination thereof:
(a) the thickness of the porous surface structure at the first composite region is less than the thickness of the porous surface structure at the second composite region;
(b) the thickness of the main body of the intermediate at the first composite region is less than the thickness of the main body of the intermediate at the second composite region;
(c) the gap between the side of the main body of the intermediate at the first composite region facing the substrate and the substrate is greater than the gap between the side of the main body of the intermediate at the second composite region facing the substrate and the substrate; and there is no porous surface structure at the gap;
wherein the said main body of the intermediate is the portion of the intermediate located between the said porous surface structure and the said substrate.

4. The connected structure as in Claim 3, **characterized in that** the thickness of the main body of the intermediate at the first composite region is 0, namely there is no main body of the intermediate at the first composite region, forming a hollowed-out structure.

5. The connected structure as in Claim 3, **characterized in that** the gap between the substrate-facing side of the main body of the intermediate at the said first composite region and the substrate forms a pocket structure between the intermediate and the substrate.

6. The connected structure as in Claim 1, **characterized in that** the said substrate is connected to the said intermediate and/or the said porous surface structure by laser welding and/or resistance welding.

7. The connected structure as in Claim 1, **characterized in that** the said substrate, the said porous surface structure, the said intermediate are made of conductive materials.

8. The connected structure as in any one of Claims 1 to 7, **characterized in that** the said porous surface structure in the said composite is called a first porous structure; the said intermediate is a solid structure, or, alternatively, the said intermediate is a second porous structure and at least a portion of the said second porous structure has a higher solid volume fraction than the said first porous structure.

9. The connected structure as in Claim 8, **characterized in that** the said intermediate further comprises: an insertion portion disposed within the porous surface structure, and/or a raised structure formed on a side of the composite body proximal to the said substrate; one or more of the said main body of the intermediate, the said insertion portion and the said raised structure having a higher solid volume fraction than that of the said porous surface structure.

10. The connected structure as in Claim 9, **characterized in that** the said insertion portion comprises a support post.

11. The connected structure as in Claim 9, **characterized in that** the said main body of the intermediate comprises an intermediate plate structure, the said intermediate plate structure being provided with a plurality of raised structures, the said raised structures being provided on the side of the said intermediate plate structure proximal to the said substrate, the bumps of the said raised structures being in contact with and fixedly connected to the said substrate.

12. The connected structure as in Claim 9, **characterized in that** the said main body of the intermediate is the said second porous structure, the said second porous structure comprising a plurality of raised structures, the said raised structures being formed on a side of the said second porous structure proximal to the said substrate, the bumps of the said raised structures being in contact with the said substrate.

13. The connected structure as in Claim 9, **characterized in that** the said first composite region comprises a plurality of dispersedly arranged composite sub-regions; each of the said sub-regions being between a plurality of adjacent raised structures corresponding thereto, or, alternatively, between a plurality of adjacent insertion portions corresponding thereto.

14. The connected structure as in Claim 9, **characterized in that** all or at least part of the said insertion portion is located within the porous surface structure; the said insertion portion is arranged in correspondence with and in contact with the said raised structure, or the said insertion portion is staggered and not in direct contact with the said raised structure.

15. The connected structure as in Claim 9, **characterized in that** the end surface of the insertion portion away from the substrate protrudes above or subsides below or is flush with the surface of the said porous surface structure;

16. The connected structure as in Claim 15, **characterized in that** when the end surface of the said insertion portion away from the substrate side protrudes above the surface of the said porous surface structure, the protruded portion is cut off after the resistance welding is completed.

17. The connected structure as in Claim 15, **characterized in that** when the surface of the said insertion portion on the side away from the substrate protrudes above the surface of the said porous surface structure: the said insertion portion is a multi-segment structure comprising at least a first segment portion that protrudes above the said porous surface structure and a remaining second segment portion; the said first segment portion is porous; the said second segment portion is porous or solid, the said surface of the second section on the side away from the substrate is flush with the surface of the said porous surface structure such that the first segment is heated by contact with the first polar electrode causing the said insertion section to sink to the surface of the said second section on the side away from the substrate.

18. The connected structure as in Claim 9, **characterized in that** the said raised structures on the said intermediate are located near the contact positions between the said porous surface structure and the said intermediate.

19. The connected structure as in any one of Claims 1 to 7 or 9 to 18, **characterized in that** at least some pores within the said porous surface structure are filled with an electrically conductive material.

20. The connected structure as in Claim 19, **characterized in that** at least some pores within the said porous surface structure are filled with powdered or filamentary or mesh-like conductive materials.

21. The connected structure as in Claim 19, **characterized in that**, at least some pores of the porous surface structure are filled with a conductive medium in the molten state, and/or, at least some portions of the pores of the porous surface structure are filled with a conductive medium which becomes molten subsequently at high temperature; the said conductive medium has a melting point lower than the melting point of the substrate and/or that of the porous surface structure.

22. The connected structure as in any one of Claims 1 to 7 or 9 to 18, **characterized in that** the said substrate is a solid structure, or, alternatively, the said substrate is a third porous structure and the porosity of the said third porous structure is less than the porosity of the said porous surface structure.

23. The connected structure as in Claim 22, **characterized in that** the said substrate is made by forging or casting or machining or powder metallurgy or metal injection molding process.

24. The connected structure as in Claim 22, **characterized in that** the said porous surface structure and the said intermediate of the said composite are manufactured with 3D printing additive manufacturing or vapor phase deposition process.

25. The connected structure as in any one of Claims 9 to 18, **characterized in that** the said porous surface structure, the said intermediate and the said insertion portion are integrally formed.

26. The connected structure as in any one of Claims 1 to 7 or 9 to 18, **characterized in that** the said first composite region of the composite body is made by machining.

27. The connected structure as in any one of Claims 1 to 7 or 9 to 18, **characterized in that** the said substrate comprises a surface attachment layer, the said surface attachment layer being pre-connected or integrally formed with the main body of the substrate, the said surface attachment layer being located between the said intermediate of the composite and the main body of the substrate, or, the said surface attachment layer being located between the porous surface structure of the said composite and the main body of the substrate. the said surface attachment layer comprising raised structures on the substrate, the bumps of the said raised structures of the said surface attachment layer being in contact with and fixedly connected to the said intermediate and/or porous surface structure of the said composite body; or, the said raised structures are located on the surface of the said substrate proximal to the composite, being in contact with and fixedly connected to the said intermediate and/or porous surface structure of the said composite body.

28. The connected structure as in Claim 27, **characterized in that** the said surface attachment layer of the said substrate is pre-welded to the body of the said substrate.

29. The connected structure as in Claim 27, **characterized in that** the said main body of the intermediate has a flat surface proximal to the said substrate , or, alternatively, the said main body of the intermediate has raised structures on the surface proximal to the said substrate staggered relative to the raised structures on the surface attachment layer of the said substrate.

30. The connected structure of a porous surface structure and a substrate, **characterized in that** it contains a plurality of composites as in any one of Claims 1 to 29, with the plurality of composites being respectively connected to different regions of the substrate.

31. a connected structure as in claim 30, **characterized in that** the plurality of composites comprises at least a first composite and a second composite, an intermediate in the first composite contacting and fixedly connected to a part of the said substrate, and intermediates in the said second composite body contacting and fixedly connected to another part of the said substrate to achieve the connection of the composite body to the said substrate; the said part of the said substrate and the said other part of the said substrate are respectively located on opposite sides of the substrate.

32. The connected structure as in Claim 31, **characterized in that** the said first composite and the said second composite have the same structure; alternatively, the said first composite and the said second composite have different structures.

33. The connected structure of a porous surface structure and a substrate, **characterized in that** it comprises: a pre-connected or integrally formed composite body of of a porous surface structure and an intermediate; a substrate, connected to the said intermediate and/or porous surface structure to achieve the connection of the said composite body to the said substrate; wherein it further comprises any one of (a)-(c) or any combination thereof:
(a) the said intermediate being divided into a first region and remaining regions other than the first region, the said remaining regions comprising at least a second region, the thickness of the main body of the intermediate of the said intermediate at the said second region being greater than that at the said first region; the thickness of the main body of the intermediate at the said first region being greater than or equal to 0;
(b) the said porous surface structure being divided into a first region and remaining regions other than the first region, the said remaining regions comprising at least a second region in which the thickness of the porous surface structure is greater than that at the said first region;
(c) the said composite body being divided into a first composite region and remaining composite regions other than the first composite region, the said remaining composite regions comprising at least a second composite region, the gap between the side of the main body of the intermediate at the second composite region facing the substrate and the substrate is smaller than the gap between the side of the main body of the intermediate at the first composite region facing the substrate and the substrate; and there is no porous surface structure at the gap;
wherein the said main body of the intermediate is the portion of the intermediate located between the said porous surface structure and the said substrate.

34. The method for preparing a connected structure of a porous surface structure and a substrate, **characterized in that** the method comprises the following process: forming a composite by pre-connecting the porous surface structure to an intermediate or integrally forming the composite; bringing the said intermediate and/or the said porous surface structure into contact with the substrate; bringing the said first polar electrode into conductive contact with the said porous surface structure and/or intermediate of the said composite and bringing the said substrate into conductive contact with a second polar electrode, thereby forming a current circuit; further comprising any one of (a)-(c) or any combination thereof:
(a) the said intermediate being divided into a first region and remaining regions other than the first region, the said remaining regions comprising at least a second region, the thickness of the main body of the intermediate of the said intermediate at the said second region being greater than that at the said first region; the thickness of the main body of the intermediate at the said first region being greater than or equal to 0;
(b) the said porous surface structure being divided into a first region and remaining regions other than the first region, the said remaining regions comprising at least a second region in which the thickness of the porous surface structure is greater than that at the said first region;
(c) the said composite body being divided into a first composite region and remaining composite regions other than the first composite region, the said remaining composite regions comprising at least a second composite region, the gap between the side of the main body of the intermediate at the second composite region facing the substrate and the substrate is smaller than the gap between the side of the main body of the intermediate at the first composite region facing the substrate and the substrate; and there is no porous surface structure at the gap;
wherein the said main body of the intermediate is the portion of the intermediate located between the said porous surface structure and the said substrate;
the first composite region and the second composite region of the said composite body are resistance welded to the said substrate, respectively, to achieve the connection of the composite body to the substrate.

35. The method for preparing a connected structure of a porous surface structure and a substrate, **characterized in that** the method comprises the following process: forming a composite by pre-connecting the porous surface structure to an intermediate or integrally forming the composite; bringing the said intermediate and/or the said porous surface structure into contact with the substrate; bringing the said first polar electrode into conductive contact with the said porous surface structure and/or intermediate of the said composite and bringing the said substrate into conductive contact with a second polar electrode, thereby forming a current circuit; the said composite body comprising a first composite region corresponding to a first stiffness; a remaining composite region in the composite body other than the first composite region, which at least contains a second composite region corresponding to a second stiffness; and the first stiffness is less than the second stiffness; the first composite region and the second composite region of the said composite body are resistance welded to the said substrate, respectively, to achieve the connection of the composite body to the substrate.

36. The method as in Claim 35, **characterized in that** the thickness of the composite at the said first composite region, is less than the thickness of the composite at the said second composite region.

37. The method as in Claim 35, **characterized in that** it further comprises any one of (a)-(c) or any combination thereof:
(a) the thickness of the porous surface structure at the first composite region is less than the thickness of the porous surface structure at the second composite region;
(b) the thickness of the main body of the intermediate at the first composite region is less than the thickness of the main body of the intermediate at the second composite region;
(c) the gap between the side of the main body of the intermediate at the first composite region facing the substrate and the substrate is greater than the gap between the side of the main body of the intermediate at the second composite region facing the substrate and the substrate; and there is no porous surface structure at the gap;
wherein the said main body of the intermediate is the portion of the intermediate located between the said porous surface structure and the said substrate.

38. The method as in Claim 37, **characterized in that** the thickness of the main body of the intermediate at the first composite region is 0; there is no main body of the intermediate at the first composite region, forming a hollowed-out structure.

39. The method as in Claim 37, **characterized in that** the gap between the substrate-facing side of the main body of the intermediate at the said first composite region and the substrate causes the intermediate to form a pocket structure with the substrate.

40. The method as in Claim 35, **characterized in that** the porous surface structure in the said composite is referred to as a first porous structure; the said intermediate is a solid structure, or, the said intermediate is a second porous structure and at least a portion of the said second porous structure has a higher solid volume fraction than the solid volume fraction of the said first porous structure.

41. The method as in Claim 37, **characterized in that** the said intermediate further comprises: an insertion portion disposed within a porous surface structure, and/or a raised structure formed on a side of the composite proximal to the said substrate; one or more of the said main body of the intermediate, the said insertion portion, and the said raised structure having a higher solid volume fraction than the solid volume fraction of the said porous surface structure.

42. The method as in Claim 35, **characterized in that** the said substrate is connected to the said intermediate by laser welding and/or resistance welding.

43. The method as in Claim 35, **characterized in that** when the said resistance welding is a projection resistance welding, the said first polar electrode is a continuous planar electrode or a segmented plurality of electrode monomers, and the said second polar electrode is a continuous planar electrode or a segmented plurality of electrode monomers; when the said resistance welding is a spot resistance welding, the said first polar electrode and/or the said second polar electrode is a segmented plurality of electrode monomers.

44. The method as in Claim 43, **characterized in that**, for spot resistance welding, moving from the current welding position to the next is achieved by moving any one or more of the following components: the first polar electrode, the second polar electrode, the combined structure of the intermediate and the substrate already welded at at least one contact position.

45. The method as in Claim 43, **characterized in that** when the said first polar electrode is divided into a plurality of electrode monomers, the said electrode monomers are inserted into prefabricated gaps within the porous surface structure, the electrode monomers being in close proximity to the said intermediates such that the said electrode monomers after insertion are in conductive contact with the said intermediates or such that the said electrode monomers after insertion are in conductive contact with the said intermediates after passing through the porous surface structure .

46. The method as in Claim 43, **characterized in that** the said electrode monomers pass through the surface of the porous surface structure up to the surface of the intermediate or into the interior of the intermediate, such that the said electrode monomers after insertion are in conductive contact with the said intermediate

47. The method as in Claim 46, **characterized in that** the said electrode monomers are in lateral clearance-fit with the said porous surface structure, such that they are not in contact at all with the said porous surface structure.

48. The method as in Claim 46, **characterized in that** the said plurality of electrode monomers are connected in parallel to another planar electrode and the said other planar electrode is connected to the power supply end, or, alternatively, the said plurality of electrode monomers are connected in parallel and directly to the power supply end.

49. The method as in Claim 35, **characterized in that** the said first polar electrode is a flexible electrode, which is deformed under pressure to conform to the surface of the said porous surface structure, thereby increasing the area of contact between the said flexible electrode and the surface of the said porous surface structure.

50. The method as in Claim 35, **characterized in that** the said first polar electrode is a positive electrode and the said second polar electrode is a negative electrode; or, that the said first polar electrode is a negative electrode and the said second polar electrode is a positive electrode.

51. The method as in Claim 35, **characterized in that** the said first polar electrode and the said second polar electrode are made of conductive materials; the said substrate, the said porous surface structure , and the said intermediate are made of conductive materials.

52. The method as in Claim 41, **characterized in that** the said insertion portion comprises a support post.

53. The method as in Claim 41, **characterized in that** the said main body of the intermediate comprises an intermediate plate structure, the said intermediate plate structure being provided with a plurality of raised structures, the said raised structures being provided on the side of the said intermediate plate structure proximal to the said substrate, the bumps of the said raised structures being in contact with the said substrate; or, alternatively, the said main body of the intermediate is the said second porous structure, the said second porous structure comprising a plurality of raised structures, the said raised structures being formed on the side of the said second porous structure proximal to the said substrate, the bumps of the said raised structures being in contact with the said substrate.

54. The method as in Claim 41, **characterized in that** the multiple composite sub-regions distributed across the said first composite region comprise of at least portions of the composite region between any two adjacent raised structures or between any two adjacent insertion portions.

55. The method as in Claim 41, **characterized in that** all or at least some parts of the said insertion portions are located within the porous surface structure; the said insertion portions are placed in contact with the corresponding raised structures , or misplaced with the said raised structures without contacting each other.

56. The method as in Claim 41, **characterized in that** the end surface of the insertion portion on the side away from the substrate protrudes above, or subsides below or is flush with the surface of the said porous surface structure.

57. The method as in Claim 41, **characterized in that** when the end surface of the said insertion portion away from the substrate side protrudes above the surface of the said porous surface structure, the protruded portion is cut off after the resistance welding is completed.

58. The method as in Claim 41, **characterized in that** when the surface of the said insertion portion on the side away from the substrate protrudes above the surface of the said porous surface structure: the said insertion portion is a multi-segment structure comprising at least a first segment portion that protrudes above the said porous surface structure and a remaining second segment portion; the said first segment portion is porous; the said second segment portion is porous or solid, the said surface of the second section on the side away from the substrate is flush with the surface of the said porous surface structure such that the first segment is heated by contact with the first polar electrode causing the said insertion section to sink to the surface of the said second section on the side away from the substrate.

59. The method as in Claim 41, **characterized in that** the said raised structures are placed on the said intermediate close to locations where the said porous surface structure is in contact with the said intermediate.

60. The method as in any one of Claims 35 to 59, **characterized in that** at least a portion of the pores of the said porous surface structure are filled with conductive materials.

61. The method as in any one of Claims 35 to 59, **characterized in that** at least a portion of the pores of the said porous surface structure are filled with powdered or filamentary or mesh-like conductive materials.

62. The method as in any one of Claims 35 to 59, **characterized in that** at least a portion of the surface of the porous surface structure is covered with a solid film-like deformable conductive medium, the said deformable conductive medium being located between the said first polar electrode and the said porous surface structure; and/or, at least a portion of the surface of the porous surface structure is sprayed with the conductive medium between the surface of the porous surface structure and the said first polar electrode

63. The method as in any one of Claims 35 to 59, **characterized in that** at least some pores of the porous surface structure are filled with a conductive medium in molten form, and/or, at least some pores of the porous surface structure are built with a conductive medium which is made molten by high temperature; the said conductive medium has a melting point lower than the melting point of the substrate and/or that of the porous surface structure.

64. The method as in any one of Claims 35 to 59, **characterized in that** the said substrate is a solid structure, or, alternatively, the said substrate is a third porous structure and the porosity of the said third porous structure is less than the porosity of the said porous surface structure.

65. The method as in Claim 64, **characterized in that** the said substrate is manufactured with a forging or casting or machining or powder metallurgy or metal injection molding process.

66. The method as in Claim 64, **characterized in that** the porous surface structure and the intermediate of the said composite, is manufactured with a 3D printing additive manufacturing process, or a vapor phase deposition process.

67. The method as in Claim 41, **characterized in that** the said porous surface structure, the said main body of the intermediate and the said insertion portion are integrally formed.

68. The method as in any one of Claims 35 to 59, **characterized in that** the said first composite region of the composite body is made by machining..

69. The method as in any one of Claims 35 to 56, **characterized in that** the said substrate comprises a surface attachment layer, the said surface attachment layer being pre-connected or integrally formed with the main body of the substrate, the said surface attachment layer being located between the said intermediate of the composite and the main body of the substrate, or, the said surface attachment layer being located between the porous surface structure of the said composite and the main body of the substrate. the said surface attachment layer comprising raised structures on the substrate, the bumps of the said raised structures of the said surface attachment layer being in contact with and fixedly connected to the said intermediate and/or porous surface structure of the said composite body; or, the said raised structures are located on the surface of the said substrate proximal to the composite, being in contact with and fixedly connected to the said intermediate and/or porous surface structure of the said composite body.

70. The method as in Claim 69, **characterized in that** the said surface attachment layer of the said substrate is pre-welded to the body of the said substrate.

71. The method as in Claim 69, **characterized in that** the said main body of the intermediate has a flat surface proximal to the said substrate, or, alternatively, the said main body of the intermediate has raised structures on the surface proximal to the said substrate staggered relative to the raised structures on the surface attachment layer of the said substrate.
wherein the said main body of the intermediate is the portion of the intermediate located between the said porous surface structure and the said substrate.

72. The method as in any one of Claims 35 to 59 or 65 to 67, **characterized in that** the said first polar electrode is in conductive contact with a side of at least a portion of the composite.

73. The method as in Claim 72, **characterized in that** the said first polar electrode is in electrically conductive contact with one or more of the sides of the porous surface structure, the sides of the insertion portion of the intermediate, and the sides of the main body of the intermediate.

74. The connected structure of a porous surface structure and a substrate, **characterized in that** it comprises: a pre-connected or integrally formed composite, comprising a porous surface structure and an intermediate; a substrate, in contact with the said intermediate and/or the said porous surface structure; the said intermediate comprising a plurality of insertion portions, all or at least part of the said insertion portions being located within the porous surface structure, the said insertion portions being conductively connected to an accessory structure conductively connected, and the said accessory structure conductively connected to the said porous surface structure, the said accessory structure being disposed within the porous surface structure or, between the porous surface structure and the intermediate, or between the porous surface structure and the substrate.

75. The connected structure as in Claim 74, **characterized in that** the said composite body comprises a first composite region corresponding to a first stiffness; the said remaining composite regions in the composite body, other than the first composite region, comprises at least a second composite region corresponding to a second stiffness; the said first stiffness being less than the said second stiffness.

76. The connected structure as in Claim 74, **characterized in that** the thickness of the said composite body at the said first composite region, is less than the thickness of the said composite body at the said second composite region.

77. The connected structure as in Claim 74, **characterized in** any one of (a)-(c) below or any combination thereof:
(a) the thickness of the porous surface structure at the first composite region is less than the thickness of the porous surface structure at the second composite region;
(b) the thickness of the main body of the intermediate at the first composite region is less than the thickness of the main body of the intermediate at the second composite region;
(c) the gap between the side of the main body of the intermediate at the first composite region facing the substrate and the substrate is greater than the gap between the side of the main body of the intermediate at the second composite region facing the substrate and the substrate; and there is no porous surface structure at the gap;
wherein the said main body of the intermediate is the portion of the intermediate located between the said porous surface structure and the said substrate.

78. The connected structure as in Claim 77, **characterized in that** the thickness of the main body of the intermediate at the first composite region is 0, namely there is no main body of the intermediate at the first composite region, forming a hollowed-out structure.

79. The connected structure as in Claim 77, **characterized in that** the gap between the substrate-facing side of the main body of the intermediate at the said first composite region and the substrate forms a pocket structure between the intermediate and the substrate.

80. The connected structure as in Claim 75, **characterized in that** the said insertion portion is good conductive solid structure or a third porous structure.

81. The connected structure as in Claim 80, **characterized in that** the said insertion portion comprises a support post.

82. The connected structure as in Claim 74, **characterized in that** the said accessory structure is a good conductive solid structure or a fourth porous structure.

83. The connected structure as in Claim 82, **characterized in that** the solid volume fraction of the said fourth porous structure is lower than the solid volume fractions of the said insertion portions.

84. The connected structure as in Claim 82, **characterized in that** the said main body of the intermediate of the said intermediate is a portion disposed between the said porous surface structure and the said substrate, the said accessory structure being connected to the said main body of the intermediate, or, alternatively, the said accessory structure not being connected to the said main body of the intermediate.

85. The connected structure as in Claim 82, **characterized in that** the respective accessory structures conductively connected on the plurality of insertion portions of the said intermediate are the same or different.

86. The connected structure as in Claim 85, **characterized in that** any one of the insertion portions is conductively connected to an accessory structure in the shape of "I", or that a plurality of accessory structures conductively connected to any one of the insertion portions form the shape of "+".

87. The connected structure as in any one of Claims 74 to 86, **characterized in that** the said substrate and the said composite are placed between a first polar electrode and a second polar electrode, forming a current circuit by means of the said first polar electrode being in conductive contact with the said porous surface structure and/or intermediate, and the said substrate being in conductive contact with the second polar electrode, allowing the said intermediate and the said substrate to be resistance welding to achieve the connection of the said composite to the said substrate; one or more insertion portions are conductively connected to the corresponding accessory structures, allowing an increase in the current of the said current circuit.

88. The connected structure as in any one of Claims 74 to 86, **characterized in that** the said substrate, the said porous surface structure, the said intermediate, and the said accessory structure are made of conductive materials.

89. The connected structure as in any one of Claims 74 to 86, **characterized in that** the said main body of the intermediate of the said intermediate is a portion disposed between the said porous surface structure and the said substrate, the said main body of the intermediate being an intermediate plate structure, the said intermediate plate structure being provided with a plurality of raised structures, the said raised structures being provided on the side of the said intermediate plate structure proximal to the said substrate.

90. The connected structure as in any one of Claims 74 to 86, **characterized in that** the said main body of the intermediate is a portion disposed between the said porous surface structure and the said substrate, the said main body of the intermediate being the said second porous structure, the said second porous structure comprising a plurality of raised structures, the said raised structures being formed on the side of the said second porous structure proximal to the said substrate.

91. The connected structure as in any one of Claims 74 to 86, **characterized in that** the said intermediate comprises a plurality of raised structures distributed on the side of the said porous surface structure proximal to the substrate.

92. The connected structure as in any one of Claims 74 to 86, **characterized in that** the said insertion portions of the intermediate are placed at locations corresponding to the said raised structures and form contacts at these locations, or they are placed in staggers relative to the said raised structures of the intermediate without forming contacts.

93. The connected structure as in Claim 92, **characterized in that** the end surface of the said insertion portion on the side away from the substrate protrudes above, or subsides below or is flush with the surface of the porous surface structure.

94. The connected structure as in Claim 93, **characterized in that**, when the end surface of the said insertion portion away from the substrate side protrudes above the surface of the said porous surface structure, the portion of the said insertion portion that protrudes above the said porous surface structure is cut off after the resistance welding is completed.

95. The connected structure as in Claim 93, **characterized in that** when the surface of the said insertion portion on the side away from the substrate protrudes above the surface of the said porous surface structure: the said insertion portion is a multi-segment structure comprising at least a first segment portion that protrudes above the said porous surface structure and a remaining second segment portion; the said first segment portion is porous; the said second segment portion is porous or solid, the said surface of the second section on the side away from the substrate is flush with the surface of the said porous surface structure such that the first segment is heated by contact with the first polar electrode causing the said insertion section to sink to the surface of the said second section on the side away from the substrate.

96. The connected structure as in Claim 92, **characterized in that** the said raised structures are located on the said intermediate in proximity to the contact position of the said porous surface structure with the said intermediate.

97. The connected structure as in any one of Claims 74 to 86, **characterized in that** at least some portions of the pores of the said porous surface structure are filled with conductive materials.

98. The connected structure as in any one of Claims 74 to 86, **characterized in that** at least some portions of the pores of the said porous surface structure are filled with powdered, filamentary or mesh-like conductive materials.

99. The connected structure as in any one of Claims 74 to 86, **characterized in that**, at least some pores of the porous surface structure are filled with a conductive medium in the molten state, and/or, at least some portions of the pores of the porous surface structure are filled with a conductive medium which becomes molten subsequently at high temperature; the said conductive medium has a melting point lower than the melting point of the substrate and/or that of the porous surface structure.

100. The connected structure as in any one of Claims 74 to 86, **characterized in that** the said substrate is a solid structure, or, alternatively, the said substrate is a second porous structure and the porosity of the said second porous structure is less than the porosity of the said porous surface structure.

101. The connected structure as in Claim 100, **characterized in that** the said substrate is made by a forging or casting or machining or powder metallurgy or metal injection molding process.

102. The connected structure as in any one of Claims 74 to 86, **characterized in that** the said porous surface structure and the said intermediate of the composite body is integrally formed.

103. The connected structure as in Claim 102, **characterized in that** the said porous surface structure and the said intermediate of the composite are manufactured with a 3D printing additive manufacturing process, or a vapor phase deposition process.

104. The connected structure as in Claim 102, **characterized in that** the said porous surface structure, the said intermediate, the said insertion portion and the said accessory structure are integrally formed.

105. The connected structure of a porous surface structure and a substrate, **characterized in that** it comprises a plurality of composites in any one of the connected structures as in Claims 74 to 104, and the plurality of composites being connected to different portions of the substrate, respectively.

106. The connected structure as in Claim 105, **characterized in that** the plurality of composites comprises at least a first composite and a second composite, an intermediate in the first composite contacting and fixedly connected to a part of the said substrate, and an intermediate in the said second composite body contacting and fixedly connected to another part of the said substrate to achieve the connection of the composite body to the said substrate; the said part of the said substrate and the said other part of the said substrate are respectively located on opposite sides of the substrate.

107. The connected structure as in Claim 106, **characterized in that** the said first composite and the said second composite have the same structure; or, that the said first composite and the said second composite have different structures.

108. The method for preparing the connected structure of a porous surface structure and a substrate as in any one of Claims 74 to 104, comprising the following process: forming a composite by pre-connecting the porous surface structure to an intermediate or integrally forming the composite; bringing the said intermediate and/or the said porous surface structure into contact with the substrate; wherein the said intermediate comprises a plurality of insertion portions, each insertion portion being wholly or at least partially located within the porous surface structure, one or more insertion portions being conductively connected to corresponding accessory structures, and the said accessory structures being conductively connected to the said porous surface structure such that the current in the said current circuit is increased, the said accessory structures being placed within the porous surface structure, or between the porous surface structure and the intermediate, or, between the porous surface structure and the substrate; the said first polar electrode is in conductive contact with the said porous surface structure and/or intermediate, and the said substrate is in conductive contact with the said second polar electrode, forming a current circuit; the said composite is resistance welded to the said substrate to achieve the connection.

109. The prosthesis, **characterized in that** it contains a connected structure as in any one of Claims 1 to 29, the said connected structure comprising: (a) a pre-connected or integrally formed composite body of a porous surface structure and an intermediate, with the said composite body comprising a first composite region corresponding to a first stiffness and the remaining composite regions other than the first composite region, comprising at least a second composite region corresponding to a second stiffness; the said first stiffness being less than the said second stiffness; and (b) a substrate for forming a prosthetic body, at least a portion of the surface of the said prosthetic body serving as an area for connecting to the said composite body, the said intermediate and/or porous surface structure being connected to the said connection area of the said prosthetic body such that the said porous surface structure is located in the said connection area of the said prosthetic body.

110. The prosthesis as in Claim 109, **characterized in that** the said prosthesis is a joint prosthesis.

111. The prosthesis as in Claim 109, **characterized in that** the said composite is formed as a shell, wrapped around the connection region of the said prosthetic body; the outer layer of the said shell comprises a porous surface structure; and the inner layer of the said shell comprises an intermediate, which is connected to the connection region of the said prosthetic body.

112. The prosthesis as in Claim 109, **characterized in that** the said shell formed by the composite is an integral unit; or, the said shell formed by the composite comprises a plurality of shell units; wherein the plurality of shell units are independent of each other, or wherein adjacent shell units are connected to each other on at least one adjacent side.

113. The prosthesis as in Claim 110, **characterized in that** the said prosthesis comprises a femoral stem of the hip joint, the said femoral stem comprising a stem, which is formed as a substrate; the said connection region is positioned at the upper surface of the stem.

114. A prosthesis as in Claim 113, **characterized in that** the said lower surface of the said stem is a smooth surface, the said lower part of the said stem is provided with a number of longitudinal grooves, and the said lower part of the said stem is inserted into the femoral medullary cavity.

115. The prosthesis as in Claim 113, **characterized in that** the said femoral stem further comprises a top part and a neck, with the said top part, neck and the said stem body being integrally formed or assembled; the said top part of the femoral stem being of a conical structure, the first end of which is connected to the stem body through the neck and the second end of which is inserted into the femoral head; the top part and neck being arranged at an angle with respect to the stem body, in the form of an inclination with respect to the side of the stem body.

116. The prosthesis as in Claim 113, **characterized in that** the said composite body is formed as a shell wrapped around the periphery of the connection region of the said stem; the said composite body comprising a plurality of shell units.

117. The prosthesis as in Claim 110, **characterized in that** the said prosthesis comprises an acetabular cup of the hip joint, the said acetabular cup comprising an inner cup body as the substrate and the outer surface of the acetabular cup as the connection region.

118. The prosthesis as in Claim 110, **characterized in that** the said prosthesis comprises a tibial component, the said tibial component comprising a tibial tray which is formed as a substrate; the said connection area is positioned at the distal surface of the tibial tray.

119. The prosthesis as in Claim 110, **characterized in that** the said prosthesis comprises a femoral condyle, the said femoral condyle comprising the condylar body as the substrate and the inner fixation surface as the connection area.

120. The prosthesis as in Claim 110, **characterized in that** the said prosthesis is any one or more of: a patella prosthesis, a spinal fusion device, an intervertebral facet joint of the spine, an ankle joint prosthesis, a shoulder joint implant, an elbow joint prosthesis, a finger joint prosthesis, a toe joint implant, an artificial disc, a mandibular joint prosthesis, a wrist joint prosthesis.

121. The prosthesis, **characterized in that** a connected structure as in any one of Claims 74 to 104 is provided, the said connected structure comprising: a pre-connected or integrally formed composite of a porous surface structure and an intermediate, and a substrate, which is the main body of the prosthesis, of which at least a part of the surface being used as the connection area to connect with the said composite. The said intermediate and/or the said porous surface structure are connected with the connection area of the main body of the prosthesis so that the porous surface structure is located at the connection region of the main body of the prosthesis. The said intermediate comprises a plurality of insertion portions, each insertion portion being wholly or at least partially located within the porous surface structure, one or more insertion portions being conductively connected to corresponding accessory structures, and the said accessory structures being conductively connected to the said porous surface structure, the said accessory structures being placed within the porous surface structure, or between the porous surface structure and the intermediate, or, between the porous surface structure and the substrate.

122. The prosthesis as in Claim 121, **characterized in that** the said prosthesis is a joint prosthesis.

123. The prosthesis as in Claim 121, **characterized in that** the said composite is formed as a shell, wrapped around the connection region of the said prosthetic body; the outer layer of the said shell comprises a porous surface structure; and the inner layer of the said shell comprises an intermediate, which is connected to the connection region of the said prosthetic body.

124. The prosthesis as in Claim 121, **characterized in that** the said shell formed by the composite is an integral unit; or, the said shell formed by the composite comprises a plurality of shell units; wherein the plurality of shell units are independent of each other, or wherein adjacent shell units are connected to each other on at least one adjacent side.

125. The prosthesis as in Claim 122, **characterized in that** the said prosthesis comprises a femoral stem of the hip joint, the said femoral stem comprising a stem, which is formed as a substrate; the said connection region is positioned at the upper surface of the stem.

126. A prosthesis as in Claim 125, **characterized in that** the said lower surface of the said stem is a smooth surface, the said lower part of the said stem is provided with a number of longitudinal grooves, and the said lower part of the said stem is inserted into the femoral medullary cavity.

127. The prosthesis as in Claim 125, **characterized in that** the said femoral stem further comprises a top part and a neck, with the said top part, neck and the said stem body being integrally formed or assembled; the said top part of the femoral stem being of a conical structure, the first end of which is connected to the stem body through the neck and the second end of which is inserted into the femoral head; the top part and neck being arranged at an angle with respect to the stem body, in the form of an inclination with respect to the side of the stem body.

128. The prosthesis as in Claim 121, **characterized in that** the said composite body is formed as a shell wrapped around the periphery of the connection region of the said stem; the said composite body comprising a plurality of shell units.

129. The prosthesis as in Claim 122, **characterized in that** the said prosthesis comprises an acetabular cup of the hip joint, the said acetabular cup comprising an inner cup body as the substrate and the outer surface of the acetabular cup as the connection region.

130. The prosthesis as in Claim 122, **characterized in that** the said prosthesis comprises a tibial component, the said tibial component comprising a tibial tray which is formed as a substrate; the said connection area is positioned at the distal surface of the tibial tray.

131. The prosthesis as in Claim 122, **characterized in that** the said prosthesis comprises a femoral condyle, the said femoral condyle comprising the condylar body as the substrate and the inner fixation surface as the connection area.

132. The prosthesis as in Claim 122, **characterized in that** the said prosthesis is any one or more of: a patella prosthesis, a spinal fusion device, an intervertebral facet joint of the spine, an ankle joint prosthesis, a shoulder joint implant, an elbow joint prosthesis, a finger joint prosthesis, a toe joint implant, an artificial disc, a mandibular joint prosthesis, a wrist joint prosthesis and a dental implant.
